(19) Europäisches Patentamt
European Patent Office
Office européen des brevets

(11) EP 4 640 828 A1

(12) # EUROPEAN PATENT APPLICATION

published in accordance with Art. 153(4) EPC

(43) Date of publication:
**29.10.2025 Bulletin 2025/44**

(21) Application number: **23907891.8**

(22) Date of filing: **22.12.2023**

(51) International Patent Classification (IPC):
***C12N 9/14*** (2006.01) ***C12N 9/10*** (2006.01)
***A61K 38/45*** (2006.01) ***A61K 38/46*** (2006.01)
***A61P 35/00*** (2006.01) ***C07K 14/82*** (2006.01)
***C12N 15/62*** (2006.01) ***A61K 39/00*** (2006.01)

(52) Cooperative Patent Classification (CPC):
**A61K 38/45; A61K 38/46; A61K 39/00; A61P 35/00; C07K 14/82; C12N 9/10; C12N 9/14; C12N 15/62**

(86) International application number:
**PCT/KR2023/021488**

(87) International publication number:
**WO 2024/136608 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 KR 20220183225**
**12.04.2023 KR 20230048320**

(71) Applicant: **Hanmi Pharm. Co., Ltd.**
**Hwaseong-si, Gyeonggi-do 18536 (KR)**

(72) Inventors:
- **KIM, Yu Yon**
**Hwaseong-si, Gyeonggi-do 18469 (KR)**
- **KIM, Inn Ah**
**Hwaseong-si, Gyeonggi-do 18469 (KR)**
- **DONG, Joo Young**
**Hwaseong-si, Gyeonggi-do 18469 (KR)**
- **SHIN, Seung Hyun**
**Hwaseong-si, Gyeonggi-do 18469 (KR)**
- **LEE, Ji Hee**
**Hwaseong-si, Gyeonggi-do 18469 (KR)**
- **LIM, Chang Gyu**
**Hwaseong-si, Gyeonggi-do 18469 (KR)**
- **JEONG, Seong Ju**
**Hwaseong-si, Gyeonggi-do 18469 (KR)**
- **HAN, Young Jin**
**Hwaseong-si, Gyeonggi-do 18469 (KR)**
- **HEO, Yong Ho**
**Hwaseong-si, Gyeonggi-do 18469 (KR)**

(74) Representative: **Brevalex**
**Tour Trinity**
**1 B Place de la Défense**
**92400 Courbevoie (FR)**

Remarks:
The complete document including Reference Table(s) and the Sequence Listing(s) can be downloaded from the EPO website

(54) # ANTIGENIC PEPTIDE HAVING MULTIPLE KRAS VARIANT PEPTIDES LINKED THERETO, NUCLEIC ACID ENCODING THE SAME, AND USE THEREOF

(57) Provided are an antigenic peptide linked to multiple KRAS variant peptides, an mRNA encoding the same, a DNA encoding the same, an immunogenic composition including an antigenic peptide linked to multiple KRAS variant peptides or an mRNA encoding the same, and a method of inducing an immune response to KRAS variant peptides in a subject.

FIG. 2

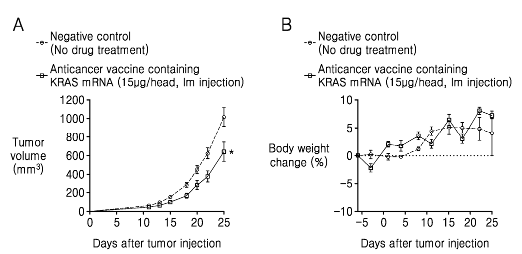


EP 4 640 828 A1

## Description

Technical Field

The disclosure relates to an antigenic peptide having multiple KRAS variant peptides linked thereto, a nucleic acid encoding the same, and uses thereof.

Background Art

Nucleic acid vaccines rely on antigens encoded by DNA or RNA. DNA vaccines generally include an antigen-encoding gene inserted into a bacterial plasmid under the control of a eukaryotic promoter. RNA vaccines, on the other hand, utilize messenger RNA (mRNA) or other antigen-encoding RNAs. Similar to protein vaccines, nucleic acid vaccines may be delivered via a variety of routes, such as intramuscular, subcutaneous, mucosal, or transdermal delivery.

DNA vaccines are known to induce weaker immune responses than peptide vaccines, cellular vaccines, viral vector vaccines, and RNA vaccines. In addition, DNA vaccines not only have low immunogenicity, but also have the risk of being inserted into the host genome and inducing oncogenesis. Accordingly, there is a demand for RNA-based vaccines.

Disclosure

Technical Problem

An aspect provides an antigenic peptide in which two or more KRAS variant peptides selected from a group consisting of KRAS variant peptides are linked, wherein the antigenic peptide includes G12C, G12D, and G13D variant peptides.

Another aspect provides an mRNA encoding the antigenic peptide.

Another aspect provides a DNA encoding the antigenic peptide.

Another aspect provides an immunogenic composition including the antigenic peptide, or the mRNA encoding the same.

Another aspect provides a method of inducing an immune response to KRAS variant peptides in a subject, comprising administering to the subject the antigenic peptide, or the mRNA encoding the same.

Technical Solution

As used herein, with respect to a polypeptide or polynucleotide, the term "identity" refers to a relationship between the sequences of two or more polypeptides or polynucleotides as determined by comparing the sequences. The identity represents the degree of sequence relatedness, which is determined by the number of matches between strings of two or more amino acid residues or nucleotide residues. The identity of related polypeptides or polynucleotides may be calculated by known methods. The term "% identity" as applied to a polypeptide or polynucleotide is defined as the percentage of residues in the candidate amino acid sequence or nucleotide sequence that are identical with residues of a second sequence after aligning the candidate amino acid or nucleotide sequences with the second sequence, introducing gaps if necessary, to obtain the highest percent identity. Methods and programs for such alignments are known. Examples of such programs include BLAST, the Smith-Waterman algorithm, or the Needleman-Wunsch algorithm.

As used herein, the term "5'-untranslated region (5'-UTR)" refers to a region of an mRNA that is directly upstream (i.e., 5') from the initiation codon of the mRNA transcript, which is the first codon translated by the ribosome, and does not encode a polypeptide.

As used herein, the term "3'-untranslated region (3'-UTR)" refers to a region of an mRNA that is directly downstream (i.e., 3') from a stop codon, which signals translation termination in the mRNA transcript, and does not encode a polypeptide.

As used herein, the term "open reading frame (ORF)" refers to a contiguous region of nucleic acid that begins with an initiation codon, for example, a methionine codon (ATG), and ends with a stop codon, for example, TAA, TAG or TGA, and encodes a polypeptide.

As used herein, the term "polyadenylate sequence", or "polyA tail", refers to a region of an mRNA located downstream, for example directly downstream (i.e., 3') from a 3' UTR, that contains multiple consecutive adenosine monophosphates. The polyA tail may include 10 to 300 adenosine monophosphate units. For example, the polyA tails may include 10, 20, 30, 40, 50, 60, 70, 80, 90, 100, 110, 120, 130, 140, 150, 160, 170, 190, 200, 210, 220, 230, 240, 250, 260, 270, 280, 290, or 300 adenosine monophosphate units. The polyA tail may include, for example, 50 to 250 adenosine monophosphate units. In vivo, such as in a cell or an organism, the polyA tail may serve to protect the mRNA from enzymatic degradation, for example, by enzymes present in the cytoplasm, and may assist in transcription termination,

export of the mRNA from the nucleus, and translation.

As used herein, the term "operatively linked" refers to nucleotide sequences being joined on a single nucleic acid fragment such that one function is affected by the other. For example, a promoter is operably linked to a coding sequence (e.g., an ORF) when it may affect the expression of the coding sequence (i.e., the coding sequence is under the transcriptional control of the promoter). The coding sequence may be operably linked to a regulatory sequence in either the sense or antisense direction.

As used herein, unless otherwise stated with respect to the position of the nucleotide sequence, the sequence are connected or positioned in the direction from 5' to 3' ends.

As used herein, the term "vector" or "nucleic acid construct" refers to any nucleic acid capable of transporting a gene, ORF, or DNA fragment into a cell. The vector may, for example, be capable of replication within a cell. The vector may be an artificial chromosome such as a virus, bacteriophage, provirus, plasmid, phagemid, transposon, yeast artificial chromosome (YAC), bacterial artificial chromosome (BAC), or plant artificial chromosome (PLAC).

A first aspect provides an antigenic peptide in which two or more KRAS variant peptides selected from a group consisting of KRAS variant peptides are linked, wherein the antigenic peptide includes G12C, G12D, and G13D variant peptides.

The KRAS variant peptide may further include G12A, G12V, G12R, G12S, G13C, G13D, G13R, Q61H, Q61R, A146T, or a combination thereof. The KRAS variant peptide may have 7 to 50 amino acid residues. The variant peptide may include, relative to the wild-type sequence, a mutation residue, and also include an extension sequence 1 extending from the mutation residue toward the N-terminus by 3 to 23 amino acids and an extension sequence 2 extending from the mutation residue toward the C-terminus by 3 to 23 amino acids. The variant peptides may be, for example, Antigens 1 to 22 described in Table 1. The extension sequence 1 may be 3 to 12 amino acids in length, and the extension sequence 2 may be 3 to 23 amino acids in length, for example, 3 to 19 amino acids in length. The extension sequence 1 may be 3, 5, 7, 8, 9, 11 or 12 amino acids in length, and the extension sequence 2 may be 3, 4, 5, 8, 9, 11, 18 or 19 amino acids in length.

The antigen peptide may include a plurality of the variant peptides linked through peptide bonds. For example, the antigen peptide may be 3, 4, 5, 6, 7, 8, 9, 10, 11, 12, 13, 14, 15, 16, 17, 18, 19, 20, 21, or 22 of the variant peptides linked via peptide bonds. The linkage may be such that the variant peptides are directly connected or connected via a peptide linker. The peptide linker may be a G4S (SEQ ID NO: 552) or LLSVGG (SEQ ID NO: 553) linker. Each variant peptide included in the antigenic peptide may include mutation residues at the same position or at different positions. For example, the antigen peptide may include mutation residues at 1, 2, 3, or 4 mutation positions. The variation position may be C12, C13, Q61 or A146.

In an embodiment, the variant peptide may be 8, 17, or 31 amino acids (aa) in length. The antigen peptide may further include one or more of the G12V, G12R, G13C, G13R, and Q61H variant peptides.

In an embodiment, the antigen peptide may include G12C, G12D, G12V, G12R, G13C, G13D, and Q61H variant peptides, each of which may be 17 aa in length.

In an embodiment, the antigen peptide may include only variant peptides that are 8 aa or 31 aa in length. The antigen peptide may include linked variant peptides having the same mutation residue, with lengths of 8 aa and 31 aa. The antigen peptides may include G12C, G12D, G12V, G12R, G13C, G13D, G13R and Q61H variant peptides.

The antigen peptide may have a signal peptide linked to the N-terminus. The signal peptide may be a secretory signal peptide. The signal peptide may include an IgK signal peptide, an AZU1 signal peptide, a LAMP1 signal peptide, or an MHC-I signal peptide.

The antigenic peptide may be linked to an AMP1 transmembrane domain, an MHC-1 trafficking domain, a diphtheria toxin (DTT), a diphtheria toxin (DTT) domain (1-87 aa), or a diphtheria toxin (DTT) domain (96-177 aa). The antigenic peptide may be linked to an AMP1 transmembrane domain, diphtheria toxin (DTT), or a diphtheria toxin (DTT) domain (1-87 aa) at the N-terminus, and may be linked to an MHC-1 trafficking domain, or a diphtheria toxin (DTT) domain (96-177 aa) at the N-terminus.

The antigenic peptide may be any one of the amino acid sequences of SEQ ID NOs: 23 to 44.

A second aspect provides an mRNA encoding the antigenic peptide.

The mRNA may include an expression regulatory sequence or a sequence that influences stabilization. The expression regulatory sequence or stabilization-influencing sequences may be 5'-UTR, 3'-UTR, and/or polyA. The 5'-UTR, 3'-UTR, and polyA may be natural or non-natural sequences. These sequences may be operably linked to ORFs or to each other.

The 5'-UTR may have a sequence identity of at least 70%, for example, at least 80%, at least 90%, or at least 95%, with the nucleotide sequence of SEQ ID NO: 553. The 5'-UTR may include SEQ ID NO: 553.

The 5'UTR may be derived from an mRNA encoding a protein selected from the group consisting of p53, OX40L, albumin, serum amyloid A, Apolipoprotein A/B/E, transferrin, alpha fetoprotein, erythropoietin, Factor VIII, MyoD, Myosin, Myoglobin, Myogenin, Herculin, Tie-1, CD36, C/EBP, AML1, G-CSF, GM-CSF, CD1 lb, MSR, Fr-1, i-NOS, CD45, CD18, CD36, GLUT4, ACRP30, adiponectin, PA/B/C/D, ORM1, HPX, FGA, CYP2E1, C3, ASC, APOA2, ALB, AGXT, $\alpha$-globin, $\beta$-globin, tyrosine hydroxylase, and collagen.

The 3'-UTR may have a sequence identity of at least 70%, for example, at least 80%, at least 90%, or at least 95%, with the nucleotide sequence of SEQ ID NO: 554. The 3'-UTR may be derived from an mRNA encoding a protein selected from the group consisting of p53, OX40L, albumin, serum amyloid A, Apolipoprotein A/B/E, transferrin, alpha fetoprotein, erythropoietin, Factor VIII, MyoD, Myosin, Myoglobin, Myogenin, Herculin, Tie-1, CD36, C/EBP, AML1, G-CSF, GM-CSF, CD1 lb, MSR, Fr-1, i-NOS, CD45, CD18, CD36, GLUT4, ACRP30, adiponectin, PA/B/C/D, ORM1, HPX, FGA, CYP2E1, C3, ASC, APOA2, ALB, AGXT, α-globin, β-globin, tyrosine hydroxylase, and collagen.

The mRNA may include one or more modified nucleotides. The modified polynucleotide, when introduced into a cell or organism, may exhibit reduced degradation in the cell or organism compared to an unmodified one. Additionally, the modified polynucleotide, when introduced into a cell or organism, may exhibit reduced immunogenicity (e.g., reduced innate response) in the cell or organism compared to the unmodified one.

A nucleotides having a modified cytosine may include N4-acetyl-cytidine (ac4C), 5-methyl-cytidine (m5C), 5-halo-cytidine (eg, 5-iodo-cytidine), 5-hydroxymethyl-cytidine (hm5C), 1-methyl-pseudoisocytidine, 2-thio-cytidine (s2C), 2-thio-5-methyl-cytidine, or a combination thereof.

A nucleotide having a modified adenine may include 7-deaza-adenine, 1-methyl-adenosine (m1A), 2-methyl-adenine (m2A), N6-methyl-adenosine (m6A), or a combination thereof.

A nucleotide having a modified guanine may include inosine (I), 1-methyl-inosine (m1I), wyosine (imG), methylwyosine (mimG), 7-deaza-guanosine, 7-cyano-7-deaza-guanosine (preQ0), 7-aminomethyl-7-deaza-guanosine (preQ1), 7-methyl-guanosine (m7G), 1-methyl-guanosine (m1G), 8-oxo-guanosine, 7-methyl-8-oxo-guanosine, or a combination thereof.

A nucleotides having the chemical modification may be pseudouridine, N1-methylpseudouridine, N1-ethylpseudouridine, 2-thiouridine, 4'-thiouridine, 5-methylcytosine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, 2'-O-methyluridine, or a combination thereof. The chemical modification may be located at the 5' position of uracil. The chemical modification may be a modification of uracil to N1-methylpseudouridine. The chemical modification may be a modification of uracil to N1-ethylpseudouridine.

The mRNA may include a 5' cap. The term "5'-cap" refers to a cap structure found at the 5'-end of an mRNA molecule. The 5'-cap may include a guanosine nucleotide linked to the mRNA via an unusual 5' to 5' triphosphate linkage. The guanosine may be methylated at the 7-position, for example, m7G, or 3'-O-Me-m7G. The term "conventional 5' cap" refers to naturally occurring RNA 5' caps, such as 7-methylguanosine (m7G). As used herein, the term "5' cap" includes 5' cap analogs that resemble an RNA cap structure, and when attached to RNA, e.g., in vivo and/or in cells, are modified to have the ability to stabilize RNA. The provision of a 5' cap or 5' cap analog to RNA may be performed by in vitro transcription of a DNA template in the presence of the 5' cap or 5' cap analog, thereby co-transcriptionally inserting the 5' cap or 5' cap analog into the RNA strand from which it is produced, or by post-transcriptionally using a capping enzyme, such as the vaccinia virus capping enzyme. The 5'-terminal cap structure may be, for example, 7mG(5')ppp(5')ImpNp, 3'-O-Me-m7G(5')ppp(5')G, m7(3'OMeG)(5')ppp(5')(2'OMeA) or m7G(5')ppp(5')(2'OMeA)pG.

The mRNA may have one or more uridines (U) substituted with N1-methyl-pseudouridine. In the mRNA, the GA at the 5' end may have a cap structure of m7(3'OMeG)(5')ppp(5')(2'OMeA). In the mRNA, all U's may be substituted with N1-methyl-pseudouridine. The mRNA may have all U's substituted with N1-methyl-pseudouridine, and the GA at the 5' end may have a cap structure of m7(3'OMeG)(5')ppp(5')(2'OMeA).

The mRNA may be codon-optimized for a host cell. The codon optimization may vary depending on the host cell in which the mRNA is produced.

The mRNA may include any one of the nucleotide sequences of SEQ ID NOs: 1 to 22. Additionally, the mRNA may include any one of the nucleotide sequences of SEQ ID NOs: 1 to 23. Additionally, the mRNA may include any one of the nucleotide sequences of SEQ ID NOs: 296 to 300.

A third aspect provides DNA encoding the antigenic peptide.

The DNA may further include expression regulatory and restriction enzyme sites. The expression reguartory element may be a promoter. The expression regulatory element may be operably linked to the ORF or to another expression regulatory element.

The 3'-UTR coding sequence may include a recognition sequence for a restriction enzyme. The estriction enzyme may be Xhol, Nhel, or Xhol and Nhel. The promoter may be operably linked to a sequence encoding the 5'-UTR. The 5'-UTR, the antigen peptide coding sequence, and the 3'-UTR may be linked to form a common transcript when transcribed in vitro or in vivo. The sequence encoding the 3'-UTR may be operably linked to a polyA sequence.

The sequence encoding the 5'-UTR may be a sequence encoding the sequence of SEQ ID NO: 553, and the sequence encoding the 3'-UTR may include a sequence encoding the sequence of SEQ ID NO: 554. In this case, the sequence encoding the KRAS antigen peptide may have any one of the nucleotide sequences of SEQ ID NOs: 1 to 22 (including only the sequence of the coding region). Additionally, the polyA sequence may include the sequence of SEQ ID NO: 556.

As used herein, the term "nucleic acids capable of being transcribed to produce a common transcript" refers to nucleic acid sequences that are functionally or operatively linked to each other such that when transcribed, an RNA molecule is formed that includes a transcript in which the nucleic acid sequences are covalently linked to each other. The transcription may be performed, where appropriate, after linearization of the nucleic acid molecule, for example, by restriction enzyme digestion of a closed circular nucleic acid molecule, under the control of a promoter. The common transcript may, where appropriate, be separated by sequences located between the nucleic acid sequences.

The DNA molecule may be a closed circular molecule or a linear molecule.

The DNA molecule may further include one or more selected from the group consisting of (i) a reporter gene, (ii) a selectable marker, and (iii) an origion of replication. The DNA molecule may be a vector. The vector may be a cloning vector or an expression vector. The DNA molecule may be suitable for in vitro transcription of mRNA after linearization.

A fourth aspect provides an immunogenic composition including the antigen peptide or the mRNA encoding the antigen peptide as an active ingredient.

The antigen peptide and the mRNA encoding it are as described in the first aspect and the second aspect.

The composition may be for inducing an immune response against KRAS variant peptides. The composition may be for preventing or treating cancer. The subject to be prevented or treated may have a KRAS mutation. The mutation may have a G12C, G12D, G12V, G12R, G13C, G13D, G12R, Q61H, Q61R, A147R, or a combination mutation thereof.

The composition may be a pharmaceutical composition including a pharmaceutically acceptable carrier, diluent or excipient.

The composition may be for treating cancer. The cancer may be a cancer containing a KRAS mutation. The cancer may be a hematologic cancer or a solid tumor. The hematologic cancer may be leukemia, malignant lymphoma, multiple myeloma, or aplastic anemia. The solid tumor may be a brain tumor, benign astrocytoma, malignant astrocytoma, pituitary adenoma, meningioma, brain lymphoma, oligodendroglioma, intracranial tumor, ependymoma, brainstem tumor, head and neck tumor, laryngeal cancer, oropharyngeal cancer, nasal cancer, nasopharyngeal cancer, salivary gland cancer, hypopharyngeal cancer, thyroid cancer, oral cancer, thoracic tumor, lung cancer, thymic cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colon cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, female external genital cancer, female urethral cancer, or skin cancer. The lung cancer may be small cell lung cancer or non-small cell lung cancer.

The composition may be administered to a subject and the mRNA may be translated in vivo to produce the antigenic peptide.

The composition may be contacted with a cell, tissue or subject in an "effective amount" to induce an immune response.

The effective amount may be determined based on the target tissue, target cells, means of administration, properties of the mRNA, such as size and content of modified nucleosides, and other components of the composition.

The composition may be administered together with other prophylactic or therapeutic compounds. The prophylactic or therapeutic compound may be, for example, an anticancer agent. As used herein, the anticancer agent may be any known agent.

The composition may have a systemic or topical administration formulation. The topical administration may be administered locally to the tissue where the cancer exists. The tissue where the cancer is present may be intramuscular, subcutaneous, intradermal, nasal, or pulmonary.

The composition may include one or more pharmaceutically acceptable carriers, diluents or excipients. The mRNA in the composition may be formulated or complexed with the excipient. The carrier, diluent or excipient may be known in the art.

The relative amounts of the active ingredient, pharmaceutically acceptable excipients, and/or other additional ingredients included in the composition may vary depending on the nature, size, and/or condition of the subject to be treated and the route of administration. For example, the composition can comprise from 0.1 % to 100 %, for example, from 0.5 % to 50 %, from 1.0 % to 30 %, from 5.0 % to 80 %, or greater than 80 % (w/w) of the active ingredient.

The composition may be formulated as nanoparticles. The nanoparticles may be known to be capable of delivering mRNA into cells. For example, the nanoparticle may be a lipid nanoparticle (LNP). The composition may be formulated as a lipid nanoparticle (LNP) or bound to an LNP. The binding may include binding within or on the surface of the LNP. For example, the composition may be formulated within a lipid polycation complex or bound to a lipid polycation complex. The lipid polycation complex is also referred to as a cationic lipid nanoparticle. The polycation may include a cationic polypeptide, such as MC3, Lipid 319, C12-200, 5A2-SC8, 306Oi10, Moderna Lipid 5, Acuitas A9, SM-102, ALC-0315, Arcturus Lipid 2,2 (8,8) 4C CH3, Genevant CL1, polylysine, polyornithine, and/or polyarginine. Additionally, the composition may be formulated within or bound to a lipid nanoparticles including non-cationic lipids such as distearoylphosphatidylcholine (DSPC), 1,2-distearoyl-sn-glycero-3-phosphocholine (DSPC), dipalmitoylphosphatidylcholine (DPPC), sterols such as cholesterol, or dioleoylphosphatidylethanolamine (DOPE). Additionally, the composition may be formulated with or bound to lipid nanoparticles comprising PEG-lipids such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), polyethylene glycol dimethacrylate (PEG-DMA).

The lipid nanoparticle formulations may include a cationic lipid, a phospholipid, a sterol such as cholesterol, a PEG-lipid, or a combination thereof. The lipid nanoparticle formulations may include, for example, a cationic lipid, a phospholipid, a sterol such as cholesterol, and a PEG-lipid. Additionally, the lipid nanoparticles may include a PEG-modified lipid, a non-cationic lipid, a sterol, and an ionizable lipid, or a combination thereof. The lipid nanoparticle may include 0.5 mol% to 15 mol % PEG-modified lipid, 5 mol% to 25 mol % non-cationic lipid, 25 mol% to 55 mol % sterol, and 20 mol% to 60 mol % ionizable lipid. The PEG-modified lipid may be 1,2 dimyristoyl-sn-glycerol methoxypolyethylene glycol (PEG2000-DMG), the non-cationic lipid may be 1,2 distearoyl-sn-glycerol-3-phosphocholine (DSPC), the sterol may be cholesterol, and the ionizable cationic lipid may be Compound 1 having the following structure.

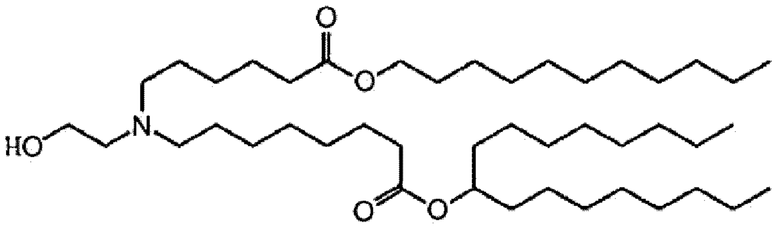

Compound 1

The PEG-modified lipid may be Compound 2 (ALC-0159) having the following structure, the non-cationic lipid may be 1,2 distearoyl-sn-glycerol-3-phosphocholine (DSPC), the sterol may be cholesterol, and the ionizable cationic lipid may be Compound 3 (ALC-0315) having the following structure.

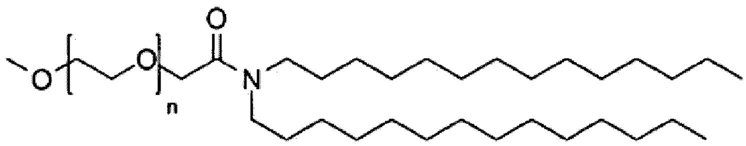

Compound 2

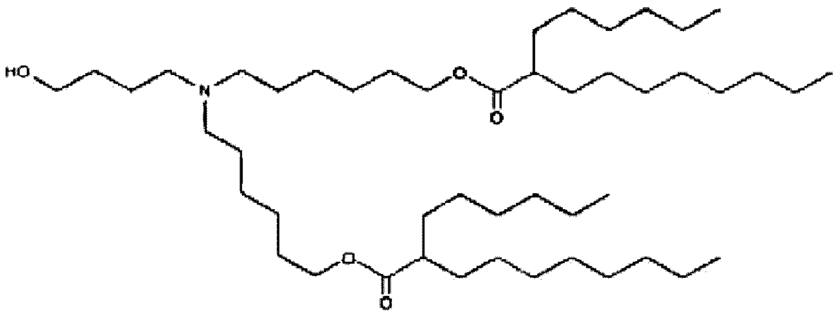

Compound 3

A fifth aspect provides a method of inducing an immune response in a subject including administering the antigenic peptide or the mRNA to the subject. The method may be for preventing or treating cancer.

The administration may be administered in an amount effective to induce an immune response in the subject. The administration may be administered in an amount effective to prevent or treat cancer in the subject. The administration may be systemic or topical. The topical administration may be administered locally to the tissue where the cancer is present. The tissue where the cancer is present may be intramuscular, subcutaneous, intradermal, nasal, or pulmonary. The administration may be parenteral or oral.

The subject may be a mammal, including a human. The administration may be local administration of the mRNA molecule either intracellularly or extracellularly to a tissue where cells with high levels of KRAS mutations are present.

The method may be to produce the antigen peptide in cells of an individual to inhibit cancer cell proliferation. Additionally, the method may be for treating a disease in the subject. The antigenic peptide may induce an immune response against cancer in the subject.

Advantageous Effects

The antigenic peptides in which two or more KRAS variant peptides are linked according to an aspect may be used to efficiently induce an immune response in a subject.
The mRNA encoding the antigenic peptide according to an aspect may be used to efficiently induce an immune response in a subject.
The DNA encoding the antigenic peptide according to an aspect may be used to produce the antigenic peptide.
The immunogenic composition according to an aspect may be used to induce an immune response in a subject.
According to a method of inducing an immune response to a KRAS variant peptide in a subject according to an aspect, an immune response to a KRAS variant peptide may be efficiently induced in the subject.

Description of Drawings

FIG. 1 is a diagram showing a vector obtained through the same process as in Examples 1 and 2.
FIG. 2 is a diagram showing tumor size and body weight changes after administration of an anticancer vaccine containing KRAS mRNA encoding Antigen 2 in a Lewis lung cancer mouse model.
FIG. 3 is a diagram showing changes in immune cell distribution within tumor tissue cells after administration of an anticancer vaccine containing KRAS mRNA encoding Antigen 2 in a Lewis lung cancer mouse model.
FIG. 4 is a diagram showing tumor size and body weight changes after administration of an anticancer vaccine containing KRAS mRNA encoding Antigen 6 in a Lewis lung cancer mouse model.
FIG. 5 is a diagram showing changes in CD8 T cell frequency among immune cells in tumor tissue after administration of an anticancer vaccine containing KRAS mRNA encoding Antigen 6 in a Lewis lung cancer mouse model.
FIG. 6 is a diagram showing tumor size and body weight changes after administration of an anticancer vaccine containing KRAS mRNA encoding Antigen 14 or 15 in a Lewis lung cancer mouse model.
FIG. 7A is a photograph showing the results of co-culturing PBMCs expressing KRAS variant peptide-linked Antigens 2 and 6 with target cells.
FIG. 7B is a diagram showing the results of measuring target cell viability when PBMCs expressing KRAS variant peptide-linked Antigens 2 and 6 are co-cultured with target cells.
FIG. 8A is a photograph showing the results of co-culturing PBMCs expressing KRAS variant peptide-linked Antigens 15 and 17 with target cells.
FIG. 8B is a diagram showing the results of measuring the viability of target cells when PBMCs expressing KRAS variant peptide-linked Antigens 15 and 17 are co-cultured with the target cells.
FIG. 9 is a diagram showing the results of a Western blot using an anti-RAS (G12D mutant) monoclonal antibody against proteins expressed in LL/2 cells, which are a Lewis lung carcinoma cell line, into which mRNAs encoding KRAS variant peptide-linked Antigens 6, 14, and 15 were introduced.

Best Mode

Hereinafter, the present disclosure will be described in more detail through examples. However, these examples are intended to exemplify the present disclosure and the scope of the present disclosure is not limited to these examples.

**Example 1: Anticancer agent sequences designed to enhance T cell activation against KRAS mutant sequences**

An antigen including multiple KRAS variant peptides was designed for the treatment of cancer patients with KRAS mutations. The variant peptides include a mutation residue based on the KRAS wild-type sequence (SEQ ID NO: 557) and includes an extension sequence 1 extending from the mutation residue toward the N-terminus, and an extension sequence 2 extending from the mutation residue toward the C-terminus. The mutation residues include G12A, G12C, G12D, G12V, G12R, G12S, G13C, G13D, G13R, Q61H, Q61R or A146T. Extended sequence 1 may have a length of 3 to 23 amino acids. Extended sequence 2 may have a length of 3 to 23 amino acids.
The variant peptide may be represented as E1-M-E2, where M represents the mutation residue, and E1 and E2 represent an extension sequence 1 extending from the mutation residue to the N-terminus and an extension sequence 2 extending from the mutation residue to the C-terminus, respectively. In the variant peptide E1-M-E2, M may be G12A, G12C, G12D, G12V, G12R, G12S, G13C, G13D, Q61H, Q61R or A146T, E1 may be 3 to 12 amino acids in length, and E2 may be 3 to 23, for example, 3 to 19 amino acids in length. E1 may have a length of 3, 5, 7, 8, 9, 11, or 12 amino acids, and E2 may have a length of 3, 4, 5, 8, 9, 11, 18, or 19 amino acids.

The antigen peptide may include a plurality of the variant peptides linked through peptide bonds. For example, the antigen peptide may include 3, 4, 5, 6, 7, 8, 9, 10, 11, 12, 13, 14, 15, 16, 17, 18, 19, 20, 21, or 22 variant peptides linked via peptide bonds. The linkage may be direct between the variant peptides or via peptide linkers. The peptide linker may be a G4S linker (SEQ ID NO: 552) or an LLSVGG linker (SEQ ID NO: 553). Each variant peptide included in the antigen peptide may include mutation residues at the same position or at different positions. For example, the antigenic peptide may include mutation residues at 1, 2, 3, or 4 mutation positions. The mutation position may be C12, C13, Q61 or A146.

Additionally, the antigenic peptides may all contain either the same mutation residue at the same mutation position, or different mutation residues at the same mutation position.

The antigenic peptide may be linked to a secretory signal peptide. The secretory signal peptide may be removed during the secretion process. The antigenic peptide may be linked to a secretory signal peptide at its N-terminus. The signal peptide may be an IgK signal peptide, an AZU1 signal peptide, a LAMP1 signal peptide, or an MHC-I signal peptide. Additionally, the antigenic peptide may be linked to a LAMP1 transmembrane domain, an MHC-1 trafficking domain, a diphtheria toxin (DTT) domain (1-87aa), or a diphtheria toxin (DTT) domain (96-177aa). The antigenic peptide may be linked at is N-terminus to a LAMP1 transmembrane domain, an MHC-1 trafficking domain, or a diphtheria toxin (DTT) domain (1-87aa). The antigenic peptide may be linked at its C-terminus to a diphtheria toxin (DTT) domain (96-177aa).

Table 1 shows the KRAS variant peptide composition and corresponding amino acid sequences (SEQ ID NOs: 23 to 44) of Antigens 1 to 22 prepared in this example. In Table 1, SEQ ID NOs: 1 to 22 listed under the column titled "KRAS Variant Peptide Composition" represent the nucleotide sequences of the ORF mRNA for each antigen peptide.

[Table 1]

| Antige n | Peptide name | Amino acid sequence |
|---|---|---|
| 1 | (11-G12R-11) (9-G12D-9) (9-G12V-9) (9-G12C-9) (9-G13D-9) (9-G13C-9) (9-Q61H-9) (SEQ ID NO: 1) | MTEYKLVVVGARGVGKSALTIQLEYKLVVVGADGVGKSALTIEYKLVVVGAVGVGKSALTIEYKLVVVGACGVGKSALTIYKLVVVGAGDVGKSALTIQYKLVVVGAGCVGKSALTIQLLDILDTAGHEEYSAMRDQ (SEQ ID NO: 23) |
| 2 | (8-G12C-8) (8-G12D-8) (8-G12V-8) (8-G12R-8) (8-G13C-8) (8-G13D-8) (8-Q61H-8) (SEQ ID NO: 2) | YKLVVVGACGVGKSALTYKLVVVGADGVGKSALTYKLVVVGAVGVGKSALTYKLVVVGARGVGKSALTKLVVVGAGCVGKSALTIKLVVVGAGDVGKSALTILDILDTAGHEEYSAMRD (SEQ ID NO: 24) |
| 3 | -(8-G12C-8)-(8-G12D-8)-(8-G12V-8)-(8-G12R-8)-(8-G13C-8)-(8-G13D-8)-(8-Q61H-8)- (SEQ ID NO: 3) | LLSVGGYKLVVVGACGVGKSALTLLSVGGYKLVVVGADGVGKSALTLLSVGGYKLVVVGAVGVGKSALTLLSVGGYKLVVVGARGVGKSALTLLSVGGKLVVVGAGCVGKSALTILLSVGGKLVVVGAGDVGKSALTILLSVGGLDILDTAGHEEYSAMRDLLSVGG (SEQ ID NO: 25) |

(continued)

| Antige n | Peptide name | Amino acid sequence |
|---|---|---|
| 4 | (3-G12C-5)-(5-G12C-3)-(3-G12C-5)-(5-G12C-3)-(3-G12C-5)-(5-G12C-3) (SEQ ID NO: 4) | VGACGVGKSLLSVGGVVVGACGVGLLSVGGVGACGVGKSLLSVGGVVVGACGVGLLSVGGVGACGVGKSLLSVGGVVVGACGVG (SEQ ID NO: 26) |
| 5 | (3-G12C-5)-(5-G12C-3)-(3-G12D-5)-(5-G12D-3)-(3-G13D-5)-(5-G13D-3) (SEQ ID NO: 5) | VGACGVGKSLLSVGGVVVGACGVGLLSVGGVGADGVGKSLLSVGGVVVGADGVGLLSVGGGAGDVGKSALLSVGGVVGAGDVGK (SEQ ID NO: 27) |
| 6 | (11-G12C-19)-(3-G12C-4)-(11-G12D-19)-(3-G12D-4)-(11-G13D-19)-(3-G13D-4) (SEQ ID NO: 6) | MTEYKLVVVGACGVGKSALTIQLIQNHFVDELLSVGGVGACGVGKLLSVGGMTEYKLVVVGADGVGKSALTIQLIQNHFVDELLSVGGVGADGVGKLLSVGGTEYKLVVVGAGDVGKSALTIQLIQNHFVDEYLLSVGGGAGDVGKS (SEQ ID NO: 28) |
| 7 | (12-G13C-18)-(11-G12C-19)-(11-G12D-19)-(12-G13D-18)-(11-G12V-19)-(11-Q61H-19)(SEQ ID NO: 7) | MTEYKLVVVGAGCVGKSALTIQLIQNHFVDELLSVGGMTEYKLVVVGACGVGKSALTIQLIQNHFVDELLSVGGMTEYKLVVVGADGVGKSALTIQLIQNHFVDELLS VGGMTEYKLVVVGAGDVGKSALTIQLIQNHFVDELLSVGGMTEYKLVVVGAVGVGKSALTIQLIQNHFVDELLSVGGTCLLDILDTAGHEEYSAMRDQYMRTGEGFLC (SEQ ID NO: 29) |
| 8 | (11-G12C-19) (3-G12C-4) (11-G12D-19) (3-G12D-4) (11-G13D-19) (3-G13D-4) (SEQ ID NO: 8) | MTEYKLVVVGACGVGKSALTIQLIQNHFVDEVGACGVGKMTEYKLVVVGADGVGKSALTIQLIQNHFVDEVGADGVGKTEYKLVVVGAGDVGKSALTIQLIQNHFVDEYGAGDVGKS (SEQ ID NO: 30) |
| 9 | DTT (12-G13C-18)-(11-G12C-19)-(11-G12D-19)-(12-G13D-18)-(11- | CINLDWDVIRDKTKTKIESLKEHGPIKNKMSESPNKTVSEEKAKQYLEEFHQTALEHPELSELKTVTGTNPVFAGANYAAWAVNVAQVIDSETADNLEKTTA |

(continued)

| Antige n | Peptide name | Amino acid sequence |
|---|---|---|
| | G12V-19)-(11-Q61H-19) (SEQ ID NO: 9) | ALSILPGIGSVMGIADGAVHHNTEEIVAQSIALSSLMVAQAIPLVGELVDIGFAAYNFVESIINLFQVVHNSYNRMTEYKLVVVGAGCVGKSALTIQLIQNHFVDELLSVGGMTEYKLVVVGACGVGKSALTIQLIQNHFVDELLSVGGMTEYKLVVVGADGVGKSALTIQLIQNHFVDELLSVGGMTEYKLVVVGAGDVGKSALTIQLIQNHFVDELLSVGGMTEYKLVVVGAVGVGKSALTIQLIQNHFVDELLSVGGTCLLDILDTAGHEEYSAMRDQYMRTGEGFLC (SEQ ID NO: 31) |
| 10 | (12-G13C-18)-(11-G12C-19)-(11-G12D-19)-(12-G13D-18)-(11-G12V-19)-(11-Q61H-19)-DTT (SEQ ID NO: 10) | MTEYKLVVVGAGCVGKSALTIQLIQNHFVDELLSVGGMTEYKLVVVGACGVGKSALTIQLIQNHFVDELLSVGGMTEYKLVVVGADGVGKSALTIQLIQNHFVDELLSVGGMTEYKLVVVGAGDVGKSALTIQLIQNHFVDELLSVGGMTEYKLVVVGAVGVGKSALTIQLIQNHFVDELLSVGGTCLLDILDTAGHEEYSAMRDQYMRTGEGFLCLLSVGGCINLDWDVIRDKTKTKIESLKEHGPIKNKMSESPNKTVSEEKAKQYLEEFHQTALEHPELSELKTVTGTNPVFAGANYAAWAVNVAQVIDSETADNLEKTTAALSILPGIGSVMGIADGAVHHNTEEIVAQSIALSSLMVAQAIPLVGELVDIGFAAYNFVESIINLFQVVHNSYNR (SEQ ID NO: 32) |
| 11 | DTT (12-G13C-18)-(11-G12C-19)-(11-G12D-19)-(12-G13D-18)-(11-G12V-19)-(11-Q61H-19) DTT (SEQ ID NO: 11) | MTEYKLVVVGAGCVGKSALTIQLIQNHFVDELLSVGGMTEYKLVVVGACGVGKSALTIQLIQNHFVDELLSVGGMTEYKLVVVGADGVGKSALTIQLIQNHFVDELLSVGGMTEYKLVVVGAGDVGKSALTIQLIQNHFVDELLSVGGMTEYKLVVVGAVGVGKSALTIQLIQNHFVDELLSVGGTCLLDILDTAGHEEYSAMRDQYMRTGEGFLCLLSVGGCINLDWDVIRDKTKTK |

(continued)

| Antige n | Peptide name | Amino acid sequence |
|---|---|---|
| | | IESLKEHGPIKNKMSESPNKTVSEEKAKQYLEEFHQTALEHPELSELKTVTGTNPVFAGANYAAWAVNVAQVIDSETADNLEKTTAALSILPGIGSVMGIADGAVHHNTEEIVAQSIALSSLMVAQAIPLVGELVDIGFAAYNFVESIINLFQVVHNSYNR (SEQ ID NO: 33) |
| 12 | (8-G12C-8) (8-G12D-8) (8-G12V-8) (8-G12R-8) (8-G13C-8) (8-G13D-8) (8-Q61H-8) (8-G13R-8) (SEQ ID NO: 12) | YKLVVVGACGVGKSALTYKLVVVGADGVGKSALTYKLVVVGAVGVGKSALTYKLVVVGARGVGKSALTKLVVVGAGCVGKSALTIKLVVVGAGDVGKSALTILDILDTAGHEEYSAMRDKLVVVGAGRVGKSALTI (SEQ ID NO: 34) |
| 13 | (11-G12C-19)-(3-G12C-4)-(11-G12D-19)-(3-G12D-4)-(11-G12V-19)-(3-G12V-4)-(11-G12R-19)-(3-G12R-4)-(11-G13C-19)-(3-G13C-4)-(11-G13D-19)-(3-G13D-4)-(11-Q61H-19)-(3-Q61H-4)-(11-G13R-19)-(3-G13R-4) (SEQ ID NO: 13) | MTEYKLVVVGACGVGKSALTIQLIQNHFVDELLSVGGVGACGVGKLLSVGGMTEYKLVVVGADGVGKSALTIQLIQNHFVDELLSVGGVGADGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTEYKLVVVGAGCVGKSALTIQLIQNHFVDEYLLSVGGGAGCVGKSLLSVGGTEYKLVVVGAGDVGKSALTIQLIQNHFVDEYLLSVGGGAGDVGKSLLSVGGTCLLDILDTAGHEEYSAMRDQYMRTGEGFLCLLSVGGTAGHEEYSLLSVGGTEYKLVVVGAGRVGKSALTIQLIQNHFVDEYLLSVGGGAGRVGKS (SEQ ID NO: 35) |
| 14 | (11-G12C-19) (3-G12C-4) (11-G12D-19) (3-G12D-4) (11-G12V-19) (3-G12V-4) (11-G12R-19) (3-G12R-4) (11- | MTEYKLVVVGACGVGKSALTIQLIQNHFVDEVGACGVGKMTEYKLVVVGADGVGKSALTIQLIQNHFVDEVGADGVGKMTEYKLVVVGAVGVGKSALTIQLIQNHFVDEVGAVGVGKMTEYKLVVVGARGVGKSALTIQLIQNHFVDEVGARGVGKTEYKLVVVGAGC |

(continued)

| Antige n | Peptide name | Amino acid sequence |
|---|---|---|
| | G13C-19) (3-G13C-4) (11-G13D-19) (3-G13D-4) (11-Q61H-19) (3-Q61H-4) (11-G13R-19) (3-G13R-4) (SEQ ID NO: 14) | VGKSALTIQLIQNHFVDEYGAGCVGKSTEYKLVVVGAGDVGKSALTIQLIQNHFVDEYGAGDVGKSTCLLDILDTAGHEEYSAMRDQYMRTGEGFLCTAGHEEYSTEYKLVVVGAGRVGKSALTIQLIQNHFVDEYGAGRVGKS (SEQ ID NO: 36) |
| 15 | (11-G12D-19)-(3-G12D-4)-(11-G12C-19)-(3-G12C-4)-(11-G12V-19)-(3-G12V-4)-(11-G12R-19)-(3-G12R-4)-(11-G13C-19)-(3-G13C-4)-(11-G13D-19)-(3-G13D-4)-(11-Q61H-19)-(3-Q61H-4)-(11-G13R-19)-(3-G13R-4) (SEQ ID NO: 15) | MTEYKLVVVGADGVGKSALTIQLIQNHFVDELLSVGGVGADGVGKLLSVGGMTEYKLVVVGACGVGKSALTIQLIQNHFVDELLSVGGVGACGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTEYKLVVVGAGCVGKSALTIQLIQNHFVDEYLLSVGGGAGCVGKSLLSVGGTEYKLVVVGAGDVGKSALTIQLIQNHFVDEYLLSVGGGAGDVGKSLLSVGGTCLLDILDTAGHEEYSAMRDQYMRTGEGFLCLLSVGGTAGHEEYSLLSVGGTEYKLVVVGAGRVGKSALTIQLIQNHFVDEYLLSVGGGAGRVGKS (SEQ ID NO: 37) |
| 16 | (8-G12C-8) (8-G12D-8) (8-G12V-8) (8-G12R-8) (8-G13C-8) (8-G13D-8) (8-Q61H-8) (8-G13R-8)(8-G12C-8) (8-G12D-8) (8-G12V-8) (8-G12R-8) (8-G13C-8) (8-G13D-8) (8-Q61H-8) (8-G13R-8) | YKLVVVGACGVGKSALTYKLVVVGADGVGKSALTYKLVVVGAVGVGKSALTYKLVVVGARGVGKSALTKLVVVGAGCVGKSALTIKLVVVGAGDVGKSALTILDILDTAGHEEYSAMRDKLVVVGAGRVGKSALTIYKLVVVGACGVGKSALTYKLVVVGADGVGKSALTYKLVVVGAVGVGKSALTYKLVVVGARGVGKSALTKLVVVGAGCVGKSALTILDILDTAGHEEYSAMRDKLVVVGAGDVGKSALTIKLVVVGAGRVGKSALTIYKLVVVGAVGVGKSALTYKLVVVGARGVGKSALTKLVVVGAGCVGKSALTILDILDTAGHEEYSAMR |

(continued)

| Antige n | Peptide name | Amino acid sequence |
|---|---|---|
| | (8-G12C-8) (8-G12D-8) (8-G12V-8) (8-G12R-8) (8-G13C-8) (8-G13D-8) (8-Q61H-8) (8-G13R-8) (SEQ ID NO: 16) | DYKLVVVGACGVGKSALTYKLVVVGADGVGKSALTKLVVVGAGDVGKSALTIKLVVVGAGRVGKSALTI (SEQ ID NO: 38) |
| 17 | (11-G12D-19)-(3-G12D-4)-(11-G12C-19)-(3-G12C-4)-(11-G12V-19)-(3-G12V-4)-(11-G12R-19)-(3-G12R-4)-(11-G13C-19)-(3-G13C-4)-(11-G13D-19)-(3-G13D-4)-(11-Q61H-19)-(3-Q61H-4) (SEQ ID NO: 17) | MTEYKLVVVGADGVGKSALTIQLIQNHFVDELLSVGGVGADGVGKLLSVGGMTEYKLVVVGACGVGKSALTIQLIQNHFVDELLSVGGVGACGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTEYKLVVVGAGCVGKSALTIQLIQNHFVDEYLLSVGGGAGCVGKSLLSVGGTEYKLVVVGAGDVGKSALTIQLIQNHFVDEYLLSVGGGAGDVGKSLLSVGGTCLLDILDTAGHEEYSAMRDQYMRTGEGFLCLLSVGGTAGHEEYS (SEQ ID NO: 39) |
| 18 | (11-G12D-19)-(3-G12D-4)-(11-G12C-19)-(3-G12C-4)-(11-G12V-19)-(3-G12 V-4)-(11-G12R-19)-(3-G12R-4)-(11-Q61H-19)-(3-Q61H-4)-(11-G13D-19)-(3 -G13D-4)-(11-G12A-19)-(3-G12A-4)-(11-A146T-19)-(3-A146T-4)-(11-G13C-19)-(3-G13C-4)-(11-G12S-19)-(3-G12S-4)- | MTEYKLVVVGADGVGKSALTIQLIQNHFVDELLSVGGVGADGVGKLLSVGGMTEYKLVVVGACGVGKSALTIQLIQNHFVDELLSVGGVGACGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTCLLDILDTAGHEEYSAMRDQYMRTGEGFLCLLSVGGTAGHEEYSLLSVGGTEYKLVVVGAGDVGKSALTIQLIQNHFVDEYLLSVGGGAGDVGKSLLSVGGMTEYKLVVVGAAGVGKSALTIQLIQNHFVDELLSVGGVGAAGVGKLLSVGGRSYGIPFIETSTKTRQRVEDAFYTLVREIRQLLSVGGETSTKTRQLLSVGGTEYKLVVVGAGCVGKSALTIQLIQNHFVD |

(continued)

| Antige n | Peptide name | Amino acid sequence |
|---|---|---|
| | (11-Q61R-19)-(3-Q61R-4) (SEQ ID NO: 18) | EYLLSVGGGAGCVGKSLLSVGGMTEYKLVVVGASGVGKSALTIQLIQNHFVDELLSVGGVGASGVGKLLSVGGTCLLDILDTAGREEYSAMRDQYMRTGEGFLCLLSVGGTAGREEYS (SEQ ID NO: 40) |
| 19 | (11-G12C-19)-(3-G12C-4)-(11-G12V-19)-(3-G12V-4)-(11-G12D-19)-(3-G12D-4)-(11-Q61H-19)-(3-Q61H-4)-(11-G13C-19)-(3-G13C-4)-(11-G13D-19)-(3-G13D-4)-(11-G12A-19)-(3-G12A-4)-(11-G12S-19)-(3-G12S-4) (SEQ ID NO: 19) | MTEYKLVVVGACGVGKSALTIQLIQNHFVDELLSVGGVGACGVGKLLSVGGMTEYKLVVVGAVGVGKSALTIQLIQNHFVDELLSVGGVGAVGVGKLLSVGGMTEYKLVVVGADGVGKSALTIQLIQNHFVDELLSVGGVGADGVGKLLSVGGTCLLDILDTAGHEEYSAMRDQYMRTGEGFLCLLSVGGTAGHEEYSSLLSVGGTEYKLVVVGAGCVGKSALTIQLIQNHFVDEYLLSVGGGAGCVGKSLLSVGGTEYKLVVVGAGDVGKSALTIQLIQNHFVDEYLLSVGGGAGDVGKSLLSVGGMTEYKLVVVGAAGVGKSALTIQLIQNHFVDELLSVGGVGAAGVGKLLSVGGMTEYKLVVVGASGVGKSALTIQLIQNHFVDELLSVGGVGASGVGK (SEQ ID NO: 41) |
| 20 | (11-G12D-19)-(3-G12D-4)-(11-G12V-19)-(3-G12V-4)-(11-G12R-19)-(3-G12R-4)-(11-Q61H-19)-(3-Q61H-4)-(11-Q61R-19)-(3-Q61R-4)-(11-G13C-19)-(3-G13C-4)-(11-G12C-19)-(3-G12C-4)-(11-G13D-19)-(3-G13D-4) (SEQ ID NO: 20) | MTEYKLVVVGADGVGKSALTIQLIQNHFVDELLSVGGVGADGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTCLLDILDTAGHEEYSAMRDQYMRTGEGFLCLLSVGGTAGHEEYSSLLSVGGTCLLDILDTAGREEYSAMRDQYMRTGEGFLCLLSVGGTAGREEYSLLSVGGTEYKLVVVGAGCVGKSALTIQLIQNHFVDEYLLSVGGGAGCVGKSLLSVGGMTEYKLVVVGACGVGKSALTIQLIQNHFVDELLSVGGVGACGVGKLLSVGGTEYKLVVVGAGDVGKSALTIQLIQNHFVDEYLLSVGGGAGDVGKS (SEQ ID NO: 42) |

(continued)

| Antige n | Peptide name | Amino acid sequence |
|---|---|---|
| 21 | (11-G12D-19)-(3-G12D-4)-(11-G12V-19)-(3-G12V-4)-(11-G13D-19)-(3-G13D-4)-(11-A146T-19)-(3-A146T-4)-(11-G12C-19)-(3-G12C-4)-(11-G12A-19)-(3-G12A-4)-(11-G12S-19)-(3-G12S-4)-(11-G13C-19)-(3-G13C-4) (SEQ ID NO: 21) | MTEYKLVVVGADGVGKSALTIQLIQNHFVDELLSVGGVGADGVGKLLSVGGMTEYKLVVVGAVGVGKSALTIQLIQNHFVDELLSVGGVGAVGVGKLLSVGGTEYKLVVVGAGDVGKSALTIQLIQNHFVDEYLLSVGGGAGDVGKSLLSVGGRSYGIPFIETSTKTRQRVEDAFYTLVREIRQLLSVGGETSTKTRQLLSVGGMTEYKLVVVGACGVGKSALTIQLIQNHFVDELLSVGGVGACGVGKLLSVGGMTEYKLVVVGAAGVGKSALTIQLIQNHFVDELLSVGGVGAAGVGKLLSVGGMTEYKLVVVGASGVGKSALTIQLIQNHFVDELLSVGGVGASGVGKLLSVGGTEYKLVVVGAGCVGKSALTIQLIQNHFVDEYLLSVGGGAGCVGKS (SEQ ID NO: 43) |
| 22 | (11-G12D-19)-(3-G12D-4)-(11-G12C-19)-(3-G12C-4)-(11-G12V-19)-(3-G12V-4)-(11-G12R-19)-(3-G12R-4)-(11-G13C-19)-(3-G13C-4)-(11-G13D-19)-(3-G13D-4)-(11-Q61H-19)-(3-Q61H-4) (SEQ ID NO: 22) | MTEYKLVMVGADGVGKSALTIQLIQNHFVDELLSVGGVGADGVGKLLSVGGMTEYKLVVVGACGVGKSALTIQLIQNHFVDELLSVGGVGACGVGKLLSVGGMTEYKLVVVGAVGVGKSALTIQLIQNHFVDELLSVGGVGAVGVGKLLSVGGMTEYKLVVVGARGVGKSALTIQLIQNHFVDELLSVGGVGARGVGKLLSVGGTEYKLVVVGAGCVGKSALTIQLIQNHFVDEYLLSVGGGAGCVGKSLLSVGGTEYKLVVVGAGDVGKSALTIQLIQNHFVDEYLLSVGGGAGDVGKSLLSVGGTCLLDILDTAGHEEYSAMRDQYMRTGEGFLCLLSVGGTAGHEEYS (SEQ ID NO: 44) |

Open reading frames (ORFs) encoding the antigenic peptides listed in Table 1 (SEQ ID NOs: 23 to 44) were synthesized with the 5'-UTR and/or a signal sequence linked thereto. The synthesized sequences were designed to include HindIII and XhoI restriction enzyme sites at the 5' end and 3' end, respectively, to allow for cloning. Each synthesized sequence encoding the antigens was digested with HindIII and XhoI along with a template vector, and ligated using DNA ligase to generate a vector including a sequence encoding "mRNA." The KRAS variant peptide compositions of Antigens 1 to 22 are as shown in Table 1. Each variant peptide is separated by parentheses, and the presence of linkers between variant peptides is indicated by a hyphen ("-"). The linker indicated by a hyphen represents a cathepsin cleavage linker having the amino acid sequence 'LLSVGG' (SEQ ID NO: 553). In antigenic sequences where no hyphen is present and the variant peptides are linked in sequenceorder, this indicates that the variant peptides are directly linked without a linker.

The full-length mRNAs encoding antigens 1 to 22 shown in Table 1 each have the structure of 5'UTR-ORF-stop codon-3'UTR-polyA. Table 2 shows the variant peptides contained in Antigen 1 and the mRNA sequence thereof.

[Table 2]

| Peptide name | Length (aa) | mRNA sequence | Peptide |
|---|---|---|---|
| 11-G12R-11 | 23 | AUGACAGAGUACAAGCUGGGUGGUCGUGGGGGCCAGGGGCGUGGGCAAGAGCGCCCUGACUAUCCAGCUG (SEQ ID NO: 45) | MTEYKLVVVGARGVGKSALTIQL (SEQ ID NO: 301) |
| 9-G12D-9 | 19 | GAAUACAAGCUGGUGGUGGUGGGCGCCGACGGCGUGGGGAAGUCCGCCCUCACAAUC (SEQ ID NO: 46) | EYKLVVVGADGVGKSALTI (SEQ ID NO: 302) |
| 9-G12V-9 | 19 | GAGUACAAGCUGGUCGUGGUGGGGGCCGUGGGCGUCGGGAAGUCCGCCCUGACAAUC (SEQ ID NO: 47) | EYKLVVVGAVGVGKSALTI (SEQ ID NO: 303) |
| 9-G 12C-9 | 19 | GAGUACAAGUUGGUGGUGGUGGGCGCCUGCGGGGUGGGCAAGAGCGCCCUGACCAUC (SEQ ID NO: 48) | EYKLVVVGACGVGKSALTI (SEQ ID NO: 304) |
| 9-G13D-9 | 19 | UACAAGCUCGUGGUGGUGGGGGCCGGGGACGUGGGGAAGAGUGCCCUGACCAUCCAG (SEQ ID NO: 49) | YKLVVVGAGDVGKSALTIQ (SEQ ID NO: 305) |
| 9-G 13C-9 | 19 | UAUAAGCUGGUGGUGGUCGGCGCCGGGUGCGUGGGCAAGUCCGCCCUGACAAUCCAG (SEQ ID NO: 50) | YKLVVVGAGCVGKSALTIQ (SEQ ID NO: 306) |

(continued)

| Peptide name | Length (aa) | mRNA sequence | Peptide |
|---|---|---|---|
| 9-Q61H-9 | 19 | CUGCUGGAUAUCCUGGGAAUACCGCCGGCCACGAGGAGUACAGCGCCAUGAGGGAUCAG (SEQ ID NO: 51) | LLDILDTAGHEEYSAMRDQ (SEQ ID NO: 307) |

Table 3 shows the variant peptides contained in Antigen 2 and their mRNA sequences.

[Table 3]

| Peptide name | Length (aa) | mRNA sequence | Peptide |
|---|---|---|---|
| 8-G 12C-8 | 17 | UACAAGCUGGUCGUCGUGGGGGCCUGCGGCGUGGGGAAGUCCGCUCUGACC (SEQ ID NO: 52) | YKLVVVGACGVGKSALT (SEQ ID NO: 308) |
| 8-G12D-8 | 17 | UACAAACUGGUGGUGGUGGGGGCCGAUGGGGUGGGCAAGUCCGCCCUGACC (SEQ ID NO: 53) | YKLVVVGADGVGKSALT (SEQ ID NO: 309) |
| 8-G12V-8 | 17 | UACAAGCUGGGUGGUGGUCGGGGCCGUCGGCGUGGGCAAAAGCGCCCUGACA (SEQ ID NO: 54) | YKLVVVGAVGVGKSALT (SEQ ID NO: 310) |
| 8-G12R-8 | 17 | UACAAGCUCGUGGUGGUGGGCGCCAGGGGGGUGGGCAAGUCCGCCCUGACA (SEQ ID NO: 55) | YKLVVVGARGVGKSALT (SEQ ID NO: 311) |
| 8-G 13C-8 | 17 | AAGCUGGUCGUGGUGGGCGCCGGGUGCGUGGGCAAGAGCGCCCUGACAAUC(SEQ ID NO: 56) | KLVVVGAGCVGKSALTI (SEQ ID NO: 312) |
| 8-G13D-8 | 17 | AAGCUGGUGGUGGUGGGGGCCGGGGAUGUGGGCAAGAGCGCCUGACCAUC (SEQ ID NO: 57) | KLVVVGAGDVGKSALTI (SEQ ID NO: 313) |

(continued)

| Peptide name | Length (aa) | mRNA sequence | Peptide |
|---|---|---|---|
| 8-Q61H-8 | 17 | CUGGACAUCCUGGACACAGCC GGGCACGAGGAGUACAGCGCC AUGAGGGAU (SEQ ID NO: 58) | LDILDTAGHEEYSAMRD (SEQ ID NO: 314) |

Table 4 shows the variant peptides contained in Antigen 3 and their mRNA sequences.

[Table 4]

| Peptide name | Length (aa) | mRNA sequence | Peptide |
|---|---|---|---|
| 8-G 12C-8 | 17 | UACAAGCUCGUGGUGGUGGGC GCCUGCGGCGUGGGGAAGUC CGCCCUGACA (SEQ ID NO: 59) | YKLVVVGACGVGKSALT (SEQ ID NO: 315) |
| 8-G12D-8 | 17 | UACAAGCUGGUGGUCGUGGGC GCCGACGGGGUGGGCAAGAG CGCCCUCACC (SEQ ID NO: 60) | YKLVVVGADGVGKSALT (SEQ ID NO: 316) |
| 8-G12V-8 | 17 | UACAAGCUGGUCGUGGUCGGG GCCGUGGGCGUGGGCAAGUC CGCUCUGACA (SEQ ID NO: 61) | YKLVVVGAVGVGKSALT (SEQ ID NO: 317) |
| 8-G12R-8 | 17 | UACAAGCUGGGUGGUGGUGG GGGCCAGGGGGGUCGGGAAA UCCGCCCUGACU (SEQ ID NO: 62) | YKLVVVGARGVGKSALT (SEQ ID NO: 318) |
| 8-G 13C-8 | 17 | AAGCUCGUGGUGGUGGGGGC CGGGUGCGUGGGGAAGUCCG CCCUGACAUU (SEQ ID NO: 63) | KLVVVGAGCVGKSALTI (SEQ ID NO: 319) |
| 8-G13D-8 | 17 | AAGCUGGUCGUGGUGGGCGC CGGGGAUGUGGGCAAGAGCG CCUGACCAUC (SEQ ID NO: 64) | KLVVVGAGDVGKSALTI (SEQ ID NO: 320) |

(continued)

| Peptide name | Length (aa) | mRNA sequence | Peptide |
|---|---|---|---|
| 8-Q61H-8 | 17 | CUGGACAUCCUGGGACACCGC CGGCCACGAGGAGUACAGCGC CAUGAGGGAU (SEQ ID NO: 65) | LDILDTAGHEEYSAMRD (SEQ ID NO: 321) |

Table 5 shows the variant peptides contained in Antigen 4 and their mRNA sequences.

[Table 5]

| Peptide name | Length (aa) | mRNA sequence | Peptide |
|---|---|---|---|
| 3-G 12C-5 | 9 | GUUGGAGCCUGUGGAGUUGG UAAAAGU (SEQ ID NO: 66) | VGACGVGKS (SEQ ID NO: 322) |
| 5-G 12C-3 | 9 | GUAGUUGUGGGAGCUUGUGG AGUAGGU (SEQ ID NO: 67) | VVVGACGVG (sequence number 323) |
| 3-G 12C-5 | 9 | GUAGGAGCUUGUGGAGUAGGA AAGUCU (SEQ ID NO: 68) | VGACGVGKS (SEQ ID NO: 324) |
| 5-G 12C-3 | 9 | GUAGUAGUAGGAGCAUGCGGA GUCGGA (SEQ ID NO: 69) | VVVGACGVG (SEQ ID NO: 325) |
| 3-G 12C-5 | 9 | GUAGGAGCAUGCGGAGUAGGA AAGUCA (SEQ ID NO: 70) | VGACGVGKS (SEQ ID NO: 326) |
| 5-G 12C-3 | 9 | GUAGUAGUAGGAGCUUGUGGA GUAGGA (SEQ ID NO: 71) | VVVGACGVG (SEQ ID NO: 327) |

Table 6 shows the variant peptides contained in Antigen 5 and their mRNA sequences.

[Table 6]

| Peptide name | Length (aa) | mRNA sequence | Peptide |
|---|---|---|---|
| 3-G 12C-5 | 9 | GUGGGCGCCUGUGGAGUAGG AAAAUCU (SEQ ID NO: 72) | VGACGVGKS (SEQ ID NO: 328) |
| 5-G 12C-3 | 9 | GUGGUGGUCGGAGCAUGCGG AGUAGGA (SEQ ID NO: 73) | VVVGACGVG (SEQ ID NO: 329) |
| 3-G12D-5 | 9 | GUGGGAGCUGACGGCGUGGG UAAAUCC (SEQ ID NO: 74) | VGADGVGKS (SEQ ID NO: 330) |

(continued)

| Peptide name | Length (aa) | mRNA sequence | Peptide |
|---|---|---|---|
| 5-G12D-3 | 9 | GUGGUCGUGGGAGCUGACGGUGUUGGU (SEQ ID NO: 75) | VVVGADGVG (SEQ ID NO: 331) |
| 3-G13D-5 | 9 | GGAGCGGGAGAUGUUGGAAAGUCAGCC (SEQ ID NO: 76) | GAGDVGKSA (SEQ ID NO: 332) |
| 5-G13D-3 | 9 | GUUGUGGGGGCUGGUGACGUGGGAAAG (SEQ ID NO: 77) | VVGAGDVGK (SEQ ID NO: 333) |

Table 7 shows the variant peptides contained in Antigen 6 and their mRNA sequences.

[Table 7]

| Peptide name | Length (aa) | mRNA sequence | Peptide |
|---|---|---|---|
| 11-G12C-19 | 31 | AUGACAGAAUACAAACUUGUGGUAGUUGGAGCAUGCGGUGUGGGUAAGAGUGCCCUCACCAUCCAACUAAUUCAGAACCACUUCGUUGACGAA (SEQ ID NO: 78) | MTEYKLVVVGACGVGKSALTIQLIQNHFVDE (SEQ ID NO: 334) |
| 3-G 12C-4 | 8 | GUUGGAGCAUGUGGUGUAGGGAAG (SEQ ID NO: 79) | VGACGVGK (SEQ ID NO: 335) |
| 11-G12D-19 | 31 | AUGACUGAAUACAAACUUGUGGUUGUUGGAGCAGAUGGGGUCGGAAAAUCCGCCUUGACAAUACAGCUUAUUCAGAAUCACUUUGUGGAUGAG (SEQ ID NO: 80) | MTEYKLVVVGADGVGKSALTIQLIQNHFVDE (SEQ ID NO: 336) |
| 3-G12D-4 | 8 | GUCGGAGCUGAUGGCGUCGGAAAG (SEQ ID NO: 81) | VGADGVGK (SEQ ID NO: 337) |
| 11-G13D-19 | 31 | ACUGAGUAUAAAUUAGUGGUAGUCGGGGCUGGAGACGUGGGCAAGAGUGCCCUUACCAUCCAGCUCAUCCAGAAUCAUUUUGUGGACGAGUAU (SEQ ID NO: 82) | TEYKLVVVGAGDVGKSALTIQLIQNHFVDEY (SEQ ID NO: 338) |

(continued)

| Peptide name | Length (aa) | mRNA sequence | Peptide |
|---|---|---|---|
| 3-G13D-4 | 8 | GGAGCUGGUGACGUCGGAAAGUCU (SEQ ID NO: 83) | GAGDVGKS (SEQ ID NO: 339) |

Table 8 shows the variant peptides contained in Antigen 7 and their mRNA sequences.

[Table 8]

| Peptide name | Length (aa) | mRNA sequence | Peptide |
|---|---|---|---|
| 12-G13C-18 | 31 | AUGACUGAAUAUAAACUCGUCGUGGUUGGAGCAGGUUGCGUUGGCAAAAGCGCCCUUACAAUCCAGCUGAUACAGAACCACUUCGUGGAUGAG (SEQ ID NO: 84) | MTEYKLVVVGAGCVGKSALTIQLIQNHFVDE (SEQ ID NO: 340) |
| 11-G12C-19 | 31 | AUGACAGAGUACAAGUUGGUGGUGGUGGGCGCCUGUGGGGUGGGGGAAAUCUGCCCUUACUAUCCAGCUGAUCCAGAACCACUUUGUGGAUGAG (SEQ ID NO: 85) | MTEYKLVVVGACGVGKSALTIQLIQNHFVDE (SEQ ID NO: 341) |
| 11-G12D-19 | 31 | AUGACAGAAUACAAACUGGUCGUGGUCGGCGCUGAUGGCGUUGGGAAGAGUGCUCUGACAAUUCAACUCAUCCAGAAUCACUUCGUAGACGAA (SEQ ID NO: 86) | MTEYKLVVVGADGVGKSALTIQLIQNHFVDE (SEQ ID NO: 342) |
| 12-G13D-18 | 31 | AUGACGGAGUAUAAGCUCGUUGUGGUAGGCGCCGGAGACGUAGGCAAGUCCGCACUGACCAUUCAGCUCAUUCAAAAUCAUUUUGUAGACGAA (SEQ ID NO: 87) | MTEYKLVVVGAGDVGKSALTIQLIQNHFVDE (SEQ ID NO: 343) |

(continued)

| Peptide name | Length (aa) | mRNA sequence | Peptide |
|---|---|---|---|
| 11-G12V-19 | 31 | AUGACCGAGUACAAGCUGGUU GUUGUGGGUGCCGUGGGGGU GGGUAAGUCAGCCCUCACCAU UCAGCUCAUCCAAAACCAUUU CGUGGACGAG (SEQ ID NO: 88) | MTEYKLVVVGAVGVGKS ALTIQLIQNHFVDE (SEQ ID NO: 344) |
| 11-Q61H-19 | 31 | ACCUGCCUCCUGGACAUAUUG GACACUGCGGGGCAUGAGGAG UAUUCCGCAAUGCGCGAUCAG UACAUGAGGACCGGAGAAGGA UUUCUGUGC (SEQ ID NO: 89) | TCLLDILDTAGHEEYSAM RDQYMRTGEGFLC (SEQ ID NO: 345) |

Table 9 shows the variant peptides contained in Antigen 8 and their mRNA sequences.

[Table 9]

| Peptide name | Length (aa) | mRNA sequence | Peptide |
|---|---|---|---|
| 11-G12C-19 | 31 | AUGACAGAGUAUAAGCUGGUC GUGGUCGGCGCGUGCGGAGU GGGGAAAUCAGCUCUCACCAU UCAGCUAAUCCAGAACCACUU CGUUGACGAA (SEQ ID NO: 90) | MTEYKLVVVGACGVGKS ALTIQLIQNHFVDE (SEQ ID NO: 346) |
| 3-G 12C-4 | 8 | GUCGGCGCUUGCGGUGUCGG AAAG (SEQ ID NO: 91) | VGACGVGK (SEQ ID NO: 347) |
| 11-G12D-19 | 31 | AUGACGGAGUAUAAACUUGUC GUAGUUGGCGCCGACGGUGU UGGAAAAUCCGCAUUAACCAU ACAAUUGAUCCAGAACCACUU CGUGGACGAA (SEQ ID NO: 92) | MTEYKLVVVGADGVGKS ALTIQLIQNHFVDE (SEQ ID NO: 348) |
| 3-G12D-4 | 8 | GUGGGGGCUGACGGCGUAGG AAAA (SEQ ID NO: 93) | VGADGVGK (SEQ ID NO: 349) |

(continued)

| Peptide name | Length (aa) | mRNA sequence | Peptide |
|---|---|---|---|
| 11-G13D-19 | 31 | ACAGAGUACAAGCUGGUGGUGGUUGGCGCAGGGGAUGUGGGUAAGAGCGCCCUGACUAUUCAGCUGAUCCAAAAUCAUUUUGUGGAUGAAUAC (SEQ ID NO: 94) | TEYKLVVVGAGDVGKSALTIQLIQNHFVDEY (SEQ ID NO: 350) |
| 3-G13D-4 | 8 | GGAGCAGGCGAUGUGGGGAAGUCU (SEQ ID NO: 95) | GAGDVGKS (SEQ ID NO: 351) |

Table 10 shows the N-terminal domain, variant peptides and their mRNA sequences contained in Antigen 9.

[Table 10]

| Peptide name | Length (aa) | mRNA sequence | Peptide |
|---|---|---|---|
| DTT | 177 | UGUAUAAACCUCGAUUGGGAUGUAAUCCGCGACAAAACCAAGACAAAAAUUGAAUCUCUCAAGGAGCACGGCCCUAUCAAAAAUAAAAUGUCAGAGUCACCCAAUAAGACGGUGUCUGAGGAAAAGGCCAAGCAAUACUUGGAAGAAUUUCAUCAAACCGCCUUGGAACAUCCGGAACUAAGCGAGCUUAAGACAGUUACAGGUACUAACCCAGUGUUUGCCGGAGCUAACUACGCCGCGUGGGCGGUCAAUGUAGCCCAGGUCAUUGAUAGUGAAACUGCUGACAAUUUAGAAAAGACAACUGCCGCUCUCUCCAUUCUGCCCGGAAUAGGGAGCGUUAUGGGUAUCGCAGAUGGCGCGGUGCAUCAUAACACCGAGGAGAUCGUCGCACAGUCGAUCGCUCUCAGUAGCCUAAUGGUGGCUCAGGCAAUUCCUCUGGUCGGGGAACUGGUUGACAUCGGAUUCGCCGCAUACAAUUUCGUAGAGUCUAUUAUAAAUCUUUUCCAGGUGGUCCACAACUCAUAUAAUCGA (SEQ ID NO: 96) | CINLDWDVIRDKTKTKIESLKEHGPIKNKMSESPNKTVSEEKAKQYLEEFHQTALEHPELSELKTVTGTNPVFAGANYAAWAVNVAQVIDSETADNLEKTTAALSILPGIGSVMGIADGAVHHNTEEIVAQSIALSSLMVAQAIPLVGELVDIGFAAYNFVESIINLFQVVHNSYNR (SEQ ID NO: 352) |
| 12-G13C-18 | 31 | AUGACUGAGUACAAGCUGGUAGUGGUAGGGGCUGGAUGCGUCGGGAAGUCAGCCCUGACUAU | MTEYKLVVVGAGCVGKSALTIQLIQNHFVDE (SEQ ID NO: 353) |

(continued)

| Peptide name | Length (aa) | mRNA sequence | Peptide |
|---|---|---|---|
| | | ACAGCUGAUCCAGAACCACUU CGUGGAUGAG (SEQ ID NO: 97) | |
| 11-G12C-19 | 31 | AUGACGGAAUAUAAGCUCGUU GUGGUAGGGGCUUGUGGAGU GGGCAAGUCCGCACUGACAAU CCAGCUGAUCCAGAAUCACUU UGUGGAUGAG (SEQ ID NO: 98) | MTEYKLVVVGACGVGKS ALTIQLIQNHFVDE (SEQ ID NO: 354) |
| 11-G12D-19 | 31 | AUGACCGAAUACAAACUUGUU GUGGUGGGCGCUGACGGAGU UGGUAAAAGCGCCCUUACCAU CCAACUGAUUCAGAACCACUU UGUCGACGAG (SEQ ID NO: 99) | MTEYKLVVVGADGVGKS ALTIQLIQNHFVDE (SEQ ID NO: 355) |
| 12-G13D-18 | 31 | AUGACCGAGUAUAAAUUGGUG GUGGUGGGCGCUGGCGACGU GGGCAAAAGUGCACUGACAAU UCAGCUGAAUUCAGAACCACU UUGUGGACGAG (SEQ ID NO: 100) | MTEYKLVVVGAGDVGKS ALTIQLIQNHFVDE (SEQ ID NO: 356) |
| 11-G12V-19 | 31 | AUGACCGAGUAUAAGUUGGUG GUUGUCGGCGCGGUUGGUGU GGGAAAAUCUGCCCUUACAAU CCAGCUGAUUCAAAACCAUUU CGUCGACGAG (SEQ ID NO: 101) | MTEYKLVVVGAVGVGKS ALTIQLIQNHFVDE (SEQ ID NO: 357) |
| 11-Q61H-19 | 31 | ACGUGCUUGCUCGACAUCCUG GAUACUGCCGGCCACGAAGAG UACUCCGCCAUGCGGGAUCAA UAUAUGCGUACCGGCGAGGGG UUCUUAUGU (SEQ ID NO: 102) | TCLLDILDTAGHEEYSAM RDQYMRTGEGFLC (SEQ ID NO: 358) |

Antigen 9 is a peptide in which the DTT domain (1-177aa) and six variant peptides described in Table 10 are sequentially linked with a linker. Table 11 shows the variant peptides included in Antigen 10, the C-terminal domain, and its mRNA sequence.

[Table 11]

| Peptide name | Length (aa) | mRNA sequence | Peptide |
|---|---|---|---|
| 12-G13C-18 | 31 | AUGACAGAGUACAAGCUGGUGGUCGUCGGAGCCGGUUGCGUAGGAAAGAGCGCAUUAACCAUCCAGCUAAUCCAAAACCAUUUCGUGGACGAA (SEQ ID NO: 103) | MTEYKLVVVGAGCVGKSALTIQLIQNHFVDE (SEQ ID NO: 359) |
| 11-G12C-19 | 31 | AUGACUGAAUACAAGUUAGUGGUGGUUGGGGCCUGUGGUGUGGGAAAAUCCGCUCUCACGAUCCAGCUGAUUCAGAACCACUUCGUCGACGAG (SEQ ID NO: 104) | MTEYKLVVVGACGVGKSALTIQLIQNHFVDE (SEQ ID NO: 360) |
| 11-G12D-19 | 31 | AUGACCGAAUAUAAACUGGUUGUCGUGGGGGCUGAUGGAGUUGGCAAGUCUGCCUUGACUAUUCAACUGAUCCAGAAUUCAUUUUGUGGAUGAG (SEQ ID NO: 105) | MTEYKLVVVGADGVGKSALTIQLIQNHFVDE (SEQ ID NO: 361) |
| 12-G13D-18 | 31 | AUGACAGAGUAUAAACUCGUCGUGGUGGGUGCCGGAGAUGUUGGGAAAUCUGCUCUUACCAUACAGUUAAUCCAAAAUCACUUCGUUGAUGAA (SEQ ID NO: 106) | MTEYKLVVVGAGDVGKSALTIQLIQNHFVDE (SEQ ID NO: 362) |

(continued)

| Peptide name | Length (aa) | mRNA sequence | Peptide |
|---|---|---|---|
| 11-G12V-19 | 31 | AUGACUGAGUACAAGUUGGUGGUGGUGGGGGCUGUGGGGGUGGGAAAGUCUGCACUGACUAUUCAGCUCAUUCAGAACCACUUCGUGGAUGAG (SEQ ID NO: 107) | MTEYKLVVVGAVGVGKSALTIQLIQNHFVDE (SEQ ID NO: 363) |
| 11-Q61H-19 | 31 | ACCUGUCUGCUAGACAUUCUGGACACGGCCGGCCAUGAGGAGUAUAGCGCAAUGAGAGACCAAUACAUGCGGACCGGCGAAGGCUUUCUUUGC (SEQ ID NO: 108) | TCLLDILDTAGHEEYSAMRDQYMRTGEGFLC (SEQ ID NO: 364) |
| DTT | 177 | UGUAUAAACCUCGACUGGGAUGUGAUCCGCGAUAAAACCAAGACCAAGAUCGAAAGCCUAAAGGAGCAUGGCCCUAUUAAGAAUAAAAUGUCAGAAUCUCCCAAUAAAACAGUAUCCGAGGAAAAGGCCAAACAGUAUUUGGAAGAGUUUCACCAGACCGCCCUGGAACACCCAGAGCUGUCCGAACUGAAGACAGUCACUGGGACAAAUCCUGUCUUCGCCGGUGCUAACUACGCGGCAUGGGCGGUCAAUGUAGCCCAAGUUAUCGAUAGUGAGACUGCCGACAACCUUGAGAAAACCACAGCAGCAUUGAGCAUAUUGCCGGGCAUCGGUUCUGUCAUGGGCAUAGCUGACGGCGCUGUGCACCACAACACAGAGG | CINLDWDVIRDKTKTKIESLKEHGPIKNKMSESPNKTVSEEKAKQYLEEFHQTALEHPELSELKTVTGTNPVFAGANYAAWAVNVAQVIDSETADNLEKTTAALSILPGIGSVMGIADGAVHHNTEEIVAQSIALSSLMVAQAIPLVGELVDIGFAAYNFVESIINLFQVVHNSYNR (SEQ ID NO: 365) |

(continued)

| Peptide name | Length (aa) | mRNA sequence | Peptide |
|---|---|---|---|
| | | AGAUCGUCGCUCAGAGUAUUGCGCUGUCCAGUCUUAUGGUGGCCCAGGCCAUCCCACUUGUUGGUGAACUGGUGGACAUCGGAUUUGCAGCGUACAACUUUGUCGAGUCAAUUAUUAAUCUGUUCCAGGUUGUCCAUAACAGUUAUAAUCGU (SEQ ID NO: 109) | |

Antigen 10 is a peptide in which the six variant peptides and the DTT domain (1-177aa) described in Table 11 are sequentially linked together with linkers. Table 12 shows the N-terminal domain, variant peptides, C-terminal domain, and mRNA sequences thereof included in Antigen 11.

[Table 12]

| Peptide name | Length (aa) | mRNA sequence | Peptide |
|---|---|---|---|
| DTT(1-87) | 87 | UGUAUAAAUCUUGACUGGGACGUCAUUAGGGAUAAGACAAAGACCAAGAUAGAGUCCUUGAAGGGAGCACGGCCCCAUCAAAAACAAAAUGUCGGAGAGCCCAAACAAAACAGUGAGUGAAGAGAAAGCAAAGCAGUAUCUUGAAGAGUUUCACCAGACGGCUCUGGAACAUCCUGAGCUGUCUGAACUUAAGACAGUGACCGGCACAAACCCUGUGUUUGCAGGAGCUAAUUAUGCGCUUGGGCCGUUAACGUGGCUCAG (SEQ ID NO: 110) | CINLDWDVIRDKTKTKIESLKEHGPIKNKMSESPNKTVSEEKAKQYLEEFHQTALEHPELSELKTVTGTNPVFAGANYAAWAVNVAQ (SEQ ID NO: 366) |

(continued)

| Peptide name | Length (aa) | mRNA sequence | Peptide |
|---|---|---|---|
| 12-G13C-18 | 31 | AUGACUGAGUAUAAACUGGUUGUGGUGGGCGCCGGCUGCGUCGGAAAGAGCGCGCUGACCAUUCAACUUAUUCAAAAUCACUUCGUUGAUGAA (SEQ ID NO: 111) | MTEYKLVVVGAGCVGKSALTIQLIQNHFVDE (SEQ ID NO: 367) |
| 11-G12C-19 | 31 | AUGACUGAAUAUAAGUUAGUGGUCGUGGGCGCCUGUGGGGUGGGUAAAAGCGCCCUAACUAUACAGCUAAUCCAGAACCACUUUGUAGACGAG (SEQ ID NO: 112) | MTEYKLVVVGACGVGKSALTIQLIQNHFVDE (SEQ ID NO: 368) |
| 11-G12D-19 | 31 | AUGACAGAGUACAAGCUGGGUGGUUGUAGGUGCAGACGGCGUCGGCAAAUCCGCCCUCACUAUCCAGCUGAUUCAGAACCACUUUGUAGACGAG (SEQ ID NO: 113) | MTEYKLVVVGADGVGKSALTIQLIQNHFVDE (SEQ ID NO: 369) |
| 12-G13D-18 | 31 | AUGACUGAAUACAAACUUGUUGUGGUUGGGGCUGGGGAUGUCGGAAAGAGUGCUCUGACGAUUCAGCUGAUCCAGAACCAUUUUGUGGACGAA (SEQ ID NO: 114) | MTEYKLVVVGAGDVGKSALTIQLIQNHFVDE (SEQ ID NO: 370) |
| 11-G12V-19 | 31 | AUGACCGAGUACAAGCUGGGUGGUUGUGGGAGCGGUCGGGGUCGGGAAGUCCGCCUUAACAAUCCAGCUGAUCCAAAAUCACUUCGUGGGAUGAA (SEQ ID NO: 115) | MTEYKLVVVGAVGVGKSALTIQLIQNHFVDE (SEQ ID NO: 371) |

(continued)

| Peptide name | Length (aa) | mRNA sequence | Peptide |
|---|---|---|---|
| 11-Q61H-19 | 31 | ACCUGCCUGCUCGACAUCCUC GAUACCGCCGGUCACGAAGAG UACUCCGCCAUGCGUGAUCAA UAUAUGCGAACGGGGGAGGGU UUCUUAUGC (SEQ ID NO: 116) | TCLLDILDTAGHEEYSAM RDQYMRTGEGFLC (SEQ ID NO: 372) |
| DTT(96-177) | 82 | AAUUUGGAGAAAACCACCGCA GCACUAUCUAUCCUGCCGGGA AUAGGUAGCGUCAUGGGCAUC GCCGACGGAGCAGUACAUCAU AAUACUGAAGAAAUUGUGGCC CAAUCUAUCGCCUUAAGUUCU UUGAUUGGUGGCUCAGGCUAU UCCCCUGGUCGGGGAGCUGG UAGAUAUUGGCUUCGCUGCCU ACAACUUCGUGGAGAGCAUCA UUAAUUUGUUCCAGGUCGUGC AUAAUAGCUACAACAGG (SEQ ID NO: 117) | NLEKTTAALSILPGIGSV MGIADGAVHHNTEEIVA QSIALSSLMVAQAIPLVG ELVDIGFAAYNFVESIINL FQVVHNSYNR (SEQ ID NO: 373) |

Antigen 11 is a peptide in which a DTT domain (1-87aa), six variant peptides, and a DTT domain (96-177aa) are sequentially linked with a linker as described in Table 12. Table 13 shows the variant peptides included in Antigen 12 and their mRNA sequences.

[Table 13]

| Peptide name | Lengt h (aa) | mRNA | Peptide |
|---|---|---|---|
| 8-G 12C-8 | 17 | UACAAGCUAGUGGUGGUCGGCGCA UGCGGCGUGGGAAAGAGCGCGCUA ACC (SEQ ID NO: 118) | YKLVVVGACGVGKS ALT (SEQ ID NO: 374) |
| 8-G12D-8 | 17 | UAUAAAUUGGUCGUUGUGGGCGCC GAUGGUGUAGGGAAAUCUGCGCUG ACC(SEQ ID NO: 119) | YKLVVVGADGVGKS ALT (SEQ ID NO: 375) |

(continued)

| Peptide name | Lengt h (aa) | mRNA | Peptide |
|---|---|---|---|
| 8-G12V-8 | 17 | UACAAGCUCGUGGUGGUGGGGGCAGUCGGCGUCGGCAAAAGUGCCCUGACU (SEQ ID NO: 120) | YKLVVVGAVGVGKSALT (SEQ ID NO: 376) |
| 8-G12R-8 | 17 | UAUAAGCUGGUCGUGGUAGGUGCUCGUGGCGUUGGAAAGUCCGCCUUGACA (SEQ ID NO: 121) | YKLVVVGARGVGKSALT (SEQ ID NO: 377) |
| 8-G 13C-8 | 17 | AAGCUGGUUGUCGUGGGGGCCGGAUGCGUGGGCAAAUCUGCUCUUACAAUC (SEQ ID NO: 122) | KLVVVGAGCVGKSALTI (SEQ ID NO: 378) |
| 8-G13D-8 | 17 | AAAUUAGUAGUUGUGGGAGCUGGAGACGUAGGUAAAUCCGCACUGACGAUA (SEQ ID NO: 123) | KLVVVGAGDVGKSALTI (SEQ ID NO: 379) |
| 8-Q61H-8 | 17 | CUCGAUAUUCUCGACACCGCUGGACACGAAGAGUACAGCGCCAUGCGCGAC (SEQ ID NO: 124) | LDILDTAGHEEYSAMRD (SEQ ID NO: 380) |
| 8-G13R-8 | 17 | AAGCUGGUCGUGGUGGGGGCCGGCAGAGUUGGUAAGUCAGCACUCACUAUC (SEQ ID NO: 125) | KLVVVGAGRVGKSALTI (SEQ ID NO: 381) |

Table 14 shows the variant peptides contained in Antigen 13 and their mRNA sequences.

[Table 14]

| Peptide name | Lengt h (aa) | mRNA | Peptide |
|---|---|---|---|
| 11-G12C-19 | 31 | AUGACCGAGUACAAACUCGUUGUGGUGGGGGCCUGCGGAGUGGGCAAGUCUGCAUUGACCAUACAGUUGAUCCAGAAUCAUUUCGUGGAUGAG (SEQ ID NO: 126) | MTEYKLVVVGACGVGKSALTIQLIQNHFVDE(SEQ ID NO: 382) |

(continued)

| Peptide name | Length (aa) | mRNA | Peptide |
|---|---|---|---|
| 3-G 12C-4 | 8 | GUCGGAGCGUGUGGCGUGGGCAAA (SEQ ID NO: 127) | VGACGVGK (SEQ ID NO: 383) |
| 11-G12D-19 | 31 | AUGACUGAGUAUAAGUUAGUAGUCGUUGGUGCCGAUGGAGUAGGCAAAUCGGCGUUAACGAUCCAGCUUAUUCAGAAUCAUUUCGUAGAUGAG (SEQ ID NO: 128) | MTEYKLVVVGADGVGKSALTIQLIQNHFVDE (SEQ ID NO: 384) |
| 3-G12D-4 | 8 | GUCGGGGCCGACGGCGUGGGCAAA (SEQ ID NO: 129) | VGADGVGK (SEQ ID NO: 385) |
| 11-G12V-19 | 31 | AUGACCGAGUAUAAGUUAGUCGUCGUGGGUGCUGUGGGCGUAGGUAAAUCCGCCCUGACUAUACAGCUUAUUCAAAACCAUUUUGUGGAUGAG (SEQ ID NO: 130) | MTEYKLVVVGAVGVGKSALTIQLIQNHFVDE (SEQ ID NO: 386) |
| 3-G12V-4 | 8 | GUGGGAGCUGUGGGCGUUGGGAAA (SEQ ID NO: 131) | VGAVGVGK (SEQ ID NO: 387) |
| 11-G12R-19 | 31 | AUGACUGAGUACAAGCUGGUGGUCGUCGGUGCCCGGGGCGUUGGUAAGAGCGCCCUGACCAUACAACUCAUUCAGAACCACUUUGUAGACGAA (SEQ ID NO: 132) | MTEYKLVVVGARGVGKSALTIQLIQNHFVDE (SEQ ID NO: 388) |
| 3-G12R-4 | 8 | GUGGGAGCAAGGGGAGUCGGGAAA (SEQ ID NO: 133) | VGARGVGK (SEQ ID NO: 389) |
| 11-G13C-19 | 31 | ACAGAAUACAAGCUGGUUGUAGUCGGCGCCGGUUGUGUCGGGAAGUCCGCACUCACAAUUCAGCUUAUCCAGAAUCACUUCGUGGACGAGUAU (SEQ ID NO: 134) | TEYKLVVVGAGCVGKSALTIQLIQNHFVDEY (SEQ ID NO: 390) |
| 3-G 13C-4 | 8 | GGGGCAGGCUGCGUUGGAAAGAGC (SEQ ID NO: 135) | GAGCVGKS (SEQ ID NO: 391) |

(continued)

| Peptide name | Length (aa) | mRNA | Peptide |
|---|---|---|---|
| 11-G13D-19 | 31 | ACAGAGUACAAAUUAGUGGUCGUGGGAGCUGGGGACGUGGGAAAGUCCGCUCUCACUAUCCAGCUGAUUCAGAACCAUUUUGUCGACGAAUAU (SEQ ID NO: 136) | TEYKLVVVGAGDVGKSALTIQLIQNHFVDEY (SEQ ID NO: 392) |
| 3-G13D-4 | 8 | GGGGCUGGGGACGUGGGCAAAUCA (SEQ ID NO: 137) | GAGDVGKS (SEQ ID NO: 393) |
| 11-Q61H-19 | 31 | ACCUGCCUGCUGGAUAUCCUGGACACAGCGGGACACGAAGAAUACAGCGCCAUGAGAGAUCAGUACAUGCGAACGGGUGAAGGCUUCCUGUGC (SEQ ID NO: 138) | TCLLDILDTAGHEEYSAMRDQYMRTGEGFLC (SEQ ID NO: 394) |
| 3-Q61H-4 | 8 | ACAGCAGGGCACGAGGAAUACUCC (SEQ ID NO: 139) | TAGHEEYS (SEQ ID NO: 395) |
| 11-G13R-19 | 31 | ACUGAAUAUAAGCUGGUAGUUGUUGGCGCAGGAAGAGUUGGGAAGAGUGCUCUAACCAUCCAACUUAUCCAGAACCACUUUGUGGACGAGUAU (SEQ ID NO: 140) | TEYKLVVVGAGRVGKSALTIQLIQNHFVDEY (SEQ ID NO: 396) |
| 3-G13R-4 | 8 | GGCGCCGGGCGCGUCGGAAAGUCU (SEQ ID NO: 141) | GAGRVGKS (SEQ ID NO: 397) |

Table 15 shows the variant peptides contained in Antigen 14 and their mRNA sequences.

[Table 15]

| Peptide name | Length (aa) | mRNA | Peptide |
|---|---|---|---|
| 11-G12C-19 | 31 | AUGACAGAAUACAAGUUAGUGGUGGUAGGAGCGUGUGGAGUGGGAAAAGCGCCCUAACUAUUCAGUUGAUACAAACCAUUUCGUGGACGAA (SEQ ID NO: 142) | MTEYKLVVVGACGVGKSALTIQLIQNHFVDE (SEQ ID NO: 398) |
| 3-G 12C-4 | 8 | GUGGGCGCAUGUGGAGUCGGCAAG (SEQ ID NO: 143) | VGACGVGK (SEQ ID NO: 399) |

(continued)

| Peptide name | Length (aa) | mRNA | Peptide |
|---|---|---|---|
| 11-G12D-19 | 31 | AUGACCGAGUAUAAGCUCGUAGUCGUGGGGGCCGACGGCGUGGGGAAAUCAGCCCUUACUAUCCAGCUUAUACAAAACCAUUUUGUUGACGAA (SEQ ID NO: 144) | MTEYKLVVVGADGVGKSALTIQLIQNHFVDE (SEQ ID NO: 400) |
| 3-G12D-4 | 8 | GUAGGCGCCGACGGGGUCGGAAAG (SEQ ID NO: 145) | VGADGVGK (SEQ ID NO: 401) |
| 11-G12V-19 | 31 | AUGACCGAGUACAAGCUGGUUGUCGUCGGGGGCCGUAGGGUUGGCAAAAGCGCCCUGACUAUCCAGCUCAUCCAAAAUCAUUUCGUCGACGAG (SEQ ID NO: 146) | MTEYKLVVVGAVGVGKSALTIQLIQNHFVDE (SEQ ID NO: 402) |
| 3-G 12V-4 | 8 | GUGGGCGCCGUUGGAGUCGGUAAA (SEQ ID NO: 147) | VGAVGVGK (SEQ ID NO: 403) |
| 11-G12R-19 | 31 | AUGACGGAAUACAAGUUGGUGGUAGUGGGGGCUCGGGGGGUGGGCAAGAGUGCUUUGACCAUACAGUUAAUUCAGAACCACUUUGUGGAUGAG (SEQ ID NO: 148) | MTEYKLVVVGARGVGKSALTIQLIQNHFVDE (SEQ ID NO: 404) |
| 3-G12R-4 | 8 | GUCGGCGCUCGCGGAGUCGGCAAA (SEQ ID NO: 149) | VGARGVGK (SEQ ID NO: 405) |
| 11-G13C-19 | 31 | ACCGAGUAUAAAUUGGUUGUUGUCGGCGCAGGCUGUGUGGGCAAGUCUGCUCUCACCAUUCAACUAAUCCAGAACCACUUUGUUGAUGAGUAC (SEQ ID NO: 150) | TEYKLVVVGAGCVGKSALTIQLIQNHFVDEY (SEQ ID NO: 406) |
| 3-G 13C-4 | 8 | GGAGCAGGCUGCGUGGGAAAAAGC (SEQ ID NO: 151) | GAGCVGKS (SEQ ID NO: 407) |

(continued)

| Peptide name | Length (aa) | mRNA | Peptide |
|---|---|---|---|
| 11-G13D-19 | 31 | ACGGAGUACAAGCUGGUUGUCGUAGGAGCAGGAGACGUGGGCAAAUCCGCUCUGACAAUUCAGCUUAUCCAGAAUCACUUUGUGGAUGAAUAU (SEQ ID NO: 152) | TEYKLVVVGAGDVGKSALTIQLIQNHFVDEY (SEQ ID NO: 408) |
| 3-G13D-4 | 8 | GGUGCCGGGGAUGUCGGUAAAAGU (SEQ ID NO: 153) | GAGDVGKS (SEQ ID NO: 409) |
| 11-Q61H-19 | 31 | ACCUGCUUACUGGACAUCCUGGACACUGCUGGUCACGAGGAAUACUCCGCGAUGCGAGAUCAGUAUAUGCGGACAGGAGAGGGUUUCCUGUGC (SEQ ID NO: 154) | TCLLDILDTAGHEEYSAMRDQYMRTGEGFLC (SEQ ID NO: 410) |
| 3-Q61H-4 | 8 | ACAGCAGGCCAUGAGGAAUAUUCU (SEQ ID NO: 155) | TAGHEEYS (SEQ ID NO: 411) |
| 11-G13R-19 | 31 | ACUGAAUAUAAGCUGGUGGUGGUGGGGGCUGGUAGAGUGGGGAAGUCAGCACUGACAAUCCAGCUCAUUCAGAAUCACUUCGUUGAUGAGUAC (SEQ ID NO: 156) | TEYKLVVVGAGRVGKSALTIQLIQNHFVDEY (SEQ ID NO: 412) |
| 3-G13R-4 | 8 | GGUGCGGGUAGAGUGGGCAAGUCC (SEQ ID NO: 157) | GAGRVGKS (SEQ ID NO: 413) |

Table 16 shows the variant peptides contained in Antigen 15 and their mRNA sequences.

[Table 16]

| Peptide name | Length (aa) | mRNA | Peptide |
|---|---|---|---|
| 11-G12D-19 | 31 | AUGACGGAAUACAAGCUCGUCGUGGUGGGCGCCGAUGGCGUCGGCAAGUCGGCACUGACUAUCCAGCUCAUACAAAAUCAUUUUGUUGAUGAA (SEQ ID NO: 158) | MTEYKLVVVGADGVGKSALTIQLIQNHFVDE (SEQ ID NO: 414) |

(continued)

| Peptide name | Length (aa) | mRNA | Peptide |
|---|---|---|---|
| 3-G12D-4 | 8 | GUGGGGGCGGACGGAGUGGGGAAG (SEQ ID NO: 159) | VGADGVGK (SEQ ID NO: 415) |
| 11-G12C-19 | 31 | AUGACCGAAUACAAACUGGUGGUGGUCGGGGCGUGUGGGGGGGCAAAUCAGCCCUCACAAUACAGCUGAUUCAGAACCACUUUGUUGACGAG (SEQ ID NO: 160) | MTEYKLVVVGACGVGKSALTIQLIQNHFVDE (SEQ ID NO: 416) |
| 3-G 12C-4 | 8 | GUGGGGGCAUGUGGAGUGGGGAAG (SEQ ID NO: 161) | VGACGVGK (SEQ ID NO: 417) |
| 11-G12V-19 | 31 | AUGACUGAGUACAAGCUGGUUGUUGUAGGUGCUGUUGGAGUGGGAAAGAGCGCCCUCACAAUCCAAUUGAUCCAGAAUCACUUCGUCGACGAG (SEQ ID NO: 162) | MTEYKLVVVGAVGVGKSALTIQLIQNHFVDE (SEQ ID NO: 418) |
| 3-G12V-4 | 8 | GUGGGGGCCGUAGGCGUGGGCAAG (SEQ ID NO: 163) | VGAVGVGK (SEQ ID NO: 419) |
| 11-G12R-19 | 31 | AUGACCGAAUAUAAGUUGGUAGUGGUGGGGGCUCGCGGAGUCGGGAAGAGUGCUCUUACUAUUCAGCUGAAUUCAGAACCAUUUCGUAGAUGAA (SEQ ID NO: 164) | MTEYKLVVVGARGVGKSALTIQLIQNHFVDE (SEQ ID NO: 420) |
| 3-G12R-4 | 8 | GUGGGCGCCAGAGGCGUGGGCAAA (SEQ ID NO: 165) | VGARGVGK (SEQ ID NO: 421) |
| 11-G13C-19 | 31 | ACUGAGUACAAACUAGUAGUCGUAGGAGCUGGGUGUGUGGGCAAAAGUGCACUCACCAUCCAGCUGAUCCAGAACCAUUUCGUGGAUGAGUAU (SEQ ID NO: 166) | TEYKLVVVGAGCVGKSALTIQLIQNHFVDEY (SEQ ID NO: 422) |
| 3-G13C-4 | 8 | GGAGCUGGCUGCGUGGGCAAAAGC (SEQ ID NO: 167) | GAGCVGKS (SEQ ID NO: 423) |

(continued)

| Peptide name | Length (aa) | mRNA | Peptide |
|---|---|---|---|
| 11-G13D-19 | 31 | ACGGAAUACAAACUAGUUGUUGUGGGGGCUGGUGAUGUCGGAAAGUCCGCCUUGACUAUUCAGCUUAUUCAAAAUCACUUUGUCGACGAGUAU (SEQ ID NO: 168) | TEYKLVVVGAGDVGKSALTIQLIQNHFVDEY (SEQ ID NO: 424) |
| 3-G13D-4 | 8 | GGCGCAGGUGACGUAGGAAAGUCU (SEQ ID NO: 169) | GAGDVGKS (SEQ ID NO: 425) |
| 11-Q61H-19 | 31 | ACCUGCCUUCUGGACAUCCUGGACACAGCCGGCCACGAGGAAUACAGCGCCAUGCGGGAUCAGUAUAUGAGGACAGGAGAAGGCUUCCUCUGC (SEQ ID NO: 170) | TCLLDILDTAGHEEYSAMRDQYMRTGEGFLC (SEQ ID NO: 426) |
| 3-Q61H-4 | 8 | ACAGCUGGCCACGAGGAGUAUUCU (SEQ ID NO: 171) | TAGHEEYS (SEQ ID NO: 427) |
| 11-G13R-19 | 31 | ACCGAGUACAAACUUGUGGGUGGUUGGAGCAGGGCGUGUCGGCAAAUCUGCCCUGACCAUCCAGCUGAUACAAAACCAUUUUGUGGAUGAGUAU (SEQ ID NO: 172) | TEYKLVVVGAGRVGKSALTIQLIQNHFVDEY (SEQ ID NO: 428) |
| 3-G13R-4 | 8 | GGCGGUGCGGGACGAGUGGGCAAGAGC (SEQ ID NO: 173) | GAGRVGKS (SEQ ID NO: 429) |

Table 17 shows the variant peptides contained in Antigen 16 and their mRNA sequences.

[Table 17]

| Peptide name | Length (aa) | mRNA | Peptide |
|---|---|---|---|
| 8-G 12C-8 | 17 | UAUAAGUUGGUGGUGGUUGGGGCAUGUGGCGUAGGUAAGAGCGCACUGACU (SEQ ID NO: 174) | YKLVVVGACGVGKSALT (SEQ ID NO: 430) |

(continued)

| Peptide name | Lengt h (aa) | mRNA | Peptide |
|---|---|---|---|
| 8-G12D-8 | 17 | UAUAAGCUAGUUGUCGUGGGAGCUGACGGAGUGGGGAAGAGUGCACUCACA (SEQ ID NO: 175) | YKLVVVGADGVGKSALT (SEQ ID NO: 431) |
| 8-G12V-8 | 17 | UACAAGCUCGUCGUGGUGGGCCGCCGUCGGCGUAGGGAAAUCAGCUCUUACC (SEQ ID NO: 176) | YKLVVVGAVGVGKSALT (SEQ ID NO: 432) |
| 8-G12R-8 | 17 | UAUAAACUCGUGGUGGUGGGCGCUCGCGGUGUGGGCAAAAGUGCCCUGACC (SEQ ID NO: 177) | YKLVVVGARGVGKSALT (SEQ ID NO: 433) |
| 8-G 13C-8 | 17 | AAGCUGGUCGUCGUGGGCGCCGGCUGCGUUGGGAAAUCCGCCCUGACAAUC (SEQ ID NO: 178) | KLVVVGAGCVGKSALTI (SEQ ID NO: 434) |
| 8-G13D-8 | 17 | AAAUUGGUCGUCGUGGGCGCCGGGGACGUGGGUAAGUCAGCCUUGACCAUC (SEQ ID NO: 179) | KLVVVGAGDVGKSALTI (SEQ ID NO: 435) |
| 8-Q61H-8 | 17 | CUCGACAUACUGGACACUGCCGGCCACGAAGAAUAUUCUGCCAUGAGGGAU (SEQ ID NO: 180) | LDILDTAGHEEYSAMRD (SEQ ID NO: 436) |
| 8-G13R-8 | 17 | AAAUUAGUGGUGGUAGGUGCCGGUAGAGUGGGGAAGUCGGCUCUCACCAUU (SEQ ID NO: 181) | KLVVVGAGRVGKSALTI (SEQ ID NO: 437) |
| 8-G 12C-8 | 17 | UACAAGCUGGGUGGUAGUCGGCGCAUGUGGAGUGGGAAAAAGCGCCCUGACA (SEQ ID NO: 182) | YKLVVVGACGVGKSALT (SEQ ID NO: 438) |

(continued)

| Peptide name | Lengt h (aa) | mRNA | Peptide |
|---|---|---|---|
| 8-G12D-8 | 17 | UACAAAUUAGUGGUGGUCGGUGCU GAUGGGGUCGGCAAGUCAGCUCUG ACC (SEQ ID NO: 183) | YKLVVVGADGVGKS ALT (SEQ ID NO: 439) |
| 8-G12V-8 | 17 | UACAAACUCGUUGUCGUUGGGGCC GUCGGAGUGGGUAAAUCCGCCCUG ACC (SEQ ID NO: 184) | YKLVVVGAVGVGKS ALT (SEQ ID NO: 440) |
| 8-G12R-8 | 17 | UAUAAAUUGGUCGUGGUAGGGGCU CGGGGAGUCGGGAAAUCUGCGCUG ACC (SEQ ID NO: 185) | YKLVVVGARGVGKS ALT (SEQ ID NO: 441) |
| 8-G 13C-8 | 17 | AAGCUCGUUGUGGUGGGAGCAGGU UGCGUUGGUAAGUCUGCUUUGACU AUC (SEQ ID NO: 186) | KLVVVGAGCVGKSA LTI (SEQ ID NO: 442) |
| 8-Q61H-8 | 17 | CUCGACAUCCUCGACACCGCAGGAC AUGAGGAGUACUCCGCCAUGCGUG AC (SEQ ID NO: 187) | LDILDTAGHEEYSA MRD (SEQ ID NO: 443) |
| 8-G13D-8 | 17 | AAGCUAGUAGUUGUAGGAGCCGGU GAUGUGGGAAAGUCCGCUCUGACAA UU (SEQ ID NO: 188) | KLVVVGAGDVGKSA LTI (SEQ ID NO: 444) |
| 8-G13R-8 | 17 | AAAUUGGUAGUCGUGGGGGCCGGC AGAGUGGGAAAAAGCGCGCUGACAA UC (SEQ ID NO: 189) | KLVVVGAGRVGKSA LTI (SEQ ID NO: 445) |
| 8-G12V-8 | 17 | UAUAAAACUUGUUGUGGUGGGCGC AGUCGGAGUUGGUAAGUCAGCUCU GACU(SEQ ID NO: 190) | YKLVVVGAVGVGKS ALT (SEQ ID NO: 446) |

(continued)

| Peptide name | Lengt h (aa) | mRNA | Peptide |
|---|---|---|---|
| 8-G12R-8 | 17 | UACAAGCUCGUUGUUGUGGGCGCUCGCGGGGUGGGCAAGUCUGCCCUAACA (SEQ ID NO: 191) | YKLVVVGARGVGKSALT (SEQ ID NO: 447) |
| 8-G 13C-8 | 17 | AAACUAGUGGUGGUCGGCGCCGGGUGCGUAGGAAAGAGUGCUCUGACCAUA (SEQ ID NO: 192) | KLVVVGAGCVGKSALTI (SEQ ID NO: 448) |
| 8-Q61H-8 | 17 | CUUGACAUUCUGGAUACUGCGGGCCACGAGGAAUAUAGCGCGAUGCGAGAU (SEQ ID NO: 193) | LDILDTAGHEEYSAMRD (SEQ ID NO: 449) |
| 8-G 12C-8 | 17 | UACAAACUGGUUGUUGUGGGAGCAUGUGGCGUCGGGAAGUCCGCAUUAACG (SEQ ID NO: 194) | YKLVVVGACGVGKSALT (SEQ ID NO: 450) |
| 8-G12D-8 | 17 | UACAAGUUAGUGGUGGUCGGAGCGGAUGGGCGUUGGGAAAGUCUGCACUUACG (SEQ ID NO: 195) | YKLVVVGADGVGKSALT (SEQ ID NO: 451) |
| 8-G13D-8 | 17 | AAGUUGGUGGUGGUGGGGGCCGGAGACGUAGGCAAGUCCGCACUUACCAUC (SEQ ID NO: 196) | KLVVVGAGDVGKSALTI (SEQ ID NO: 452) |
| 8-G 13R-8 | 17 | AAACUGGUUGUCGUCGGCGCCGGGCGGGUGGGCAAGAGCGCACUGACUAUU (SEQ ID NO: 197) | KLVVVGAGRVGKSALTI (SEQ ID NO: 453) |

Table 18 shows the variant peptides contained in Antigen 17 and their mRNA sequences.

[Table 18]

| Peptide name | Length (aa) | mRNA | Peptide |
|---|---|---|---|
| 11-G12D-19 | 31 | AUGACGGAAUACAAGCUCGUCGUGGUGGGCGCCGAUGGCGUCGGCAAGUCGGCACUGACUAUCCAGCUCAUACAAAAUCAUUUUGUUGAUGAA (SEQ ID NO: 198) | MTEYKLVVVGADGVGKSALTIQLIQNHFVDE (SEQ ID NO: 454) |
| 3-G12D-4 | 8 | GUGGGGGCGGACGGAGUGGGGAAG (SEQ ID NO: 199) | VGADGVGK (SEQ ID NO: 455) |
| 11-G12C-19 | 31 | AUGACCGAAUACAAACUGGUGGUGGUCGGGGGCUGUGGGGGGUGGGCAAAUCAGCCCUCACAAUACAGCUGAUUCAGAACCACUUUGUUGACGAG (SEQ ID NO: 200) | MTEYKLVVVGACGVGKSALTIQLIQNHFVDE (SEQ ID NO: 456) |
| 3-G 12C-4 | 8 | GUGGGGGCAUGUGGAGUGGGGAAG (SEQ ID NO: 201) | VGACGVGK (SEQ ID NO: 457) |
| 11-G12V-19 | 31 | AUGACUGAGUACAAGCUGGUUGUUGUAGGUGCUGUUGGAGUGGGAAAGAGCGCCCUCACAAUCCAAUUGAUCCAGAAUCACUUCGUCGACGAG (SEQ ID NO: 202) | MTEYKLVVVGAVGVGKSALTIQLIQNHFVDE (SEQ ID NO: 458) |
| 3-G12V-4 | 8 | GUGGGGGCCGUAGGCGUGGGCAAG (SEQ ID NO: 203) | VGAVGVGK (SEQ ID NO: 459) |
| 11-G12R-19 | 31 | AUGACCGAAUAUAAGUUGGUAGUGGUGGGGGCUCGCGGAGUCGGGAAGAGUGCUCUUACUAUUCAGCUGAAUUCAGAACCAUUUCGUAGAUGAA (SEQ ID NO: 204) | MTEYKLVVVGARGVGKSALTIQLIQNHFVDE (SEQ ID NO: 460) |
| 3-G12R-4 | 8 | GUGGGCGCCAGAGGCGUGGGCAAA (SEQ ID NO: 205) | VGARGVGK (SEQ ID NO: 461) |

(continued)

| Peptide name | Length (aa) | mRNA | Peptide |
|---|---|---|---|
| 11-G13C-19 | 31 | ACUGAGUACAAACUAGUAGUCGUAGGAGCUGGGUGUGUGGGCAAAAGUGCACUCACCAUCCAGCUGAUCCAGAACCAUUUCGUGGAUGAGUAU (SEQ ID NO: 206) | TEYKLVVVGAGCVGKSALTIQLIQNHFVDEY (SEQ ID NO: 462) |
| 3-G 13C-4 | 8 | GGAGCUGGCUGCGUGGGCAAAAGC (SEQ ID NO: 207) | GAGCVGKS (SEQ ID NO: 463) |
| 11-G13D-19 | 31 | ACGGAAUACAAACUAGUUGUUGUGGGGGCUGGUGAUGUCGGAAAGUCCGCCUUGACUAUUCAGCUUAUUCAAAAUCACUUUGUCGACGAGUAU (SEQ ID NO: 208) | TEYKLVVVGAGDVGKSALTIQLIQNHFVDEY (SEQ ID NO: 464) |
| 3-G13D-4 | 8 | GGCGCAGGUGACGUAGGAAAGUCU (SEQ ID NO: 209) | GAGDVGKS (SEQ ID NO: 465) |
| 11-Q61H-19 | 31 | ACCUGCCUUCUGGACAUCCUGGACACAGCCGGCCACGAGGAAUACAGCGCCAUGCGGGAUCAGUAUAUGAGGACAGGAGAAGGCUUCCUCUGC (SEQ ID NO: 210) | TCLLDILDTAGHEEYSAMRDQYMRTGEGFLC (SEQ ID NO: 466) |
| 3-Q61H-4 | 8 | ACAGCUGGCCACGAGGAGUAUUCU (SEQ ID NO: 211) | TAGHEEYS (SEQ ID NO: 467) |

Table 19 shows the variant peptides contained in Antigen 18 and their mRNA sequences.

[Table 19]

| Peptide name | Length (aa) | mRNA | Peptide |
|---|---|---|---|
| 11-G12D-19 | 31 | AUGACAGAGUAUAAACUCGUGGUGGUCGGCGCAGAUGGCGUUGGAAAGAGCGCCCUGACUAUUCAGCUCAUCCAGAACCAUUUCGUAGACGAA (SEQ ID NO: 212) | MTEYKLVVVGADGVGKSALTIQLIQNHFVDE(SEQ ID NO: 468) |

(continued)

| Peptide name | Length (aa) | mRNA | Peptide |
|---|---|---|---|
| 3-G12D-4 | 8 | GUCGGCGCAGACGGUGUGGGAAAG (SEQ ID NO: 213) | VGADGVGK (SEQ ID NO: 469) |
| 11-G12C-19 | 31 | AUGACCGAAUACAAGUUAGUGGUGGUCGGGGCCUGCGGUGUGGGUAAAAGCGCUCUGACCAUACAGCUUAUCCAGAACCACUUCGUGGAUGAG (SEQ ID NO: 214) | MTEYKLVVVGACGVGKSALTIQLIQNHFVDE (SEQ ID NO: 470) |
| 3-G 12C-4 | 8 | GUGGGGGCUUGCGGAGUUGGCAAG (SEQ ID NO: 215) | VGACGVGK (SEQ ID NO: 471) |
| 11-G12V-19 | 31 | AUGACAGAAUAUAAAUUGGUGGUAGUGGGGGCCGUAGGCGUCGGCAAGAGCGCCCUGACUAUACAACUGAUCCAAAACCACUUCGUGGACGAG (SEQ ID NO: 216) | MTEYKLVVVGAVGVGKSALTIQLIQNHFVDE (SEQ ID NO: 472) |
| 3-G12V-4 | 8 | GUGGGCGCCGUUGGAGUCGGGAAA (SEQ ID NO: 217) | VGAVGVGK (SEQ ID NO: 473) |
| 11-G12R-19 | 31 | AUGACUGAGUAUAAACUUGUUGUAGUCGGGGCACGGGGCGUGGGAAAGUCCGCUUUAACCAUACAAUUGAUACAGAACCACUUUGUUGAUGAA (SEQ ID NO: 218) | MTEYKLVVVGARGVGKSALTIQLIQNHFVDE (SEQ ID NO: 474) |
| 3-G12R-4 | 8 | GUGGGUGCCAGGGGAGUCGGUAAG (SEQ ID NO: 219) | VGARGVGK (SEQ ID NO: 475) |
| 11-Q61H-19 | 31 | ACCUGUCUCCUAGACAUUCUGGACACAGCCGGCCACGAGGAGUACUCAGCUAUGCGCGAUCAGUAUAUGAGAACAGGGGAGGGAUUUCUGUGU (SEQ ID NO: 220) | TCLLDILDTAGHEEYSAMRDQYMRTGEGFLC (SEQ ID NO: 476) |
| 3-Q61H-4 | 8 | ACAGCAGGGCACGAAGAGUACAGC (SEQ ID NO: 221) | TAGHEEYS (SEQ ID NO: 477) |

(continued)

| Peptide name | Length (aa) | mRNA | Peptide |
|---|---|---|---|
| 11-G 13D-19 | 31 | ACUGAGUACAAGCUAGUCGUGGUUGGCGCAGGCGACGUGGGUAAAUCUGCCCUCACCAUUCAGUUAAUUCAGAAUCAUUUCGUCGAUGAAUAU (SEQ ID NO: 222) | TEYKLVVVGAGDVGKSALTIQLIQNHFVDEY (SEQ ID NO: 478) |
| 3-G13D-4 | 8 | GGCGCUGGAGACGUCGGCAAAUCC (SEQ ID NO: 223) | GAGDVGKS (SEQ ID NO: 479) |
| 11-G12A-19 | 31 | AUGACGGAGUAUAAACUGGUAGUCGUAGGUGCUGCCGGCGUCGGCAAAAGCGCGCUAACCAUCCAGCUGAUCCAGAAUCACUUUGUGGAUGAA (SEQ ID NO: 224) | MTEYKLVVVGAAGVGKSALTIQLIQNHFVDE (SEQ ID NO: 480) |
| 3-G 12A-4 | 8 | GUGGGAGCCGCUGGAGUGGGGAAG (SEQ ID NO: 225) | VGAAGVGK (SEQ ID NO: 481) |
| 11-A146T-19 | 31 | AGAUCUUACGGAAUCCCUUUCAUCGAAACCUCAACCAAGACUCGUCAAAGGGUUGAAGACGCUUUCUAUACACUUGUCAGAGAGAUUAGGCAG (SEQ ID NO: 226) | RSYGIPFIETSTKTRQRVEDAFYTLVREIRQ (SEQ ID NO: 482) |
| 3-A146T-4 | 8 | GAAACCUCCACAAAGACGCGGCAA (SEQ ID NO: 227) | ETSTKTRQ (SEQ ID NO: 483) |
| 11-G13C-19 | 31 | ACUGAGUACAAGCUGGUGGUAGUGGGAGCCGGUUGCGUGGGCAAGUCAGCGCUGACAAUCCAGCUCAUCCAGAAUCAUUUUGUUGAUGAGUAC (SEQ ID NO: 228) | TEYKLVVVGAGCVGKSALTIQLIQNHFVDEY (SEQ ID NO: 484) |
| 3-G 13C-4 | 8 | GGCGCAGGCUGCGUGGGGAAAUCG (SEQ ID NO: 229) | GAGCVGKS (SEQ ID NO: 485) |

(continued)

| Peptide name | Length (aa) | mRNA | Peptide |
|---|---|---|---|
| 11-G12S-19 | 31 | AUGACUGAGUACAAGCUGGUGGUGGUGGGGGCGUCUGGGGUGGGGGAAAUCUGCACUGACGAUUCAACUGAUCCAGAAUCAUUUUGUAGACGAG (SEQ ID NO: 230) | MTEYKLVVVGASGVGKSALTIQLIQNHFVDE (SEQ ID NO: 486) |
| 3-G 12S-4 | 8 | GUGGGCGCGUCCGGCGUGGGUAAG (SEQ ID NO: 231) | VGASGVGK (SEQ ID NO: 487) |
| 11-Q61R-19 | 31 | ACCUGUUUGCUGGAUAUUCUGGACACUGCCGGACGAGAAGAAUACAGUGCAAUGAGGGAUCAGUAUAUGCGAACCGGAGAGGGCUUUCUUUGU (SEQ ID NO: 232) | TCLLDILDTAGREEYSAMRDQYMRTGEGFLC (SEQ ID NO: 488) |
| 3-Q61R-4 | 8 | ACAGCCGGCCGCGAAGAGUACAGC (SEQ ID NO: 233) | TAGREEYS (SEQ ID NO: 489) |

Table 20 shows the variant peptides contained in Antigen 19 and their mRNA sequences.

[Table 20]

| Peptide name | Length (aa) | mRNA | Peptide |
|---|---|---|---|
| 11-G12C-19 | 31 | AUGACGGAAUAUAAGCUCGUGGUCGUUGGGGCCUGUGGCGUGGGUAAGAGCGCACUAACCAUUCAGUUAAUCCAGAACCACUUUGUUGAUGAA (SEQ ID NO: 234) | MTEYKLVVVGACGVGKSALTIQLIQNHFVDE (SEQ ID NO: 490) |
| 3-G 12C-4 | 8 | GUUGGGGCCUGCGGCGUUGGAAAA (SEQ ID NO: 235) | VGACGVGK (SEQ ID NO: 491) |
| 11-G12V-19 | 31 | AUGACAGAGUACAAACUGGUCGUGGUGGGGGCUGUGGGUGUGGGCAAGUCCGCCCUGACUAUCCAACUGAUACAGAACCAUUUCGUAGAUGAA (SEQ ID NO: 236) | MTEYKLVVVGAVGVGKSALTIQLIQNHFVDE (SEQ ID NO: 492) |

(continued)

| Peptide name | Length (aa) | mRNA | Peptide |
|---|---|---|---|
| 3-G12V-4 | 8 | GUCGGCGCAGUCGGGGUAGGUAAA (SEQ ID NO: 237) | VGAVGVGK (SEQ ID NO: 493) |
| 11-G12D-19 | 31 | AUGACUGAGUAUAAACUCGUGGUUGUGGGUGCAGAUGGAGUAGGAAAGAGUGCCCUCACAAUACAACUGAUUCAGAAUCAUUUUGUCGAUGAA (SEQ ID NO: 238) | MTEYKLVVVGADGVGKSALTIQLIQNHFVDE (SEQ ID NO: 494) |
| 3-G12D-4 | 8 | GUGGGAGCUGAUGGUGUUGGCAAG (SEQ ID NO: 239) | VGADGVGK (SEQ ID NO: 495) |
| 11-Q61H-19 | 31 | ACCUGCCUGCUGGACAUCCUGGACACCGCGGGGCACGAAGAGUAUAGCGCUAUGCGGGACCAGUACAUGAGAACCGGUGAGGGCUUUCUGUGU (SEQ ID NO: 240) | TCLLDILDTAGHEEYSAMRDQYMRTGEGFLC (SEQ ID NO: 496) |
| 3-Q61H-4 | 8 | ACAGCCGGCCACGAGGAGUAUUCU (SEQ ID NO: 241) | TAGHEEYS (SEQ ID NO: 497) |
| 11-G13C-19 | 31 | ACUGAGUACAAGCUGGUGGUGGUUGGCGCUGGCUGUGUGGGGAAAUCUGCAUUAACGAUUCAACUGAUCCAGAAUCACUUCGUCGAUGAGUAC (SEQ ID NO: 242) | TEYKLVVVGAGCVGKSALTIQLIQNHFVDEY (SEQ ID NO: 498) |
| 3-G 13C-4 | 8 | GGCGCAGGCUGCGUGGGCAAGUCA (SEQ ID NO: 243) | GAGCVGKS (SEQ ID NO: 499) |
| 11-G13D-19 | 31 | ACAGAGUACAAGCUGGUUGUCGUUGGGGCAGGGGACGUCGGGAAGUCCGCCCUGACUAUCCAGCUGAUUCAGAACCAUUUUGUGGACGAAUAU (SEQ ID NO: 244) | TEYKLVVVGAGDVGKSALTIQLIQNHFVDEY (SEQ ID NO: 500) |
| 3-G13D-4 | 8 | GGUGCUGGAGACGUGGGAAAAUCC (SEQ ID NO: 245) | GAGDVGKS (SEQ ID NO: 501) |

(continued)

| Peptide name | Length (aa) | mRNA | Peptide |
|---|---|---|---|
| 11-G12A-19 | 31 | AUGACAGAGUACAAACUUGUGGUGGUUGGCGCCUGCUGGAGUAGGAAAAUCCGCCCUGACCAUACAACUUAUUCAGAAUCACUUCGUGGACGAG (SEQ ID NO: 246) | MTEYKLVVVGAAGVGKSALTIQLIQNHFVDE (SEQ ID NO: 502) |
| 3-G 12A-4 | 8 | GUCGGGGCGGCCGGGGUGGGGAAG (SEQ ID NO: 247) | VGAAGVGK (SEQ ID NO: 503) |
| 11-G12S-19 | 31 | AUGACUGAAUACAAGUUGGUGGUGGUGGGCGCGAGCGGGGGUGGGUAAGUCUGCCUUGACCAUCCAGCUGAUCCAGAACCAUUUCGUUGAUGAA (SEQ ID NO: 248) | MTEYKLVVVGASGVGKSALTIQLIQNHFVDE (SEQ ID NO: 504) |
| 3-G 12S-4 | 8 | GUCGGCGCCUCGGGCGUGGGGAAA (SEQ ID NO: 249) | VGASGVGK (SEQ ID NO: 505) |

Table 21 shows the variant peptides contained in Antigen 20 and their mRNA sequences.

[Table 21]

| Peptide name | Length (aa) | mRNA | Peptide |
|---|---|---|---|
| 11-G12D-19 | 31 | AUGACAGAGUACAAACUGGUAGUAGUGGGAGCAGAUGGGGUUGGCAAAUCCGCGCUCACCAUCCAGCUGAUUCAAAACCAUUUCGUCGAUGAG (SEQ ID NO: 250) | MTEYKLVVVGADGVGKSALTIQLIQNHFVDE(SEQ ID NO: 506) |
| 3-G12D-4 | 8 | GUGGGCGCUGACGGGGUGGGCAAG (SEQ ID NO: 251) | VGADGVGK (SEQ ID NO: 507) |
| 11-G12V-19 | 31 | AUGACCGAGUAUAAACUCGUAGUGGUCGGAGCUGUGGGAGUGGGAAAGUCAGCGCUCACUAUCCAACUAAUACAGAAUCACUUUGUCGACGAA (SEQ ID NO: 252) | MTEYKLVVVGAVGVGKSALTIQLIQNHFVDE (SEQ ID NO: 508) |

(continued)

| Peptide name | Length (aa) | mRNA | Peptide |
|---|---|---|---|
| 3-G12V-4 | 8 | GUGGGCGCCGUGGGUGUCGGCAAG (SEQ ID NO: 253) | VGAVGVGK (SEQ ID NO: 509) |
| 11-G12R-19 | 31 | AUGACAGAAUACAAGUUGGUCGUUGUUGGUGCCCGCGGAGUGGGCAAAUCUGCACUGACCAUCCAACUGAUCCAGAACCACUUUGUGGAUGAG (SEQ ID NO: 254) | MTEYKLVVVGARGVGKSALTIQLIQNHFVDE (SEQ ID NO: 510) |
| 3-G12R-4 | 8 | GUCGGUGCCAGGGGUGUGGGAAAG (SEQ ID NO: 255) | VGARGVGK (SEQ ID NO: 511) |
| 11-Q61H-19 | 31 | ACAUGUUUGUUAGACAUUCUCGACACGGCUGGGCAUGAGGAGUAUAGCGCUAUGCGGGAUCAGUAUAUGAGAACCGGCGAAGGCUUUCUCUGC (SEQ ID NO: 256) | TCLLDILDTAGHEEYSAMRDQYMRTGEGFLC (SEQ ID NO: 512) |
| 3-Q61H-4 | 8 | ACAGCCGGGCACGAGGAAUAUUCC (SEQ ID NO: 257) | TAGHEEYS (SEQ ID NO: 513) |
| 11-Q61R-19 | 31 | ACUUGCUUGCUGGACAUAUUAGACACAGCAGGGAGGGAAGAGUAUUCCGCAAUGCGAGAUCAGUACAUGCGUACUGGGGAAGGGUUCCUGUGC (SEQ ID NO: 258) | TCLLDILDTAGREEYSAMRDQYMRTGEGFLC (SEQ ID NO: 514) |
| 3-Q61R-4 | 8 | ACCGCCGGGAGGGAGGAAUACAGC (SEQ ID NO: 259) | TAGREEYS (SEQ ID NO: 515) |
| 11-G13C-19 | 31 | ACUGAGUACAAGCUGGUUGUGGUGGGAGCCGGAUGUGUCGGUAAAAGUGCCCUAACCAUCCAGCUCAUUCAGAACCACUUCGUCGAUGAGUAU (SEQ ID NO: 260) | TEYKLVVVGAGCVGKSALTIQLIQNHFVDEY (SEQ ID NO: 516) |
| 3-G 13C-4 | 8 | GGGGCCGGCUGUGUUGGGAAAAGC (SEQ ID NO: 261) | GAGCVGKS (SEQ ID NO: 517) |

(continued)

| Peptide name | Length (aa) | mRNA | Peptide |
|---|---|---|---|
| 11-G12C-19 | 31 | AUGACUGAGUACAAGCUCGUUGUCGUGGGUGCAUGCGGAGUAGGGAAGUCAGCGCUGACAAUUCAGUUAAUCCAGAAUCACUUCGUCGAUGAA (SEQ ID NO: 262) | MTEYKLVVVGACGVGKSALTIQLIQNHFVDE (SEQ ID NO: 518) |
| 3-G 12C-4 | 8 | GUCGGCGCCUGUGGAGUGGGUAAG (SEQ ID NO: 263) | VGACGVGK (SEQ ID NO: 519) |
| 11-G13D-19 | 31 | ACGGAGUACAAGCUGGUCGUGGUUGGCGCCGGCGACGUGGGCAAAAGCGCUUUGACCAUUCAACUGAUCCAGAAUCAUUUUGUGGAUGAAUAC (SEQ ID NO: 264) | TEYKLVVVGAGDVGKSALTIQLIQNHFVDEY (SEQ ID NO: 520) |
| 3-G13D-4 | 8 | GGGGCAGGGGACGUGGGGAAAUCU (SEQ ID NO: 265) | GAGDVGKS (SEQ ID NO: 521) |

Table 22 shows the variant peptides contained in Antigen 21 and their mRNA sequences.

[Table 22]

| Peptide name | Length (aa) | mRNA | Peptide |
|---|---|---|---|
| 11-G12D-19 | 31 | AUGACUGAAUAUAAAUUAGUUGUGGUGGGGAGCCGAUGGAGUGGGGAAAUCCGCGUUGACAAUCCAGCUGAUCCAGAACCAUUUUGUUGACGAA (SEQ ID NO: 266) | MTEYKLVVVGADGVGKSALTIQLIQNHFVDE (SEQ ID NO: 522) |
| 3-G12D-4 | 8 | GUUGGAGCUGAUGGCGUUGGCAAG (SEQ ID NO: 267) | VGADGVGK (SEQ ID NO: 523) |
| 11-G12V-19 | 31 | AUGACGGAAUACAAGCUGGUCGUCGUGGGCGCGGUCGGGGUAGGUAAGUCCGCGCUGACAAUUCAGCUGAAUUCAGAAUCACUUCGUGGAUGAG (SEQ ID NO: 268) | MTEYKLVVVGAVGVGKSALTIQLIQNHFVDE (SEQ ID NO: 524) |

(continued)

| Peptide name | Length (aa) | mRNA | Peptide |
|---|---|---|---|
| 3-G12V-4 | 8 | GUGGGCGCCGUGGGAGUCGGUAAG (SEQ ID NO: 269) | VGAVGVGK (SEQ ID NO: 525) |
| 11-G130-19 | 31 | ACCGAGUACAAGCUCGUGGUUGUCGGUGCUGGUGACGUGGGCAAGUCGGCUCUGACGAUCCAACUGAUCCAGAAUCAUUUCGUUGAUGAGUAC (SEQ ID NO: 270) | TEYKLVVVGAGDVGKSALTIQLIQNHFVDEY (SEQ ID NO: 526) |
| 3-G13D-4 | 8 | GGGGCCGGAGAUGUCGGCAAAUCU (SEQ ID NO: 271) | GAGDVGKS (SEQ ID NO: 527) |
| 11-A146T-19 | 31 | AGGUCAUACGGGAUACCUUUCAUUGAGACUUCCACCAAAACUCGUCAACGCGUCGAGGAUGCCUUUUAUACCCUAGUGAGAGAGAUCCGACAG (SEQ ID NO: 272) | RSYGIPFIETSTKTRQRVEDAFYTLVREIRQ (SEQ ID NO: 528) |
| 3-A146T-4 | 8 | GAAACAAGCACCAAGACCCGGCAG (SEQ ID NO: 273) | ETSTKTRQ (SEQ ID NO: 529) |
| 11-G12C-19 | 31 | AUGACAGAAUACAAGUUGGUAGUUGUCGGGCCCUGCGGGGUGGGGAAAAGUGCUCUAACCAUUCAAUUAAUCCAAAAUCACUUUGUCGACGAG (SEQ ID NO: 274) | MTEYKLVVVGACGVGKSALTIQLIQNHFVDE (SEQ ID NO: 530) |
| 3-G 12C-4 | 8 | GUGGGCGCCUGCGGGGUAGGCAAA (SEQ ID NO: 275) | VGACGVGK (SEQ ID NO: 531) |
| 11-G12A-19 | 31 | AUGACCGAAUAUAAACUGGUGGUGGUGGGAGCUGCAGGAGUUGGAAAAAGCGCUCUGACUAUCCAACUGAUUCAGAACCAUUUCGUGGACGAG (SEQ ID NO: 276) | MTEYKLVVVGAAGVGKSALTIQLIQNHFVDE (SEQ ID NO: 532) |
| 3-G 12A-4 | 8 | GUCGGGGCAGCCGGCGUGGGAAAA (SEQ ID NO: 277) | VGAAGVGK (SEQ ID NO: 533) |

(continued)

| Peptide name | Length (aa) | mRNA | Peptide |
|---|---|---|---|
| 11-G12S-19 | 31 | AUGACCGAAUACAAGCUGGUGGUGGUUGGCGCAUCCGGUGUGGGAAAGUCAGCCCUGACAAUCCAGCUUAUACAGAACCACUUCGUCGACGAA (SEQ ID NO: 278) | MTEYKLVVVGASGVGKSALTIQLIQNHFVDE (SEQ ID NO: 534) |
| 3-G 12S-4 | 8 | GUGGGCGCAUCUGGAGUCGGAAAG (SEQ ID NO: 279) | VGASGVGK (SEQ ID NO: 535) |
| 11-G13C-19 | 31 | ACAGAGUAUAAAUUGGUGGUGGUAGGUGCUGGAUGUGUCGGCAAGUCCGCACUUACUAUACAGUCUCAUUCAGAACCACUUUGUAGACGAGUAU (SEQ ID NO: 280) | TEYKLVVVGAGCVGKSALTIQLIQNHFVDEY (SEQ ID NO: 536) |
| 3-G 13C-4 | 8 | GGGGCAGGGUGCGUAGGCAAGUCC (SEQ ID NO: 281) | GAGCVGKS (SEQ ID NO: 537) |

Table 23 shows the variant peptides contained in Antigen 22 and their mRNA sequences.

[Table 23]

| Peptide name | Length (aa) | mRNA | Peptide |
|---|---|---|---|
| 11-G12D-19 | 31 | AUGACGGAAUAUAAGUUGGUCAUGGUGGGUGCGGAUGGUGUAGGCAAAUCAGCUCUGACUAUCCAACUGAUCCAGAAUCACUUUGUGGACGAA (SEQ ID NO: 282) | MTEYKLVMVGADGVGKSALTIQLIQNHFVDE (SEQ ID NO: 538) |
| 3-G12D-4 | 8 | GUCGGGGCUGACGGUGUCGGAAAA (SEQ ID NO: 283) | VGADGVGK (SEQ ID NO: 539) |
| 11-G12C-19 | 31 | AUGACCGAAUAUAAGCUCGUUGUGGUGGGGGCUUGUGGAGUUGGCAAGUCCGCUCUGACUAUCCAACUAAUUCAGAAUCAUUUCGUCGAUGAG (SEQ ID NO: 284) | MTEYKLVVVGACGVGKSALTIQLIQNHFVDE (SEQ ID NO: 540) |

(continued)

| Peptide name | Length (aa) | mRNA | Peptide |
|---|---|---|---|
| 3-G 12C-4 | 8 | GUCGGCGCUUGCGGAGUAGGCAAA (SEQ ID NO: 285) | VGACGVGK (SEQ ID NO: 541) |
| 11-G12V-19 | 31 | AUGACCGAAUACAAGCUCGUGGUGGUAGGCGCCGUUGGUGUAGGGAAGAGUGCGCUAACCAUUCAACUUAUCCAGAACCAUUUUGUUGACGAG (SEQ ID NO: 286) | MTEYKLVVVGAVGVGKSALTIQLIQNHFVDE (SEQ ID NO: 542) |
| 3-G12V-4 | 8 | GUAGGCGCGGUGGGAGUGGGCAAG (SEQ ID NO: 287) | VGAVGVGK (SEQ ID NO: 543) |
| 11-G12R-19 | 31 | AUGACCGAGUAUAAACUUGUAGUGGUGGGCGCCAGGGGCGUGGGGAAAAGCGCCCUGACAAUCCAGCUCAUACAGAAUCACUUCGUAGACCGAG (SEQ ID NO: 288) | MTEYKLVVVGARGVGKSALTIQLIQNHFVDE (SEQ ID NO: 544) |
| 3-G12R-4 | 8 | GUGGGCGCCCGCGGCGUUGGUAAG (SEQ ID NO: 289) | VGARGVGK (SEQ ID NO: 545) |
| 11-G13C-19 | 31 | ACUGAAUAUAAACUCGUCGUCGUGGGAGCAGGCUGUGUGGGGAAGAGUGCCUUGACCAUUCAGUUGAUCCAGAACCACUUCGUCGACGAGUAU (SEQ ID NO: 290) | TEYKLVVVGAGCVGKSALTIQLIQNHFVDEY (SEQ ID NO: 546) |
| 3-G 13C-4 | 8 | GGAGCAGGGUGCGUUGGAAAGAGC (SEQ ID NO: 291) | GAGCVGKS (SEQ ID NO: 547) |
| 11-G13D-19 | 31 | ACAGAAUACAAACUGGUUGUCGUGGGGGCCGGCGACGUGGGCAAGAGCGCCUUGACUAUUCAACUCAUACAGAACCACUUUGUGGAUGAGUAC (SEQ ID NO: 292) | TEYKLVVVGAGDVGKSALTIQLIQNHFVDEY (SEQ ID NO: 548) |
| 3-G13D-4 | 8 | GGCGCCGGGGACGUGGGGAAAAGC (SEQ ID NO: 293) | GAGDVGKS (SEQ ID NO: 549) |

(continued)

| Peptide name | Length (aa) | mRNA | Peptide |
|---|---|---|---|
| 11-Q61H-19 | 31 | ACGUGUCUUCUGGAUAUUUUAGACACCGCAGGGCAUGAGGAGUACUCCGCAAUGCGAGAUCAGUACAUGAGGACAGGUGAGGGUUUCCUGUGC (SEQ ID NO: 294) | TCLLDILDTAGHEEYSAMRDQYMRTGEGFLC (SEQ ID NO: 550) |
| 3-Q61H-4 | 8 | ACAGCCGGCCACGAGGAAUACUCU (SEQ ID NO: 295) | TAGHEEYS (SEQ ID NO: 551) |

**Example 2: Cloning of a sequence encoding an antigenic peptide linked to multiple KRAS variant peptides**

For cloning of a nucleotide sequence having as an open reading frame (ORF) the DNA sequence encoding an antigenic peptide linked to multiple KRAS variant peptides described in Example 1 above, a T7 promoter and a 5'-UTR sequence were added to the 5' region of the sequence, and HindIII and XhoI restriction enzyme sequences were inserted to the 5' and 3' ends, respectively, to secure the sequence through gene synthesis.

The synthesized sequence contained HindIII and XhoI restriction enzyme sequences at the 5' and 3' ends, respectively, so that it could be used for cloning. Each synthesized sequence encoding the antigens and the template vector were digested with HindIII and XhoI, and then ligated using DNA ligase to construct a vector comprising a sequence encoding "mRNA."

The full-length mRNAs of Antigens 1 to 22 shown in Table 1 each have the structure of 5'UTR (SEQ ID NO: 554)-ORF-stop codon-3'UTR (SEQ ID NO: 555)-polyA (SEQ ID NO: 556).

Table 24 shows the full-length mRNA sequences of Antigens 2, 6, 14, 15, and 17, which are KRAS mutant anticancer vaccine candidates used for efficacy evaluation.

[Table 24]

| | Full-length mRNA sequence | ORF |
|---|---|---|
| Candidate S | AAGCTTTAATACGACTCACTATAAGTCCTCCCCATCCTCTCCCTCTGTCCCTCTGTCCCTCTGACCCTGCACTGTCCCAGCACCATGGATATGAGGGTGCCCGCCCAGCTGCTGGGGCTGCTGCTGCTGTGGCTGCGGGGCGCCCGGTGCTACAAGCTGGTCGTCGTGGGGGCCTGCGGCGTGGGGAAGTCCGCTCTGACCTACAAACTGGTGGTGGTGGGGGCCGATGGGGTGGGCAAGTCCGCCCTGACCTACAAGCTGGTGGTGGTCGGGGCCGTCGGCGTGGGCAAAAGCGCCCTGACATACAAGCTCGTGGTGGTGGGCGCCAGGGGGGTGGGCAAGTCCGCCCTGACAAAGCTGGTCGTGG | (SEQ ID NO: 2) |

(continued)

| | Full-length mRNA sequence | ORF |
|---|---|---|
| ubstance 2 | TGGGCGCCGGGTGCGTGGGCAAGAGCGCCCTGACAATCAAGCTGGTGGTGGTGGGGGCCGGGGATGTGGGCAAGAGCGCCCTGACCATCCTGGACATCCTGGACACAGCCGGGCACGAGGAGTACAGCGCCATGAGGGATTGATGACTCGAGGTGTGTGGAGGACACCCTGAACCCCCCGCTTTCAAACAAGTTTTCAAATTGTTTGAGGTCAGGATTTCTCAAACTGATTCCTTTCTTTGCATATGAGTATTTGAAAATAAATATTTTCCCAGAATATAAATAAATCATCACATGATTATTTTAACTATGCTAGCAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAGAAGAGCGAATTC (SEQ ID NO: 296) | |
| Candidate Substance 6 | AAGCTTTAATACGACTCACTATAAGTCCTCCCCATCCTCTCCCTCTGTCCCTCTGTCCCTCTGACCCTGCACTGTCCCAGCACCATGGATATGAGAGTGCCCGCCCAACTGCTGGGGCTGCTGCTGCTGTGGCTGAGAGGCGCGAGATGCATGACAGAATACAAACTTGTGGTAGTTGGAGCATGCGGTGTGGGTAAGAGTGCCCTCACCATCCAACTAATTCAGAACCACTTCGTTGACGAACTCCTCTCAGTGGGGGGGGTTGGAGCATGTGGTGTAGGGAAGCTGTTGAGCGTCGGCGGGATGACTGAATACAAACTTGTGGTTGTTGGAGCAGATGGGGTCGGAAAATCCGCCTTGACAATACAGCTTATTCAGAATCACTTTGTGGATGAGCTGTTAAGCGTTGGCGGCGTCGGAGCTGATGGCGTCGGAAAGCTCTTGTCTGTGGGCGGCACTGAGTATAAATTAGTGGTAGTCGGGGCTGGAGACGTGGGCAAGAGTGCCCTTACCATCCAGCTCATCCAGAATCATTTTGTGGACGAGTATCTATTGTCCGTGGGAGGTGGAGCTGGTGACGTCGGAAAGTCTTAATGACTCGAGGTGTGTGGAGGACACCCTGAACCCCCCGCTTTCAAACAAGTTTTCAAATTGTTTGAGGTCAGGATTTCTCAAACTGATTCCTTTCTTTGCATATGAGTATTTGAAAATAAATATTTTCCCAGAATATAAATAAATCATCACATGATTATTTTAACTATGCTAGCAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAGAAGAGCGAATTC (SEQ ID NO: 297) | (SEQ ID NO: 6) |

(continued)

| | Full-length mRNA sequence | ORF |
|---|---|---|
| Candidate Substance 14 | AAGCTTTAATACGACTCACTATAAGAATCCAGTCAACGCATATCCACG TATACCCGAACACATCACAGCCACCATGGATATGCGAGTGCCCGCTC AGCTTCTGGGGTTGCTTCTGTTATGGCTGAGGGGGGCCCGGTGTAT GACAGAATACAAGTTAGTGGTGGTAGGAGCGTGTGGAGTGGGGAAA AGCGCCCTAACTATTCAGTTGATACAAAACCATTTCGTGGACGAAGT GGGCGCATGTGGAGTCGGCAAGATGACCGAGTATAAGCTCGTAGTC GTGGGGGCCGACGGCGTGGGGAAATCAGCCCTTACTATCCAGCTTA TACAAAACCATTTTGTTGACGAAGTAGGCGCCGACGGGGTCGGAAA GATGACCGAGTACAAGCTGGTTGTCGTCGGGGCCGTAGGGGTTGGC AAAAGCGCCCTGACTATCCAGCTCATCCAAAATCATTTCGTCGACGA GGTGGGCGCCGTTGGAGTCGGTAAAATGACGGAATACAAGTTGGTG GTAGTGGGGGCTCGGGGGGTGGGCAAGAGTGCTTTGACCATACAGT TAATTCAGAACCACTTTGTGGATGAGGTCGGCGCTCGCGGAGTCGG CAAAACCGAGTATAAATTGGTTGTTGTCGGCGCAGGCTGTGTGGGC AAGTCTGCTCTCACCATTCAACTAATCCAGAACCACTTTGTTGATGAG TACGGAGCAGGCTGCGTGGGAAAAAGCACGGAGTACAAGCTGGTTG TCGTAGGAGCAGGAGACGTGGGCAAATCCGCTCTGACAATTCAGCT TATCCAGAATCACTTTGTGGATGAATATGGTGCCGGGGATGTCGGTA AAAGTACCTGCTTACTGGACATCCTGGACACTGCTGGTCACGAGGAA TACTCCGCGATGCGAGATCAGTATATGCGGACAGGAGAGGGTTTCC TGTGCACAGCAGGCCATGAGGAATATTCTACTGAATATAAGCTGGTG GTGGTGGGGGCTGGTAGAGTGGGGAAGTCAGCACTGACAATCCAG CTCATTCAGAATCACTTCGTTGATGAGTACGGTGCGGGTAGAGTGGG CAAGTCCTAATGACTCGAGGTGTGTGGAGGACACCCTGAACCCCCC GCTTTCAAACAAGTTTTCAAATTGTTTGAGGTCAGGATTTCTCAAACT GATTCCTTTCTTTGCATATGAGTATTTGAAAATAAATATTTTCCCAGAA TATAAATAAATCATCACATGATTATTTTAACTATGCTAGCAAAAAAAAA AAAAAAAAAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAAAAGAAGA GCGAATTC (SEQ ID NO: 298) | (SEQ ID NO: 14) |

(continued)

| | Full-length mRNA sequence | ORF |
|---|---|---|
| Candidate Substance 15 | AAGCTTTAATACGACTCACTATAAGAATCCAGTCAACGCATATCCACG TATACCCGAACACATCACAGCCACCATGGATATGCGAGTGCCCGCTC AGCTTCTGGGGTTGCTTCTGTTATGGCTGAGGGGGCCCGGTGTAT GACGGAATACAAGCTCGTCGTGGTGGGCGCCGATGGCGTCGGCAA GTCGGCACTGACTATCCAGCTCATACAAAATCATTTTGTTGATGAACT TTTATCCGTCGGTGGAGTGGGGGCGGACGGAGTGGGGAAGCTGCT GAGCGTTGGTGGGATGACCGAATACAAACTGGTGGTGGTCGGGGC GTGTGGGGTGGGCAAATCAGCCCTCACAATACAGCTGATTCAGAAC CACTTTGTTGACGAGCTTCTGTCCGTGGGCGGGGTGGGGGCATGTG GAGTGGGGAAGCTCCTGTCCGTAGGGGGCATGACTGAGTACAAGCT GGTTGTTGTAGGTGCTGTTGGAGTGGGAAAGAGCGCCCTCACAATC CAATTGATCCAGAATCACTTCGTCGACGAGCTCTTGAGCGTTGGAGG TGTGGGGGCCGTAGGCGTGGGCAAGCTGCTATCTGTGGGTGGAAT GACCGAATATAAGTTGGTAGTGGTGGGGGCTCGCGGAGTCGGGAAG AGTGCTCTTACTATTCAGCTGATTCAGAACCATTTCGTAGATGAACTC CTATCAGTCGGTGGCGTGGGCGCCAGAGGCGTGGGCAAACTGTTAT CAGTGGGCGGTACTGAGTACAAACTAGTAGTCGTAGGAGCTGGGTG TGTGGGCAAAAGTGCACTCACCATCCAGCTGATCCAGAACCATTTCG TGGATGAGTATTTGTTGAGTGTCGGAGGTGGAGCTGGCTGCGTGGG CAAAAGCCTGCTGTCCGTTGGGGGGACGGAATACAAACTAGTTGTT GTGGGGGCTGGTGATGTCGGAAAGTCCGCCTTGACTATTCAGCTTAT TCAAAATCACTTTGTCGACGAGTATCTTCTGAGCGTCGGCGGCGGC GCAGGTGACGTAGGAAAGTCTTTACTGTCAGTTGGGGGAACCTGCC TTCTGGACATCCTGGACACAGCCGGCCACGAGGAATACAGCGCCAT GCGGGATCAGTATATGAGGACAGGAGAAGGCTTCCTCTGCCTCTTAA GTGTCGGCGGTACAGCTGGCCACGAGGAGTATTCTCTGCTGTCTGT CGGAGGCACCGAGTACAAACTTGTGGTGGTTGGAGCAGGGCGTGTC | (SEQ ID NO: 15) |

(continued)

| | Full-length mRNA sequence | ORF |
|---|---|---|
| | GGCAAATCTGCCCTGACCATCCAGCTGATACAAAACCATTTTGTGGA TGAGTATTTACTTTCCGTGGGAGGCGGTGCGGGACGAGTGGGCAAG AGCTAATGACTCGAGGTGTGTGGAGGACACCCTGAACCCCCCGCTT TCAAACAAGTTTTCAAATTGTTTGAGGTCAGGATTTCTCAAACTGATT CCTTTCTTTGCATATGAGTATTTGAAAATAAATATTTTCCCAGAATATA AATAAATCATCACATGATTATTTTAACTATGCTAGCAAAAAAAAAAAAA AAAAAAAAAAAAAAAAAAAAAAAAAAAAAAAAAAAAAAAAAAAAAAA AAAAAAAAAAAAAAAAAAAAAAAAAAAAAAAAAAAAAAAGAAGAGCGA ATTC (SEQ ID NO: 299) | |
| Candidate Substance 17 | AAGCTTTAATACGACTCACTATAAGAATCCAGTCAACGCATATCCACG TATACCCGAACACATCACAGCCACCATGGACATGAGGGTGCCCGCC CAGTTGCTGGGGCTGCTGCTCTTGTGGCTCAGAGGGGCACGGTGCA TGACGGAATACAAGCTCGTCGTGGTGGGCGCCGATGGCGTCGGCAA GTCGGCACTGACTATCCAGCTCATACAAAATCATTTTGTTGATGAACT TTTATCCGTCGGTGGAGTGGGGGCGGACGGAGTGGGGAAGCTGCT GAGCGTTGGTGGGATGACCGAATACAAACTGGTGGTGGTCGGGGC GTGTGGGGTGGGCAAATCAGCCCTCACAATACAGCTGATTCAGAAC CACTTTGTTGACGAGCTTCTGTCCGTGGGCGGGGTGGGGGCATGTG GAGTGGGGAAGCTCCTGTCCGTAGGGGGCATGACTGAGTACAAGCT GGTTGTTGTAGGTGCTGTTGGAGTGGGAAAGAGCGCCCTCACAATC CAATTGATCCAGAATCACTTCGTCGACGAGCTCTTGAGCGTTGGAGG TGTGGGGGCCGTAGGCGTGGGCAAGCTGCTATCTGTGGGTGGAAT GACCGAATATAAGTTGGTAGTGGTGGGGGCTCGCGGAGTCGGGAAG AGTGCTCTTACTATTCAGCTGATTCAGAACCATTTCGTAGATGAACTC CTATCAGTCGGTGGCGTGGGCGCCAGAGGCGTGGGCAAACTGTTAT CAGTGGGCGGTACTGAGTACAAACTAGTAGTCGTAGGAGCTGGGTG TGTGGGCAAAAGTGCACTCACCATCCAGCTGATCCAGAACCATTTCG TGGATGAGTATTTGTTGAGTGTCGGAGGTGGAGCTGGCTGCGTGGG CAAAAGCCTGCTGTCCGTTGGGGGGACGGAATACAAACTAGTTGTT | (SEQ ID NO: 17) |

(continued)

| | Full-length mRNA sequence | ORF |
|---|---|---|
| | GTGGGGGCTGGTGATGTCGGAAAGTCCGCCTTGACTATTCAGCTTATTCAAAATCACTTTGTCGACGAGTATCTTCTGAGCGTCGGCGGCGGCGCAGGTGACGTAGGAAAGTCTTTACTGTCAGTTGGGGGAACCTGCCTTCTGGACATCCTGGACACAGCCGGCCACGAGGAATACAGCGCCATGCGGGATCAGTATATGAGGACAGGAGAAGGCTTCCTCTGCCTCTTAAGTGTCGGCGGTACAGCTGGCCACGAGGAGTATTCTTAATGACTCGAGGTGTGTGGAGGACACCCTGAACCCCCCGCTTTCAAACAAGTTTTCAAATTGTTTGAGGTCAGGATTTCTCAAACTGATTCCTTTCTTTGCATATGAGTATTTGAAAATAAATATTTTCCCAGAATATAAATAAATCATCACATGATTATTTTAACTATGCTAGCAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAAGAAGAGCGAATTC (SEQ ID NO: 300) | |

## Example 3: Evaluation of anticancer efficacy of mRNA encoding Antigen 2 linked to KRAS variant peptide in Lewis lung cancer mouse model

The anticancer efficacy of a vaccine containing mRNA encoding Antigen 2 linked to KRAS variant peptide according to the present disclosure was evaluated. The vaccine containing the mRNA encoding Antigen 2 was administered to a Lewis lung carcinoma (LL/2) syngeneic mouse model, in which Lewis lung carcinoma cells (LL/2) had been allografted, and antitumor efficacy was assessed by observing tumor size changes following administration. LL/2 cells are a murine lung cancer cell line established from the lungs of C57BL mice bearing tumors induced by implantation of primary Lewis lung cancer.

The administration group was administered the vaccine at a dose of 30 μg/head/week per subject for 5 weeks. The mRNA-containing vaccine was formulated to be loaded into lipid nanoparticles (LNPs) in PBS. The lipid nanoparticles (LNPs) contained ionizable lipid (ALC0315, 46.3 mol%), phospholipid (DSPC, 9.4 mol%), cholesterol (42.7 mol%), and PEG-lipid (ALC0159, 1.6 mol%) in PBS. The concentration of the loaded mRNA was 0.3 mg/mL. The same formulation of the mRNA-containing vaccine was used throughout the examples described herein. Specifically, after the first administration, LL/2 cells (ATCC), cultured in DMEM medium in a cell culture flask, were subcutaneously injected into the dorsal side of C57BL/6 female mice (6 weeks old, average body weight 18 g, Daehan Bio). A few days later, when tumors became visible to the naked eye, tumor size was monitored over time. Each group was assigned to seven animals with similar body weight. The administration group received 50 μl of the vaccine at a dose of 15 μg/head via intramuscular injection twice a week.

The tumor size of each group was observed for up to Day 26 from the date of tumor injection. FIG. 2 is a diagram showing tumor size and body weight changes after administration of an anti-cancer vaccine containing mRNA encoding Antigen 2 in a Lewis lung cancer mouse model. As shown in FIG. 2, the anti-cancer vaccine containing mRNA encoding Antigen 2 exhibited a significant tumor growth inhibition effect compared to the vehicle-treated group in the Lewis lung cancer mouse model (FIG. 2A). In addition, there was no decrease in mouse body weight after continuous vaccination for 5 weeks, indicating that the vaccine was safe in terms of side effects (FIG. 2B). According to previous reports, Lewis lung tumor is known as a representative immunosuppressive tumor model in which immunosuppressive cells such as myeloid derived suppressor cells (MDSC) and tumor-associated macrophages (TAM) are mainly distributed (Viitala et al., 2019, Clin Cancer Res (2019) 25 (11): 3289-3303).

Additionally, tumors were isolated from the mice, and tumor tissue cells were separated therefrom to perform flow

cytometry analysis (FACS analysis). Using flow cytometry, the proportions of CD8+ T cells (CD3+CD8+ cells), memory/effector CD8+ T cells (CD3+CD8+CD44+ cells), and regulatory T cells (regulatory T cells) Treg cells (CD3+CD8+Foxp3+ cells) in the tumor tissue cells were confirmed.

After administering an mRNA-containing anticancer vaccine to a Lewis lung cancer mouse model, changes in the distribution of immune cells among tumor tissue cells were confirmed. As a result, in the group treated with the mRNA-containing anticancer vaccine which encodes Antigen 2, the proportions of CD8+ T cells (CD3+CD8+ cells) and memory/effector CD8+ T cells (CD3+CD8+CD44+ cells) in the tumor tissue increased (FIGS. 3A and 3B), while the proportion of regulatory T cells (Treg; CD3+CD8+Foxp3+ cells), known to suppress immune responses, decreased (FIG. 3C).

These results indicate that the KRAS mRNA anticancer vaccine exerts its anticancer efficacy by promoting the proliferation of tumor-killing cells such as CD8+ T cells (CD3+CD8+ cells) and memory/effector CD8+ T cells (CD3+CD8+CD44+ cells), while suppressing the proliferation of regulatory T cells (Tregs; CD3+CD8+Foxp3+ cells), which inhibit immune responses. Moreover, the observation of anti-tumor effects even in Lewis lung tumors-which are known to be a representative immunosuppressive tumor model-suggests that the KRAS mRNA anticancer vaccine may exhibit potent anticancer efficacy regardless of the tumor immune microenvironment.

This demonstrates that the KRAS mRNA anticancer vaccine is effective in immunosuppressive tumors.

## Example 4: Evaluation of anticancer efficacy of mRNA encoding Antigen 6 linked to KRAS variant peptide in a Lewis lung cancer mouse model

The anticancer efficacy of an anticancer vaccine containing mRNA encoding Antigen 6 linked to a KRAS variant peptide according to the present disclosure was evaluated. After administering the mRNA-containing vaccine to a Lewis lung cancer mouse model (LL/2 tumor syngeneic mouse model), the antitumor efficacy was evaluated by observing changes in tumor size. The administration group was administered the vaccine at a dose of 20 μg/head/week per individual for 5 weeks. The mRNA-containing vaccine has the same composition as described in Example 3. Specifically, following the first administration, LL/2 cells (ATCC), cultured in DMEM medium in a cell culture flask, were subcutaneously injected into the dorsal side of C57BL/6 female mice (6 weeks old, average body weight 18 g, Daehan Bio). A few days later, when tumors became visibly apparent, tumor sizes were monitored. Each group was composed of seven mice with similar body weights. The administration group received intramuscular injections of the vaccine at a dose of 10 μg/head (50 μL per dose), twice per week.

The tumor size of each group was observed for up to Days 26 from the date of tumor injection. After administering an anticancer vaccine containing mRNA encoding Antigen 6 to a Lewis lung cancer mouse model, the anticancer vaccine containing mRNA encoding antigen 6 showed significant tumor growth inhibition efficacy compared to the vehicle-administered group (FIG. 4A). Additionally, no reduction in body weight was observed in mice over the 5-week vaccination period, confirming its safety in terms of side effects (FIG. 4B).

Furthermore, flow cytometry (FACS) analysis was performed on tumors isolated from the mice, and the frequency of CD8+ T cells (CD3+CD8+ cells) within the tumor tissue was examined.

After administering an mRNA-containing anticancer vaccine to a Lewis lung cancer mouse model, changes in the distribution of immune cells among tumor tissue cells were confirmed. When an anticancer vaccine containing mRNA encoding Antigen 6 was administered, the proportion of CD8+ T cells (CD3+CD8+ cells) among tumor tissue cells increased (FIG. 5).

These results indicate that the KRAS mRNA anticancer vaccine exerts its anticancer efficacy by inducing the proliferation of cells involved in tumor killing, such as CD8+ T cells (CD3+CD8+ cells). Moreover, the observation of anti-tumor effects in Lewis lung tumors-known to be representative immunosuppressive tumor models-suggests that the KRAS mRNA anti-cancer vaccine may exhibit strong anticancer efficacy regardless of the cancer immune microenvironment.

This indicates that the KRAS mRNA anticancer vaccine is effective in immunosuppressive tumors.

## Example 5: Evaluation of anticancer efficacy of mRNA encoding Antigen 14 or Antigen 15 linked to KRAS variant peptide in a Lewis lung cancer mouse model

The anticancer efficacy of an anticancer vaccine containing mRNA encoding Antigen 14 or Antigen 15 linked to a KRAS variant peptide according to the disclosure was evaluated. After administering the mRNA-containing vaccine to a Lewis lung cancer mouse model (LL/2 tumor syngeneic mouse model), the antitumor efficacy was evaluated by observing changes in tumor size. The administration group was administered the above vaccine at a dose of 30 μg/head/week per subject for 5 weeks. The mRNA-containing vaccine used was the same as that described in Example 3. Specifically, after the first administration, LL/2 cells (ATCC) cultured in DMEM medium in a cell culture flask were subcutaneously injected into the dorsal side of C57BL/6 female mice (6 weeks old, average body weight 18 g; Daehan Bio). A few days later, when

tumors became visible to the naked eye, tumor sizes were monitored over time.. Each group was assigned to seven animals with similar body weight. The administration group received 50 μl of the vaccine at 15 μg/head per session via intramuscular injection twice a week.

Tumor sizes in each group were monitored up to Day 26 from the day of tumor implantation. As a result, the anticancer vaccine containing mRNA of SEQ ID NO: 14 or 15 showed significant tumor growth inhibition compared to the vehicle-treated group (FIG. 6A). In addition, mRNA encoding Antigen 14 or Antigen 15 was confirmed to be safe in terms of side effects as no decrease in mouse body weight was observed even when continuous vaccination was administered for 5 weeks (FIG. 6B).

These results indicate that the anticancer vaccine containing mRNA encoding Antigen 14 or 15 exerts anticancer efficacy by inducing the proliferation of cells involved in tumor death.

Moreover, the observation of anticancer effects in Lewis lung tumors-which are known as representative immunosuppressive tumor models-suggests that the KRAS mRNA anti-cancer vaccine may demonstrate strong anti-tumor efficacy regardless of the tumor immune microenvironment.

This indicates that the KRAS mRNA anticancer vaccine is effective in immunosuppressive tumors.

## Example 6: Determination of cell viability during co-culture of mRNA-transfected human peripheral blood mononuclear cells and target cells

In this example, it was evalutated whether immune cells expressing mRNA encoding Antigens 2, 6, 15 and 17 linked to KRAS variant peptides specifically responded to target cells. To this end, a co-culture experiment was conducted between human peripheral blood mononuclear cells (PBMCs) transfected with the mRNA and target cells. The target cells included cancer cell lines expressing wild-type KRAS and cancer cell lines expressing mutant KRAS, respectively. The mRNA was synthesized through in vitro transcription. For in vivo experiments, the mRNA was delivered via LNP-loaded formulations, whereas in cell-based experiments, transfection was performed using Lipofectamine.

PBMCs were isolated from human blood collected from healthy donors using density gradient centrifugation with Histopaque®-1077 (Sigma, Cat. No. 10771). The isolated PBMCs were cultured for 2 hours in RPMI-1640 medium supplemented with 10% FBS and 1 % P/S, then seeded at $1.5 \times 10^6$ cells/well in 1.5 mL medium in a 6-well plate. The mRNAs encoding each KRAS variant peptide-linked antigen were mixed with Lipofectamine™ MessengerMAX™ Transfection Reagent (Thermo Scientific™, Cat. No. LMRNA001) at a ratio of 0.5 μL Lipofectamine per 1 μg mRNA and incubated for 20 minutes to form Lipofectamine/mRNA complexes. The reaction product corresponding to 5 μg of mRNA was added to the seeded PBMCs and cultured for 36 hours at 37°C in 5% $CO_2$. As a result, PBMCs transfected with the respective mRNAs were obtained.

For the target cells, MRC-5 ($0.5 \times 10^4$ cells/well, 10% FBS DMEM), H1299 ($1 \times 10^4$ cells/well, 10 % FBS RPMI-1640), and AsPC-1 ($1 \times 10^4$ cells/well, 10 % FBS RPMI-1640) were seeded into 96-well plates 24 hours prior to co-culture, allowing for adherence to the plate surface. To the adhered target cells, the mRNA-transfected PBMCs ($6 \times 10^4$ cells/well, 10 % FBS RPMI-1640) were added at effector-to-target (E:T) ratios of 1:12 or 1:6, and co-cultured for 48 hours at 37 °C in 5 % $CO_2$.

The cells were then stained with sulforhodamine B (SRB) (Sigma, Cat. No. S1402) and was stained with Cytation 5 Imaging system (Agilent, Cat No. S1402), and images were acquired using the Cytation 5 Imaging System (Agilent, Cat. No. 132BR022520). In addition, Tris (Sigma, Cat. No. 10708976001) was added to the wells to lyse the cells, and absorbance was measured at 540 nm. The absorbance at 540 nm was measured to calculate the cell viability (%) shown in FIGS. 7B and 8B.

In FIG. 7A, the "Control" refers to the result of culturing MRC-5, H1299, and AsPC-1 cells alone, without target cells. MRC-5 cells are normal cells derived from human embryonic lung fibroblasts. H1299 cells are a human non-small cell lung carcinoma cell line derived from lymph node tissue, which expresses wild-type KRAS protein. AsPC-1 cells are a nude mouse xenograft-derived cell line initiated from ascites-derived cells of a human pancreatic cancer patient, and are a pancreatic cancer cell line expressing the G12D mutant KRAS.

Additionally, among the negative controls, "Ctrl" and "Lipo" represent PBMCs not transfected with mRNA and co-cultured with PBMC cells transfected in the absence of mRNA and in the presence of Lipofectamine, respectively. In Candidate Substances 2 and 6, 2.5 μg and 5.0 μg represent cases in which transfection was performed using 2.5 μg and 5.0 μg of mRNA encoding Antigens 2 and 6, respectively.

As shown in FIG. 7A, when MRC-5 and H1299 cell lines expressing KRAS wild type were co-cultured with PBMCs transfected with mRNA encoding Antigen 2 or Antigen 6 linked to KRAS variant peptides, there was no difference in the degree of sulforhodamine B staining compared to the negative control. In contrast, when AsPC-1 cells expressing the G12D mutant protein were co-cultured with PBMCs transfected with mRNA encoding Antigen 2 or Antigen 6 linked to the KRAS variant peptide, the degree of sulforhodamine B staining was reduced compared to the negative control. The cell viability was confirmed by staining the surviving cells with sulforhodamine B and measuring the absorbance at a wavelength of 540 nm, thereby quantifying it.

As shown in FIG. 7B, when MRC-5 and H1299 cell lines expressing KRAS wild type were co-cultured with hPBMCs transfected with mRNA encoding Antigen 2 or Antigen 6 linked to KRAS variant peptides, there was no difference in the survival rate of target cells compared to the negative control group. In contrast, when AsPC-1 cells expressing the KRAS G12D mutant protein were co-cultured with PBMCs transfected with mRNA encoding Antigen 2 or Antigen 6 linked to the KRAS variant peptide, the viability of target cells was reduced compared to the negative control.

In FIG. 7B, the survival rate of target cells was calculated according to the following formula.

$$\text{Target cell survival rate} = \text{Ti/Tc} \times 100$$

Tc: Absorbance of 100% growth control target cells alone
Ti: absorbance of target cells under co-culture conditions with PBMC

The results indicate that PBMCs transfected with mRNA encoding Antigen 2 or Antigen 6 linked to KRAS variant peptides selectively inhibit the growth of cancer cells expressing the G12D mutant KRAS, i.e., AsPC-1 cells.

In FIG. 8A, the control group represents the result of co-culturing BEAS-2B, MIA PaCa-2, AsPC-1, Capan-2, and H460 cells (seeded at $1 \times 10^4$ cells/well in 10 % FBS RPMI-1640) with PBMCs ($6 \times 10^4$ cells/well in 10 % FBS RPMI-1640) at a 1:6 ratio, without target cells.

BEAS-2B cells are epithelial cells derived from the bronchial epithelium of a noncancerous individual and represent a normal cell line. MIA PaCa-2 cells are an epithelial pancreatic cancer cell line derived from human pancreatic tumor tissue and express the G12C mutant form of KRAS.

AsPC-1 cells are a pancreatic cancer cell line derived from a xenograft established using ascites fluid from a human pancreatic cancer patient, and express the G12D mutant form of KRAS.

Capan-2 cells are a pancreatic cancer cell line with polygonal morphology, isolated from the pancreas of a human pancreatic cancer patient, and express the G12V mutant form of KRAS.

H460 cells are a lung cancer cell line derived from pleural fluid of a patient with large cell lung cancer, and express the Q61H mutant form of KRAS.

Additionally, among the negative controls, "Control" refers to co-culture with PBMCs that were not transfected with mRNA, and "Lipofectamine" refers to co-culture with PBMCs transfected in the absence of mRNA but in the presence of Lipofectamine. In Candidate Substances 15 and 17, 2.5 μg and 5.0 μg represent cases in which transfection was performed using 2.5 μg and 5.0 μg of mRNA encoding Antigens 15 and 17, respectively.

As shown in Fig. 8A, when BEAS-2B, a normal cell line expressing KRAS wild type, was co-cultured with PBMCs transfected with mRNA encoding Antigen 15 or 17 linked to KRAS variant peptides, there was no difference in the degree of sulforhodamine B staining compared to the negative control. In contrast, when MIA PaCa-2, AsPC-1, Capan-2, and H460 cell lines expressing KRAS G12C, G12D, G12V, and Q61H mutant proteins, respectively, were co-cultured with PBMCs transfected with mRNA encoding Antigen 15 or 17 linked to KRAS variant peptides, the degree of sulforhodamine B staining was reduced compared to the negative control.

As shown in FIG. 8B, when the normal cell line BEAS-2B cell line, which is a cell line expressing the wild type KRAS, was co-cultured with PBMCs transfected with mRNA encoding Antigen 15 or 17 linked to a KRAS variant peptide, there was no difference in the survival rate of target cells compared to the negative control group. In contrast, when MIA PaCa-2, AsPC-1, Capan-2, and H460 cell lines expressing KRAS G12C, G12D, G12V, and Q61H mutant proteins, respectively, were co-cultured with PBMCs transfected with mRNA encoding Antigen 15 or 17 linked to KRAS variant peptides, the viability of target cells decreased compared to the negative control.

In FIG. 8B, the survival rate of target cells was calculated according to the following formula.

$$\text{Target cell survival rate} = \text{Ti/Tc} \times 100$$

Tc: Absorbance of 100% growth control target cells alone
Ti: absorbance of target cells under co-culture conditions with PBMC

The results above indicate that PBMCs transfected with mRNA encoding Antigen 15 or 17 linked to KRAS variant peptides selectively inhibit the growth of only cancer cells expressing KRAS G12C, G12D, G12V, and Q61H mutant proteins, namely MIA PaCa-2, AsPC-1, Capan-2, and H460 cells.

The results in FIGS. 7A, 7B, 8A, and 8B demonstrate that immune cells, such as PBMCs, transfected with mRNA encoding an antigen linked to a KRAS variant peptide selectively inhibit only cancer cells expressing the KRAS variant peptide. Therefore, the antigen linked to the above KRAS variant peptide may be used to specifically inhibit cancer cells

without acting on normal cells.

**Example 7: Confirmation of expression of KRAS mutant mRNA-based anticancer agent in LL/2 cells**

In this example, the mRNAs encoding each KRAS variant peptide-linked antigen were delivered into LL/2 cells using Lipofectamine™ MessengerMAX™ Transfection Reagent (Thermo Scientific™, Cat. No. LMRNA001), and the expression of each antigen was confirmed. For this purpose, the following in vitro experiment was conducted. LL/2 cells were seeded in 6-well plates at a density of $4 \times 10^5$ cells/2 mL/well and incubated for 24 hours. Each mRNA was mixed with Lipofectamine™ MessengerMAX™ Transfection Reagent at a ratio of 0.5 μL per 1 μg of mRNA and incubated for 20 minutes. The resulting complex was added to the wells containing cultured LL/2 cells in an amount equivalent to 5 μg of mRNA, and the cells were incubated at 37 °C for 24 hours. At this time, DMEM medium was used.

After the incubation, 100 μL/well of lysis buffer and M-PER™ (Mammalian Protein Extraction Reagent) (Thermo Scientific™, Cat. No. 78501) were added to each well to lyse the cells and extract proteins. Protein quantification was performed using the Pierce™ BCA Protein Assay Kit (Thermo Scientific™, Cat. No. 23225). The obtained data were used to determine the amount of protein used in the experiment. The extracted samples were diluted with lysis buffer and 5× reducing dye (containing 10 % SDS, 0.5% bromophenol blue, 50 % glycerol, 0.5 % 2-mercaptoethanol, and 250 mM Tris, pH 6.8) to equalize the protein concentrations.

Western blot analysis was performed on the diluted samples using an anti-RAS (G12D mutant) monoclonal antibody (CST, Cat. No. #14429S) and anti-β-actin antibody (Abcam, Cat. No. ab8227).

FIG. 9 shows the results of Western blotting using the anti-RAS (G12D mutant) monoclonal antibody for proteins expressed in LL/2 cells, a Lewis lung carcinoma cell line, into which mRNAs encoding KRAS variant peptide-linked Antigens 6, 14, and 15 were introduced.

As shown in FIG. 9, Antigens 6, 14, and 15, each encoding linked KRAS variant peptides, exhibited different molecular size and expression levels within the cells. When comparing expression levels with Antigen 6 as a control, Antigens 14 and 15 showed relatively higher expression, though still lower than that of Antigen 6.

These results suggest that the expression level of an antigen may vary depending on factors such as the amino acid sequence, composition, length of the KRAS variant peptides included in the antigen, and the overall sequence length. For example, among antigens containing the G12D variant peptide, the level of G12D protein expression may differ depending on sequence design, including the peptide's own sequence, its length, and the composition between peptides.

**Claims**

1. An antigenic peptide in which two or more KRAS variant peptides selected from the group consisting of KRAS variant peptides are linked, wherein the antigenic peptide comprises G12C, G12D, and G13D variant peptides.

2. The antigenic peptide of claim 1, wherein the variant peptide is an antigenic peptide having a length of 8, 17, or 31 amino acids (aa).

3. The antigenic peptide of claim 1, further comprising one or more of G12V, G12R, G13C, G13R, and Q61H variant peptides.

4. The antigenic peptide of claim 1, comprising G12C, G12D, G12V, G12R, G13C, G13D, and Q61H variant peptides, wherein each variant peptide has a length of 17 aa.

5. The antigenic peptide of claim 3, wherein the variant peptides have a length of 8 aa or 31 aa.

6. The antigenic peptide of claim 5, comprising linked variant peptides having identical mutant residues and having lengths of 8 aa and 31 aa.

7. The antigenic peptide of claim 6, comprising G12C, G12D, G12V, G12R, G13C, G13D, G13R and Q61H variant peptides.

8. The antigenic peptide of claim 1, wherein one or more of the variant peptides are linked to each other via a linker.

9. The antigenic peptide of claim 6, wherein the linker is G4S (SEQ ID NO: 552) or LLSVGG (SEQ ID NO: 553).

10. The antigenic peptide of claim 1, comprising the amino acid sequence of SEQ ID NO: 24, 28, 36, or 37.

**11.** An mRNA encoding the antigenic peptide of any one of claims 1 to 10.

**12.** The mRNA of claim 11, comprising the nucleotide sequence of SEQ ID NO: 2, 6, 14, 15, 296, 297, 298, 299, or 300.

**13.** An immunogenic composition comprising any one of antigenic peptides of claims 1 to 10, or an mRNA encoding the antigenic peptide.

**14.** The immunogenic composition of claim 13, for preventing or treating cancer.

**15.** The immunogenic composition of claim 13, wherein the mRNA comprises a nucleotide sequence of SEQ ID NO: 2, 6, 14, 15, 296, 297, 298, 299, or 300.

**16.** The immunogenic composition of claim 15, wherein the mRNA is bound to a lipid nanoparticle.

**17.** The immunogenic composition of claim 14, wherein the cancer is pancreatic cancer or lung cancer.

**18.** A method of inducing an immune response to KRAS variant peptides in a subject, comprising administering to the subject any one of antigenic peptides of claims 1 to 10, or an mRNA encoding the antigenic peptide.

**19.** The method of claim 18, for preventing or treating cancer.

**20.** The method of claim 19, wherein the cancer is pancreatic cancer or lung cancer.

FIG. 1

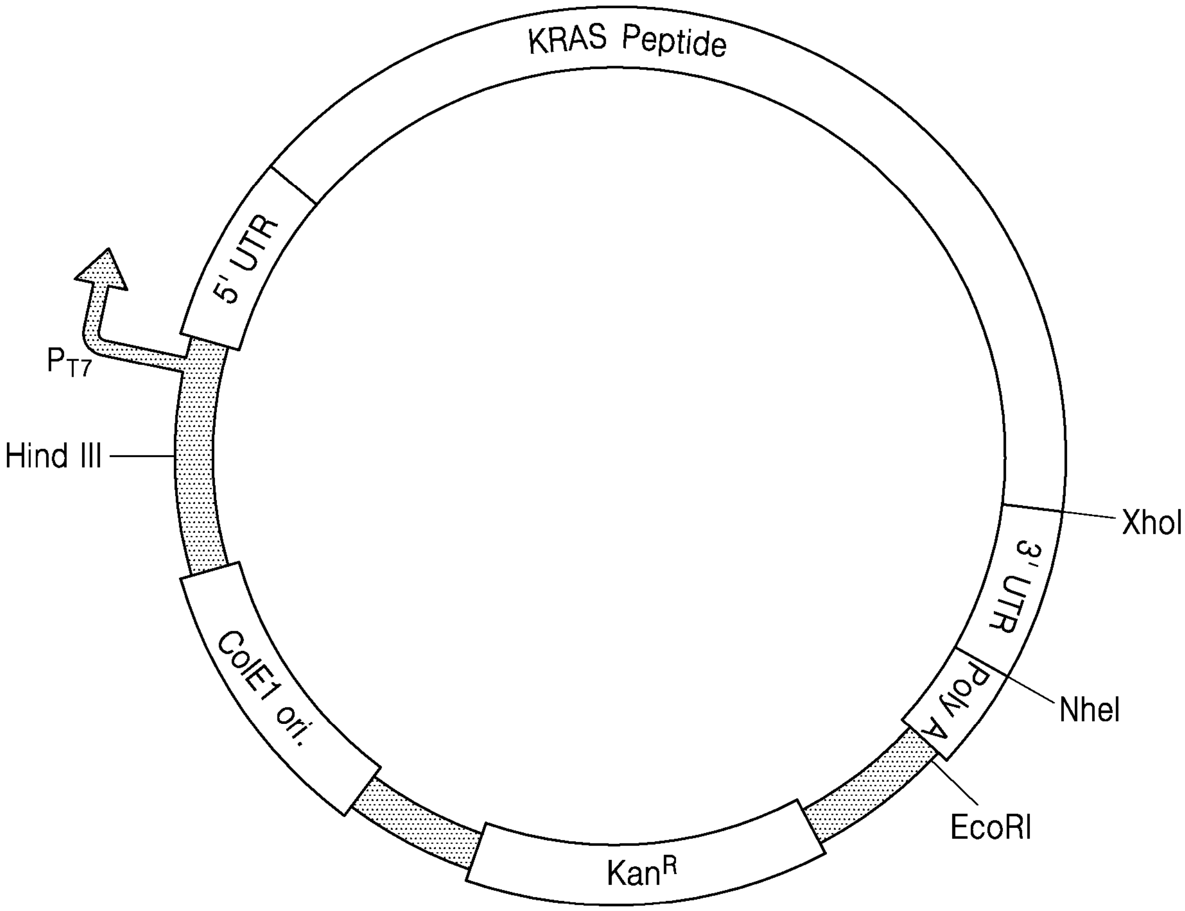
KRAS Peptide
5' UTR
P T7
Hind III
XhoI
3' UTR
Poly A
NheI
EcoRI
ColE1 ori.
KanR

FIG. 2

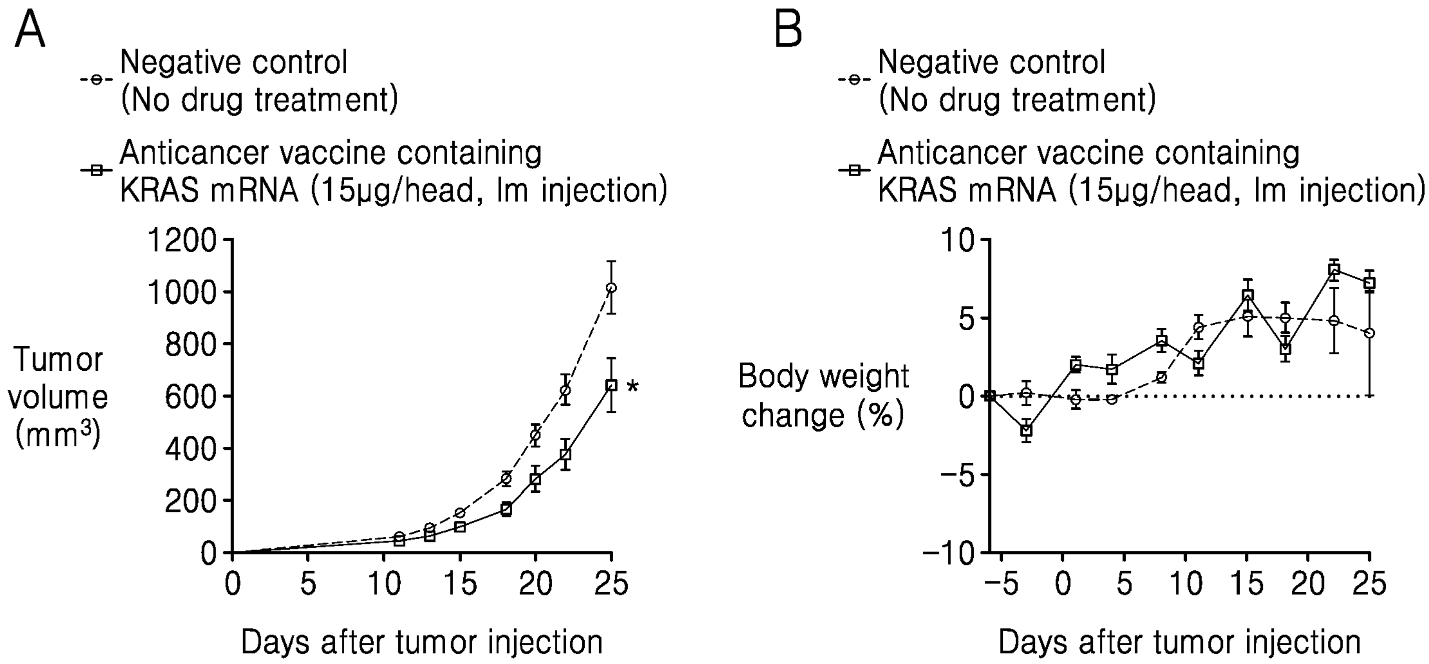
A
Negative control
(No drug treatment)
Anticancer vaccine containing
KRAS mRNA (15μg/head, Im injection)
Tumor volume (mm3)
1200
1000
800
600
400
200
0
0
5
10
15
20
25
*
Days after tumor injection
B
Negative control
(No drug treatment)
Anticancer vaccine containing
KRAS mRNA (15μg/head, Im injection)
Body weight change (%)
10
5
0
−5
−10
−5
0
5
10
15
20
25
Days after tumor injection

FIG. 3

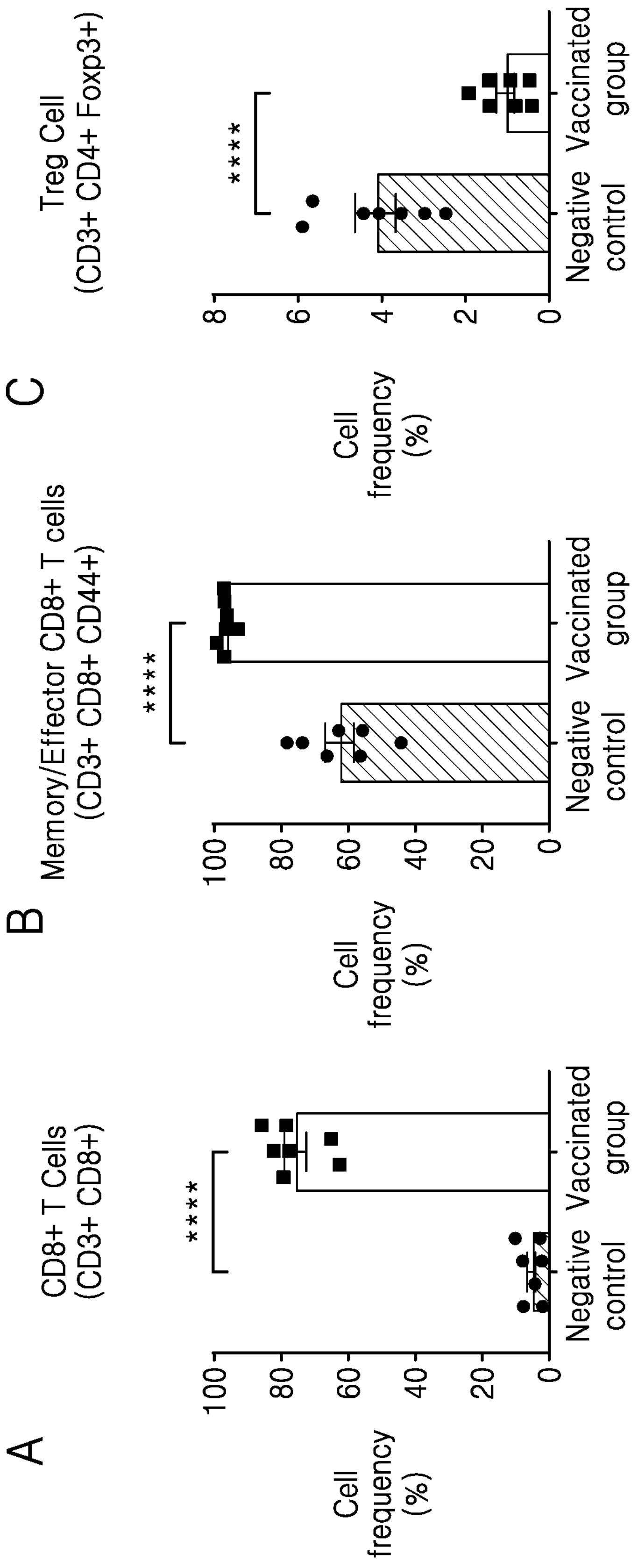
A
CD8+ T Cells
(CD3+ CD8+)
Cell frequency (%)
0
20
40
60
80
100
****
Negative control
Vaccinated group
B
Memory/Effector CD8+ T cells
(CD3+ CD8+ CD44+)
Cell frequency (%)
0
20
40
60
80
100
****
Negative control
Vaccinated group
C
Treg Cell
(CD3+ CD4+ Foxp3+)
Cell frequency (%)
0
2
4
6
8
****
Negative control
Vaccinated group

# FIG. 4

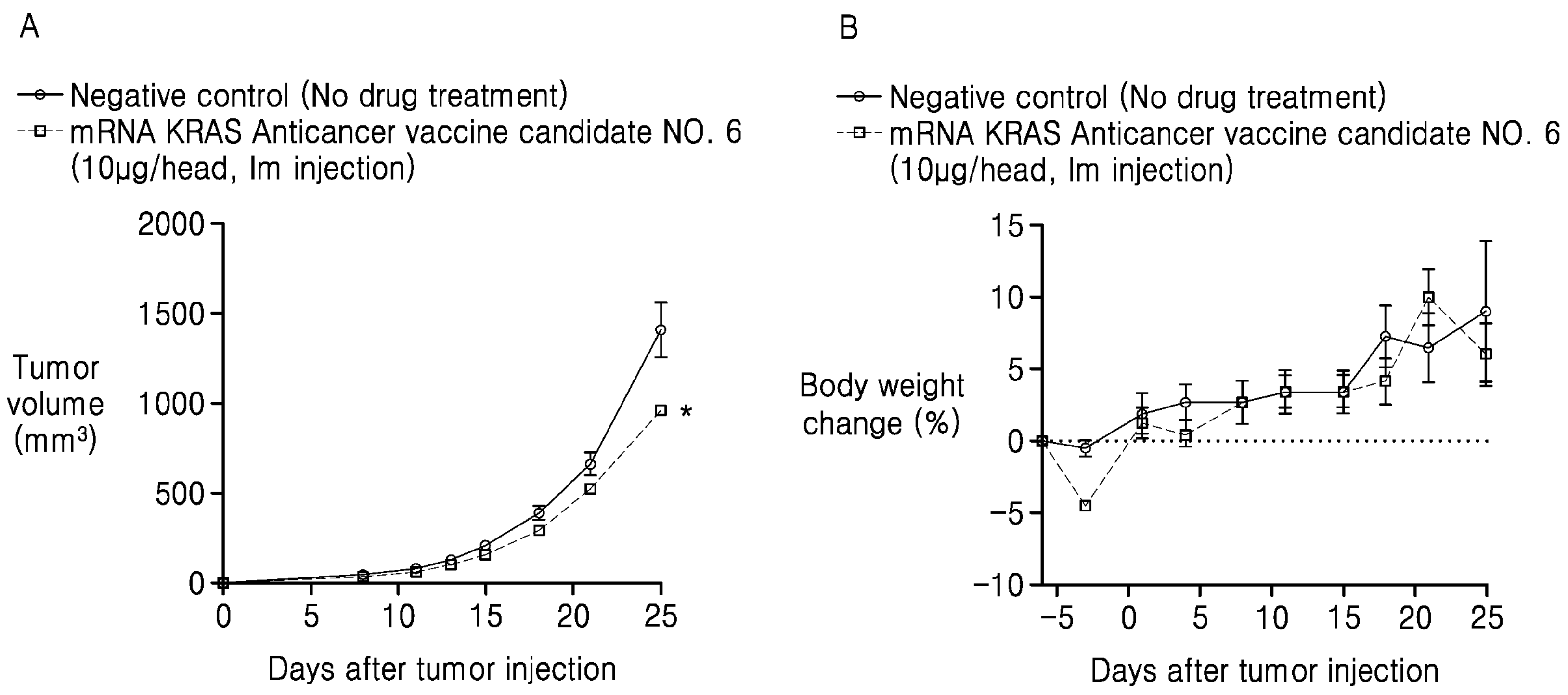
A
Negative control (No drug treatment)
mRNA KRAS Anticancer vaccine candidate NO. 6
(10μg/head, Im injection)
Tumor volume (mm³)
2000
1500
1000
500
0
*
0
5
10
15
20
25
Days after tumor injection
B
Negative control (No drug treatment)
mRNA KRAS Anticancer vaccine candidate NO. 6
(10μg/head, Im injection)
Body weight change (%)
15
10
5
0
−5
−10
−5
0
5
10
15
20
25
Days after tumor injection

FIG. 5

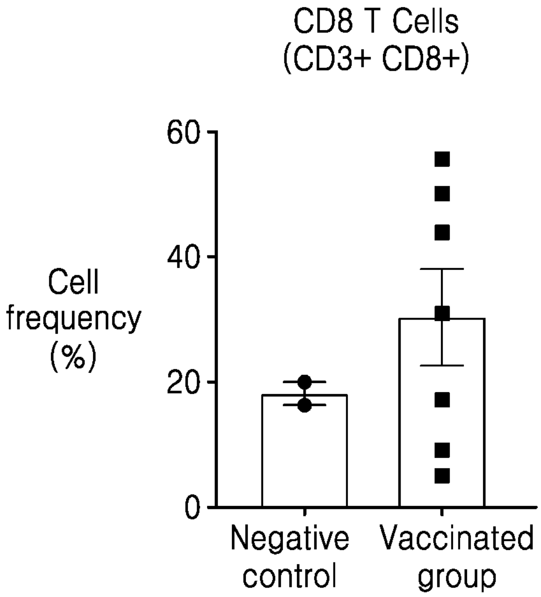
CD8 T Cells
(CD3+ CD8+)
Cell frequency (%)
60
40
20
0
Negative control
Vaccinated group

FIG. 6

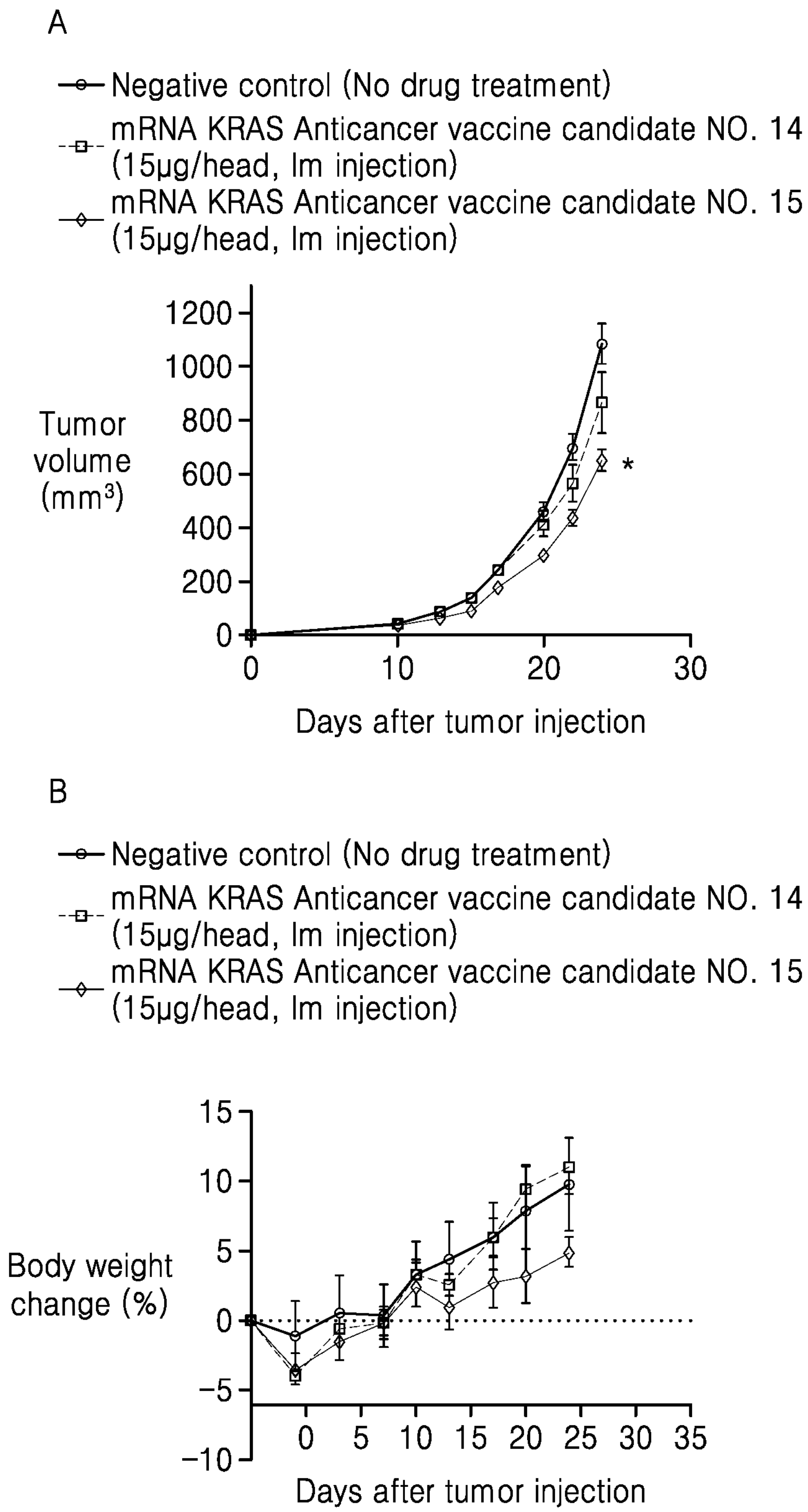
A
Negative control (No drug treatment)
mRNA KRAS Anticancer vaccine candidate NO. 14 (15μg/head, Im injection)
mRNA KRAS Anticancer vaccine candidate NO. 15 (15μg/head, Im injection)
Tumor volume (mm3)
1200
1000
800
600
400
200
0
*
0
10
20
30
Days after tumor injection
B
Negative control (No drug treatment)
mRNA KRAS Anticancer vaccine candidate NO. 14 (15μg/head, Im injection)
mRNA KRAS Anticancer vaccine candidate NO. 15 (15μg/head, Im injection)
Body weight change (%)
15
10
5
0
−5
−10
0
5
10
15
20
25
30
35
Days after tumor injection

FIG. 7A

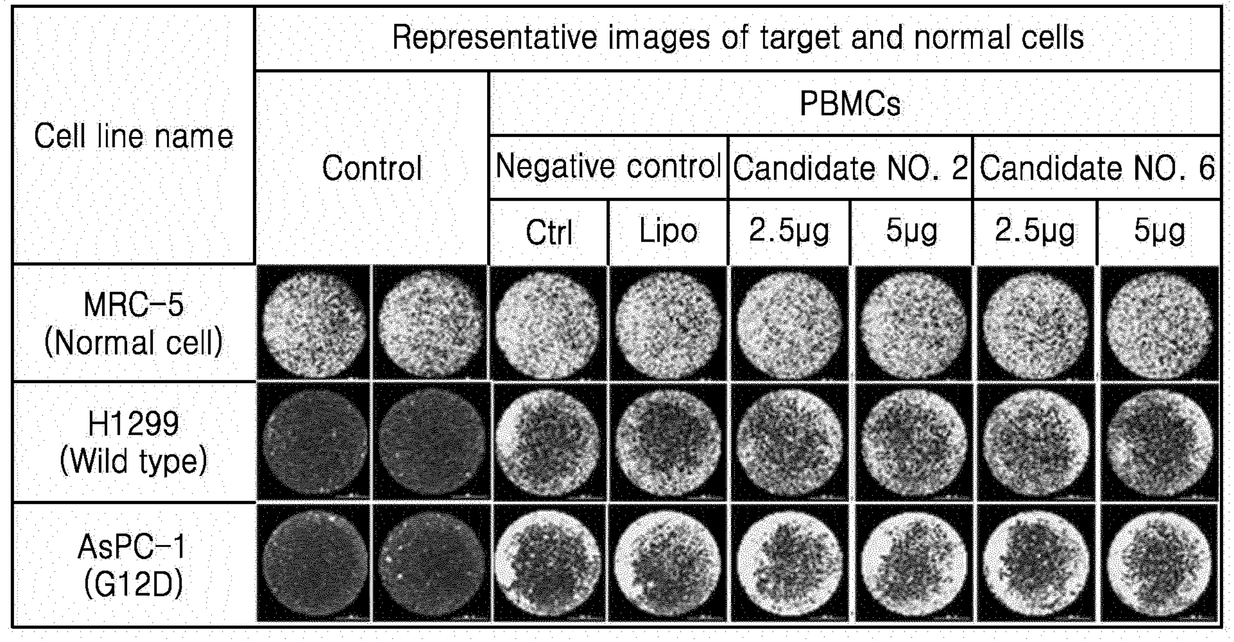
Cell line name
Representative images of target and normal cells
Control
PBMCs
Negative control
Candidate NO. 2
Candidate NO. 6
Ctrl
Lipo
2.5μg
5μg
2.5μg
5μg
MRC-5 (Normal cell)
H1299 (Wild type)
AsPC-1 (G12D)

FIG. 7B

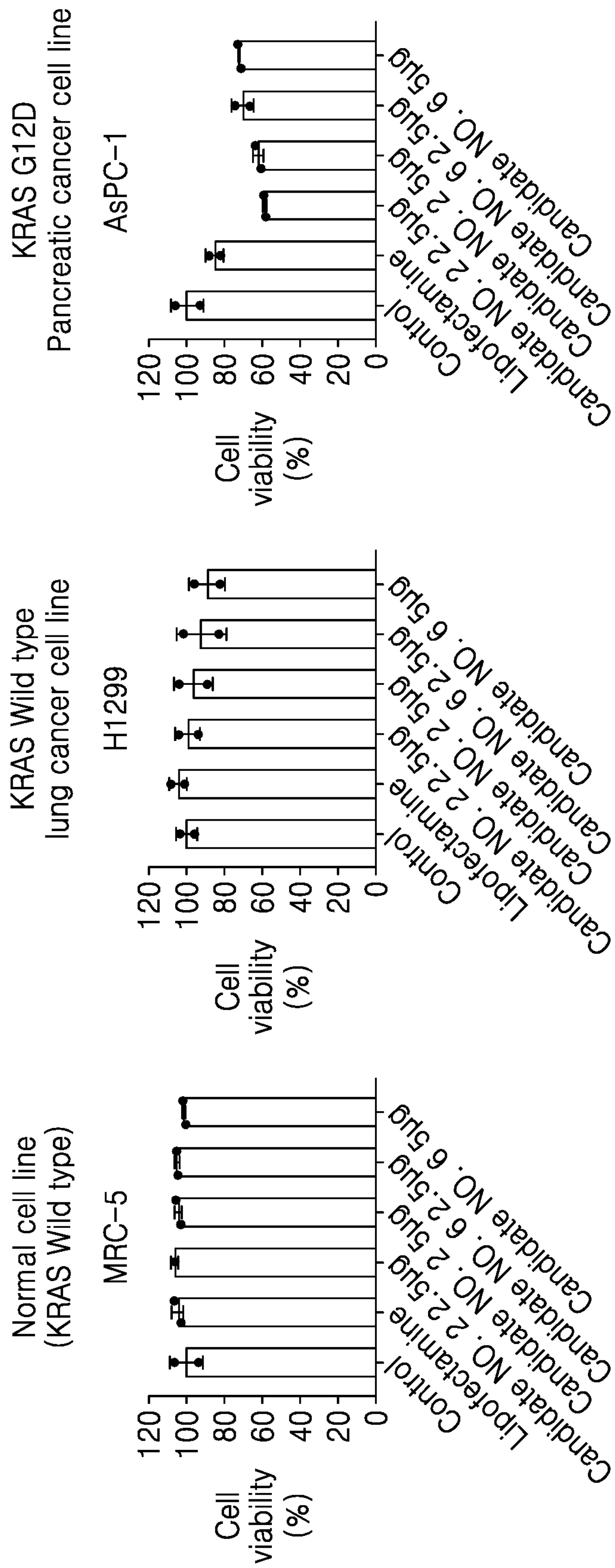

KRAS G12D
Pancreatic cancer cell line
AsPC-1
KRAS Wild type
lung cancer cell line
H1299
Normal cell line
(KRAS Wild type)
MRC-5
Cell viability (%)
0
20
40
60
80
100
120
Control
Lipofectamine
Candidate NO. 2 2.5μg
Candidate NO. 2 5μg
Candidate NO. 6 2.5μg
Candidate NO. 6 5μg

FIG. 8A

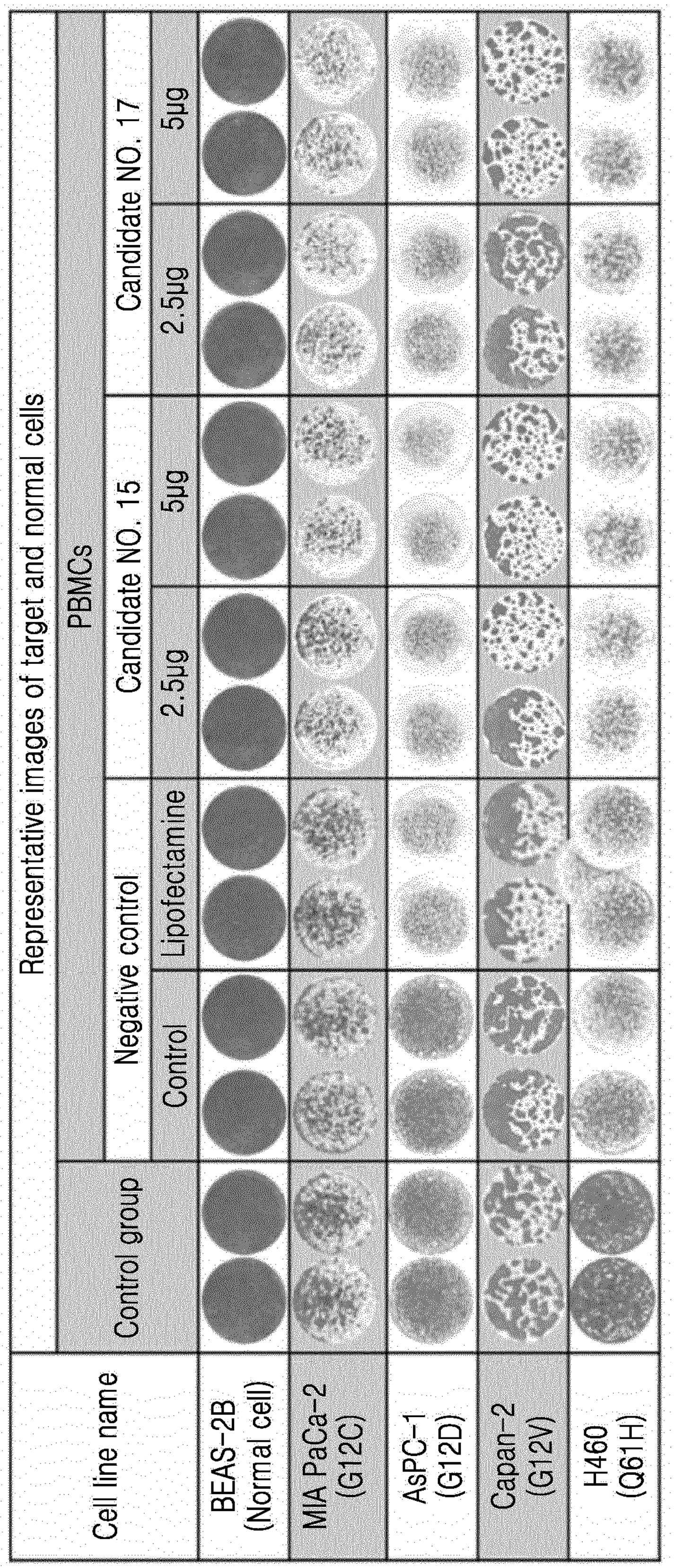
Representative images of target and normal cells
Cell line name
Control group
PBMCs
Negative control
Control
Lipofectamine
Candidate NO. 15
Candidate NO. 17
2.5µg
5µg
BEAS-2B (Normal cell)
MIA PaCa-2 (G12C)
AsPC-1 (G12D)
Capan-2 (G12V)
H460 (Q61H)

# FIG. 8B

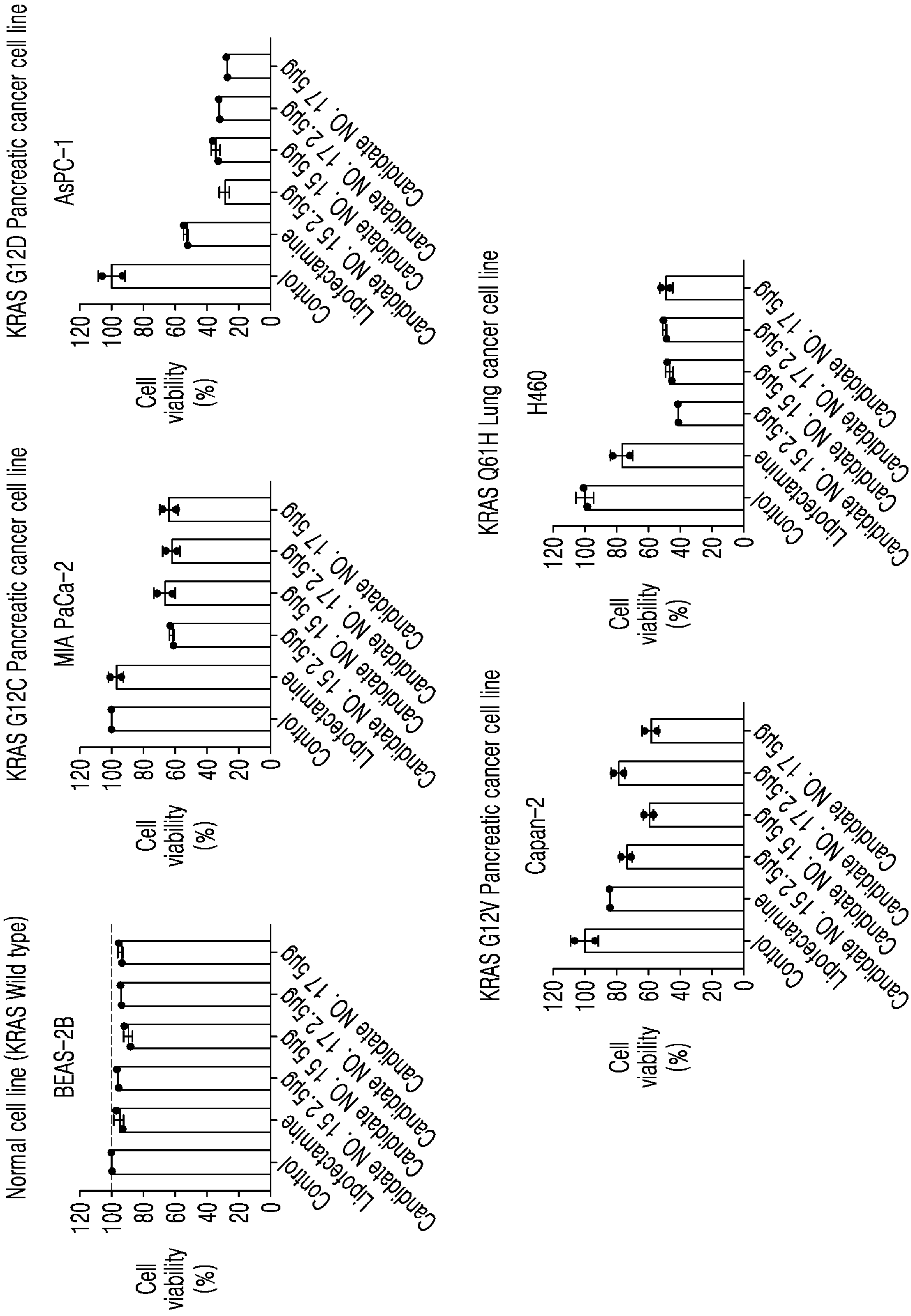
Normal cell line (KRAS Wild type)
BEAS-2B
KRAS G12C Pancreatic cancer cell line
MIA PaCa-2
KRAS G12D Pancreatic cancer cell line
AsPC-1
KRAS G12V Pancreatic cancer cell line
Capan-2
KRAS Q61H Lung cancer cell line
H460
Cell viability (%)
0
20
40
60
80
100
120
Control
Lipofectamine
Candidate NO. 15 2.5μg
Candidate NO. 15 5μg
Candidate NO. 17 2.5μg
Candidate NO. 17 5μg

FIG. 9

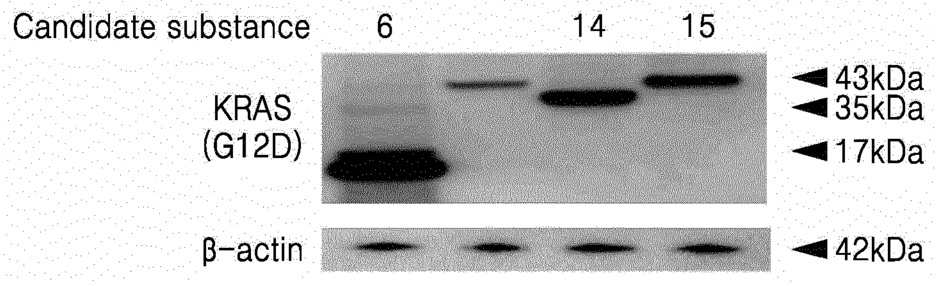
Candidate substance
6
14
15
KRAS
(G12D)
β-actin
43kDa
35kDa
17kDa
42kDa

# INTERNATIONAL SEARCH REPORT

International application No.
**PCT/KR2023/021488**

**A. CLASSIFICATION OF SUBJECT MATTER**

**C12N 9/14**(2006.01)i; **C12N 9/10**(2006.01)i; **A61K 38/45**(2006.01)i; **A61K 38/46**(2006.01)i; **A61P 35/00**(2006.01)i; **C07K 14/82**(2006.01)i; **C12N 15/62**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

C12N 9/14(2006.01); A61K 39/00(2006.01); A61K 39/39(2006.01); A61P 35/00(2006.01); C07K 14/725(2006.01); C07K 14/82(2006.01); C12N 5/0783(2010.01); C12Q 1/6827(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: KRAS, 항원(antigen), 변이체(variant), G12C, G12D, G13D, 링커(linker), 암(cancer), mRNA

**C. DOCUMENTS CONSIDERED TO BE RELEVANT**

| Category* | Citation of document, with indication, where appropriate, of the relevant passages | Relevant to claim No. |
|---|---|---|
| X | KR 10-2021-0032393 A (BIONTECH US INC.) 24 March 2021 (2021-03-24)<br>See claims 1, 19-20, 31-34, 40, 45, 75 and 92; and paragraphs [0032]-[0033], [0055], [0060], [0143], [0188]-[0189], [0280], [0291]-[0292] and [0395]. | 1-9,11,13-14,17 |
| A | | 10,12,15-16 |
| A | KR 10-2019-0110612 A (MODERNATX, INC.) 30 September 2019 (2019-09-30)<br>See entire document. | 1-17 |
| A | KR 10-2021-0013105 A (GRITSTONE ONCOLOGY, INC.) 03 February 2021 (2021-02-03)<br>See entire document. | 1-17 |
| A | US 2022-0088190 A1 (THE TRUSTEES OF THE UNIVERSITY OF PENNSYLVANIA) 24 March 2022 (2022-03-24)<br>See entire document. | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 April 2024** | **09 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/021488**

**C. DOCUMENTS CONSIDERED TO BE RELEVANT**

| Category* | Citation of document, with indication, where appropriate, of the relevant passages | Relevant to claim No. |
|---|---|---|
| A | KR 10-2022-0062488 A (BIONTECH US INC.) 17 May 2022 (2022-05-17)<br>See entire document. | 1-17 |
| PX | WO 2023-085657 A1 (MYONGJI HOSPITAL et al.) 19 May 2023 (2023-05-19)<br>See claims 1-5; paragraphs [0009] and [0034]; and figure 1. | 1-9,11,13-14,17 |

Form PCT/ISA/210 (second sheet) (July 2022)

**INTERNATIONAL SEARCH REPORT**

International application No.
**PCT/KR2023/021488**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/021488**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-20**
because they relate to subject matter not required to be searched by this Authority, namely:

Claims 18-20 pertain to a method for treatment of the human body by therapy, and thus pertain to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/021488**

| Patent document cited in search report | Publication date (day/month/year) | Patent family member(s) | Publication date (day/month/year) |
|---|---|---|---|
| KR 10-2021-0032393 A | 24 March 2021 | AU 2019-290127 A1 | 28 January 2021 |
| | | BR 112020025764 A2 | 11 May 2021 |
| | | CA 3103983 A1 | 26 December 2019 |
| | | CN 112638404 A | 09 April 2021 |
| | | EP 3810182 A1 | 28 April 2021 |
| | | IL 279458 A | 31 January 2021 |
| | | JP 2021-527426 A | 14 October 2021 |
| | | TW 202015719 A | 01 May 2020 |
| | | US 2021-0268091 A1 | 02 September 2021 |
| | | WO 2019-246286 A1 | 26 December 2019 |
| KR 10-2019-0110612 A | 30 September 2019 | AU 2017-397458 A1 | 15 August 2019 |
| | | AU 2017-397458 A1 | 09 August 2018 |
| | | AU 2018-214556 A1 | 15 August 2019 |
| | | BR 112019015797 A2 | 17 March 2020 |
| | | CA 3051252 A1 | 09 August 2018 |
| | | CA 3052255 A1 | 09 August 2018 |
| | | CN 110430894 A | 08 November 2019 |
| | | CN 110505877 A | 26 November 2019 |
| | | CN 117224710 A | 15 December 2023 |
| | | EP 3576751 A1 | 11 December 2019 |
| | | EP 3576780 A1 | 11 December 2019 |
| | | IL 268361 A | 26 September 2019 |
| | | JP 2020-506189 A | 27 February 2020 |
| | | JP 2020-514321 A | 21 May 2020 |
| | | JP 2023-103250 A | 26 July 2023 |
| | | JP 2023-164537 A | 10 November 2023 |
| | | KR 10-2019-0120233 A | 23 October 2019 |
| | | US 10881730 B2 | 05 January 2021 |
| | | US 2019-0175727 A1 | 13 June 2019 |
| | | US 2019-0351039 A1 | 21 November 2019 |
| | | US 2019-0351040 A1 | 21 November 2019 |
| | | US 2021-0128721 A1 | 06 May 2021 |
| | | WO 2018-144082 A1 | 09 August 2018 |
| | | WO 2018-144775 A1 | 09 August 2018 |
| KR 10-2021-0013105 A | 03 February 2021 | AU 2019-275072 A1 | 21 January 2021 |
| | | AU 2019-275072 A2 | 04 February 2021 |
| | | CA 3099644 A1 | 28 November 2019 |
| | | CN 112368386 A | 12 February 2021 |
| | | EP 3796927 A1 | 31 March 2021 |
| | | IL 278864 A | 31 January 2021 |
| | | JP 2021-525076 A | 24 September 2021 |
| | | US 2021-0196806 A1 | 01 July 2021 |
| | | WO 2019-226941 A1 | 28 November 2019 |
| US 2022-0088190 A1 | 24 March 2022 | AU 2020-213119 A1 | 09 September 2021 |
| | | CA 3127673 A1 | 30 July 2020 |
| | | CN 113631172 A | 09 November 2021 |
| | | EP 3914270 A1 | 01 December 2021 |
| | | EP 3914270 A4 | 11 January 2023 |
| | | JP 2022-523052 A | 21 April 2022 |
| | | KR 10-2021-0119468 A | 05 October 2021 |

Form PCT/ISA/210 (patent family annex) (July 2022)

**INTERNATIONAL SEARCH REPORT**
**Information on patent family members**

International application No.
**PCT/KR2023/021488**

| Patent document cited in search report | Publication date (day/month/year) | Patent family member(s) | Publication date (day/month/year) |
|---|---|---|---|
| | | WO 2020-154617 A1 | 30 July 2020 |
| KR 10-2022-0062488 A | 17 May 2022 | BR 112021025050 A2 | 03 May 2022 |
| | | CA 3141084 A1 | 17 December 2020 |
| | | CN 114269357 A | 01 April 2022 |
| | | EP 3982980 A1 | 20 April 2022 |
| | | JP 2022-536695 A | 18 August 2022 |
| | | TW 202126327 A | 16 July 2021 |
| | | US 2023-0000960 A1 | 05 January 2023 |
| | | WO 2020-252039 A1 | 17 December 2020 |
| WO 2023-085657 A1 | 19 May 2023 | KR 10-2023-0068628 A | 18 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VIITALA et al.** *Clin Cancer Res*, 2019, vol. 25 (11), 3289-3303